# EUROPEAN PATENT APPLICATION

(11) **EP 2 476 461 A2**
(43) Date of publication of application: **18.07.2012**
(21) Application number: 11192725.7
(22) Date of filing: 16.01.2008
(51) Int. Cl.: A61P 35/00, A61K 9/48, A61K 31/166, A61K 47/10, A61K 47/12, A61K 47/18, A61K 47/26, A61K 47/48, A61K 9/00, A61K 9/107, A61K 9/16, B82Y 5/00

(54) **Formulations For Cancer Treatment**

(30) Priority: 16.01.2007 US 88075507 P
(62) Divisional of application: 08727771.1
(71) Applicant: BiPar Sciences, Inc., Bridgewater NJ 08807 (US)
(72) Inventor: Bastin, Richard J., Wiltshire (GB); Han, Helen N., San Francisco, CA 94107-6013 (US); Li, Lingyun, Foster City, CA 94404 (US); Nava, Stephen, San Francisco, CA 94110 (US); Ossovskaya, Valeria S., San Francisco, CA 94105 (US); Powell, Kathy, Cary, NC 27519 (US); Sherman, Barry M., Hillsborough, CA 94010 (US)
(74) Representative: Adamson Jones

(57) **Abstract**

The present invention provides compositions of matter, kits, and methods for use in treating cancer and viral conditions. In particular the invention provides for pharmaceutical compositions containing nitrobenzamide compounds that have enhanced solubility in aqueous solutions.

## Description

### FIELD OF THE INVENTION

The present invention relates to formulations useful in the treatment of cancer viral diseases and other disease states. Specifically, the invention relates to formulations that facilitate use and bioavailability of aromatic nitrobenzamide compounds.

### BACKGROUND OF THE INVENTION

Cancer is a serious threat to modem society. Malignant cancerous growths, due to their unique characteristics, pose serious challenges for modem medicine. Their characteristics include uncontrollable cell proliferation resulting in unregulated growth of malignant tissue, an ability to invade local and even remote tissues, lack of differentiation, lack of detectable symptoms and most significantly, the lack of effective therapy and prevention.

Cancer can develop in any tissue of any organ at any age. The etiology of cancer is not clearly defined but mechanisms such as genetic susceptibility, chromosome breakage disorders, viruses, environmental factors and immunologic disorders have all been linked to a malignant cell growth and transformation. Cancer encompasses a large category of medical conditions, affecting millions of individuals worldwide. Cancer cells can arise in almost any organ and/or tissue of the body. Cancer develops when cells in a part of the body begin to grow or differentiate out of control. All cancer types begin with the out-of-control growth of abnormal cells.

There are many types of cancer, including, breast, lung, ovarian, bladder, prostate, pancreatic, cervical, and leukemia. Currently, some of the main treatments available are surgery, radiation therapy, and chemotherapy. Surgery is often a drastic measure and can have serious consequences. For example, all treatments for ovarian cancer may result in infertility. Some treatments for cervical cancer and bladder cancer may cause infertility and/or sexual dysfunction. Surgical procedures to treat pancreatic cancer may result in partial or total removal of the pancreas and can carry significant risks to the patient. Breast cancer surgery invariably involves removal of part of or the entire breast. Some surgical procedures for prostate cancer carry the risk of urinary incontinence and impotence. The procedures for lung cancer patients often have significant post-operative pain as the ribs must be cut through to access and remove the cancerous lung tissue. In addition, patients who have both lung cancer and another lung disease, such as emphysema or chronic bronchitis, typically experience an increase in their shortness of breath following the surgery.

Radiation therapy has the advantage of killing cancer cells but it also damages non-cancerous tissue at the same time. Chemotherapy involves the administration of various anti-cancer drugs to a patient but often is accompanied by adverse side effects.

Worldwide, more than 10 million people are diagnosed with cancer every year and it is estimated that this number will grow to 15 million new cases every year by 2020. Cancer causes six million deaths every year or 12% of the deaths worldwide. There remains a need for methods that can treat cancer. These methods can provide the basis for pharmaceutical compositions useful in the prevention and treatment of cancer in humans and other mammals.

Viral infections are also a serious threat to human health throughout the world. Human immunodeficiency virus (HIV) infections known as acquired immunodeficiency syndrome (AIDS), presently constitute a worldwide health hazard. HIV infections are almost always fatal due to a weakened immuno-resistance, leading to opportunistic infections, malignancies and neurologic lesions.

There is no effective treatment for AIDS other than the treatment of the opportunistic infections, neoplasms and other complications. Available cytostatic (AZT) and antiviral (acyclovir) drugs are extremely toxic and cause severe adverse reactions.

Thus it would be highly desirable to have available an effective and yet nontoxic treatment of viral diseases, in particular, AIDS.

Herpes simplex virus type-1 and 2 are also wide-spread infections. They may occur in AIDS patients as one of the opportunistic infections. Type-1 HSV strain (HSV-1) commonly causes herpes labialism located on a lip, and keratitis, an inflammation of the cornea. Type-2 HSV is usually located on or around the genital area and is generally transmitted primarily by direct contact with herpetic sore or lesions. HSV-2 has been related to the development of uterine cancer.

Herpes simplex virus is very infectious and is rapidly and easily transferable by contact. There is no specific therapy to this extremely painful viral infection. Current treatment of HSV infections is limited primarily to systemic administration of the above-mentioned antiviral drugs with corresponding adverse side affects.

The antiviral agents used for HSV treatment are non-selective inhibitors of HSV replication affecting the replication of normal cells as well. Therefore, when used in doses large enough to inactivate all of the active herpes viruses dormant in the sensory ganglia, these compounds may also be highly disruptive to host cell DNA replication.

Thus, it would be advantageous to have available non-toxic treatment of HSV infections.

Cytomegalovirus (CMV), a dangerous co-infection of HIV, is a subgroup of highly infectious viruses having the propensity for remaining latent in man. CMVs are very common among the adult population and as many as 90% of adults have been exposed to and experienced CMV infections. CMVs are normally present in body liquids such as blood, lymph, saliva, urine, feces, milk, etc. CMV infections may cause abortion, stillbirth, postnatal death from hemorrhage, anemia, severe hepatic or CNS damage. Particularly dangerous are CMV infections afflicting AIDS patients, where CMV may cause pulmonary, gastrointestinal or renal complications. There is no specific therapy for CMVs. Unlike HSV, CMV is resistant to acyclovir, and to other known antiviral drugs.

Thus, it would be extremely advantageous to have available a drug which would effectively inhibit CMV infections.

A series of anti-tumor drugs and anti-viral have been identified. These drugs include nitro and nitroso compounds and their metabolites, which are the subject of U.S. Pat. No. 5,464,871 issued on Nov. 7, 1995 entitled "Aromatic Nitro and Nitroso Compounds and their Metabolites Useful as Anti-viral and Anti-tumor Agents," Pat. No. 5,670,518 issued on Sept. 23, 1997 entitled "Aromatic Nitro and Nitroso Compounds and their Metabolites Useful as Anti-viral and Anti-tumor Agents," Pat. No. 6,004,978 issued on Dec. 21, 1999 entitled "Methods of Treating Cancer with Aromatic Nitro and Nitroso Compounds and their Metabolites" the disclosures of which are incorporated herein by reference. The use of the compounds has been described in the art as useful in treating mammary gland adenocarcinomas, mammary gland duct carcinomas, lymphocytic leukemia, Karposi's sarcoma in immuno-suppressed patients with AIDS, and neoplastic growths such as non-Hodgkin lymphoma, and primary lymphomas.

While nitrobenzamide compounds have been shown to be useful anti-tumor and anti-viral agents, the compounds tend to be poorly soluble in water. In order to maximize the bioavailability of a compound, it is often desirable for the compound to have good solubility in aqueous solution. Enhanced solubility in aqueous solution can increase bioavailability for many compound delivery modes including injection, oral ingestion, or transdermal delivery, because of the aqueous nature of the blood and other aqueous bodily fluids.

### SUMMARY OF THE INVENTION

The present invention relates generally to pharmaceutical compositions of aromatic nitrobenzamide compounds or their metabolites and solubilizers having enhanced solubility. More specifically, it relates to pharmaceutical compositions comprising the nitro compound 4-iodo-3-nitrobenzamide or a salt, solvate, isomer, tautomer, metabolite, analog, or prodrug thereof and solubilizers with enhanced solubility. Typically, the pharmaceutical composition of the invention will have an aromatic benzamide solubility in aqueous solution greater than 1.5 times the solubility of the aromatic benzamide compound in pure water; preferably the solubility is greater than 2 times higher, more preferably the solubility is greater than 5 times higher, even more preferably the solubility is greater that 10 times higher, and most preferably the solubility is greater than 50 times higher. Most preferably the solubility is up to about 55 times greater. Examples of suitable solubilizers include, but are not limited to, Tween 80, glycofural, glycerin formal, and DMA. Other types of solubilizers include Cremophor EL, 30% Solutol, and PEG 400 (50%).

The present invention relates to pharmaceutical compositions comprising an aromatic nitrobenzamide compound or its metabolites and a solubilizer wherein the solubilizer comprises an oligosaccharide. A preferred embodiment of an oligosaccharide is a cyclic oligosaccharide, such as cyclodextrin. More specifically, the invention relates to pharmaceutical compositions comprising the nitro compound 4-iodo-3-nitrobenzamide or a salt, solvate, isomer, tautomer, metabolite, analog, or prodrug thereof and a cyclodextrin.

The present invention also relates to pharmaceutical compositions comprising an aromatic nitrobenzamide compound or its metabolites and a solubilizer, where the solubilizer comprises a surfactant. More specifically, it relates to pharmaceutical compositions comprising the nitro compound 4-iodo-3-nitrobenzamide or a salt, solvate, isomer, tautomer, metabolite, analog, or prodrug thereof and a surfactant having enhanced solubility.

The present invention also relates to pharmaceutical compositions comprising an aromatic nitrobenzamide compound or its metabolites and a solubilizer where the solubilizer comprises a co-solvent. More specifically, it relates to pharmaceutical compositions comprising the nitro compound 4-iodo-3-nitrobenzamide or a salt, solvate, isomer, tautomer, metabolite, analog, or prodrug thereof and a co-solvent having enhanced solubility.

The present invention also relates to pharmaceutical compositions comprising an aromatic nitrobenzamide compound or its metabolites and a mixture of (1) a cylodextrin and a surfactant, (2) a cyclodextrin and a co-solvent, (3) a surfactant and a co-solvent, or (4) a cyclodextrin, a surfactant, and a co-solvent having enhanced solubility. More specifically, it relates to pharmaceutical compositions comprising the nitro compound 4-iodo-3-nitrobenzamide or a salt, solvate, isomer, tautomer, metabolite, analog, or prodrug thereof and a mixture of (1) a cylodextrin and a surfactant, (2) a cyclodextrin and a co-solvent, (3) a surfactant and a co-solvent, or (4) a cyclodextrin, a surfactant, and a co-solvent having enhanced solubility. Most preferred formulation is with 25% beta-cyclodextrin and 10 mM phosphate at pH 7.4.

The present invention also relates to methods of treating subjects suspected of having a viral condition or cancer comprising treating the subject with a pharmaceutical composition comprising an aromatic nitrobenzamide compound or its metabolites and a solubilizer, where the solubilizer is a cyclodextrin, a surfactant, a co-solvent, or a mixture of (1) a cylodextrin and a surfactant, (2) a cyclodextrin and a co-solvent, (3) a surfactant and a co-solvent, or (4) a cyclodextrin, a surfactant, and a co-solvent.

The present invention also relates more specifically to methods of treating subjects suspected of having a viral condition or cancer by treating the subject with a pharmaceutical composition comprising nitro compound 4-iodo-3-nitrobenzamide or a salt, solvate, isomer, tautomer, metabolite, analog, or prodrug thereof and a solubilizer, where the solubilizer is a cyclodextrin, a surfactant, a co-solvent, or a mixture of (1) a cylodextrin and a surfactant, (2) a cyclodextrin and a co-solvent, (3) a surfactant and a co-solvent, or (4) a cyclodextrin, a surfactant, and a co-solvent.

The present invention also relates to a kit comprising an aromatic nitrobenzamide compound or a metabolite and a solubilizer, where the solubilizer is a cyclodextrin, a surfactant, a co-solvent, or a mixture of (1) a cylodextrin and a surfactant, (2) a cyclodextrin and a co-solvent, (3) a surfactant and a co-solvent, or (4) a cyclodextrin, a surfactant, and a co-solvent.

The present invention also relates more specifically to a kit comprising a nitro compound of 4-iodo-3-nitrobenzamide or a salt, solvate, isomer, tautomer, metabolite, analog, or prodrug thereof and a solubilizer, where the solubilizer is a cyclodextrin, a surfactant, a co-solvent, or a mixture of (1) a cylodextrin and a surfactant, (2) a cyclodextrin and a co-solvent, (3) a surfactant and a co-solvent, or (4) a cyclodextrin, a surfactant, and a co-solvent.

The pharmaceutical compositions of this invention can be used to treat various cancers, including leukemia, breast cancer, ovarian cancer, lung cancer, bladder cancer, prostate cancer, pancreatic cancer, and cervical cancer, as well as other cancer types described herein.

The pharmaceutical compositions of this invention can also be used as anti-viral agents against various viruses including Human immunodeficiency virus (HIV), Herpes simplex virus (HSV), and Cytomegalovirus (CMV).

A composition of the invention can be a combination of two or more compounds described herein and/or a combination of two or more forms of a compound described herein. A pharmaceutical composition of the invention may be a composition suitable for administration to a subject.

### INCORPORATION BY REFERENCE

All publications and patent applications mentioned in this specification are herein incorporated by reference to the same extent as if each individual publication or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

Figure 1 provides in graph form the solubility of 4-iodo-3-nitrobenzamide in water versus the concentration of hydroxypropyl-β-cyclodextrin (HPBCD; also referred to herein as "HPβCD").

Figure 2 shows the solubility of 4-iodo-3-nitrobenzamide ("BA") in water versus the concentration of N,N-dimethylacetamide/Capmul Solutions.

Figure 3 shows the Bioavailability results from Phase I and II animal studies on encapsulated BA formulations.

Figure 4 shows the trend for BA saturation in semi-solid lipid samples over time.

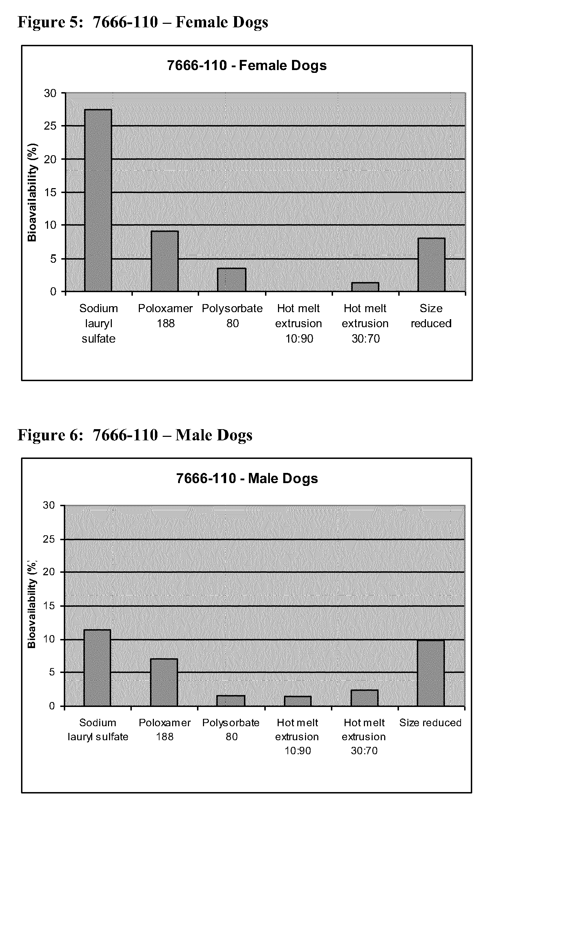

Figure 5 shows the bioavailability results for several formulations of BA in female dogs.

Figure 6 shows the bioavailability results for several formulations of BA in male dogs.

Figure 7 shows the pharmacokinetic (PK) profiles for size-reduced BA and metabolites (IABM and IABA) in Dogs following oral (PO) dosing of 60 mg/kg of BA with sodium lauryl sulfate (SLS).

Figure 8 shows the pharmacokinetic (PK) profiles for size-reduced BA and metabolites (IABM and IABA) in Dogs oral (PO) doses of 60 mg/kg of BA with and without 1% sodium lauryl sulfate (SLS).

Figure 9 shows the bioavailability results for granulated BA with 0%, 1% or 2% SLS in female dogs.

Figure 10 shows the bioavailabilityresults for granulated BA with 0%, 1% or 2% SLS in male dogs.

Figure 11 shows the pharmacokinetic profiles for BA and metabolites in dogs given an oral dose of 60 mg/kg of BA in either micronized or size-reduced forms.

Figure 12 shows comparison of the PK profiles of BA and its metabolites (IABM, IABA) in dogs given of either an IV infusion or an oral dose of BA (micronized with 2% SLS). Giving BA orally prolonged the exposure of BA and its metabolites in dogs.

### DETAILED DESCRIPTION OF THE INVENTION

Some embodiments described herein provide pharmaceutical composition comprising a compound of formula (Ia) 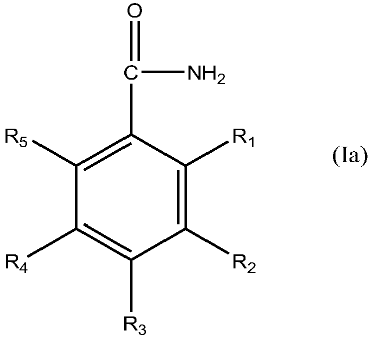 wherein R₁, R₂, R₃, R₄, and R₅ are, independently selected from the group consisting of hydrogen, hydroxy, amino, nitro, iodo, bromo, fluoro, chloro, (C₁ -C₆) alkyl, (C₁ -C₆) alkoxy, (C₃ -C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof, and at least one pharmaceutically acceptable solubilizer. In some embodiments, the solubilizer comprises a cyclodextrin, a surfactant, a co-solvent, or mixtures of two or more thereof. In some embodiments, the solubility of the compound of formula Ia is at least about 1.5 times the solubility of that compound in pure water. In some embodiments, the solubility of the compound of formula Ia is at least about 2 times the solubility of that compound in pure water. In some embodiments, the solubility of the compound of formula Ia is at least about 5 times the solubility of that compound in pure water. In some embodiments, the solubility of the compound of formula Ia is at least about 10 times the solubility of that compound in pure water. In some embodiments, the solubility of the compound of formula Ia is at least about 50 times the solubility of that compound in pure water. In some embodiments, the composition is an oral composition, such as a tablet or capsule. In some embodiments, the composition is a parenteral composition, such as an intravenous or intraperitoneal injection.

A pharmaceutical composition comprising a compound of formula (Ia) 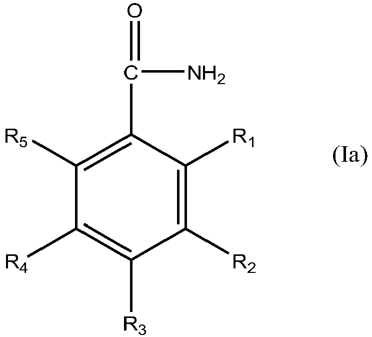 wherein R₁, R₂, R₃, R₄, and R₅ are, independently selected from the group consisting of hydrogen, hydroxy, amino, nitro, iodo, bromo, fluoro, chloro, (C₁ -C₆) alkyl, (C₁ -C₆) alkoxy, (C₃ -C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof, and a surfactant.
The composition of Claim 8, wherein the surfactant comprises one or more of: a poloxamer, a polysorbate, a polyethoxylated triglyceride, a polyethoxylated fatty acid or a compound of formula II or III: 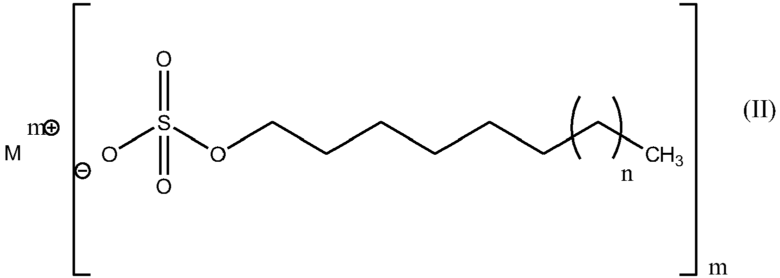 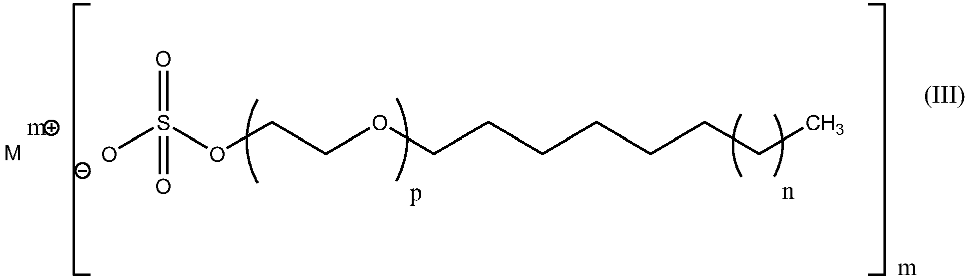 wherein M is a metal ion having a positive charge m+, m is an integer of value 1, 2 or 3 and n is an integer of value 1 to 11 and p is a value of 1 to 10. In some embodiments, the surfactant comprises one or more of: sodium lauryl sulfate, sodium laureth sulfate, Polysorbate 80, Polysorbate 20, Cremophor EL, Cremophor RH40, Poloxamer 118 or Solutol HS-15. In some embodiments, the formulation is an oral formulation, such as a tablet or capsule. In some embodiments, the formulation is a parenteral formulation, such as in intravenous or intraperitoneal injection.

A pharmaceutical composition comprising a compound of formula (Ia) 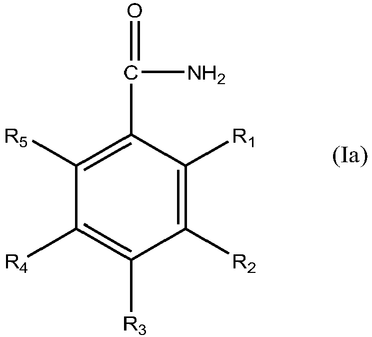 wherein R₁, R₂, R₃, R₄, and R₅ are, independently selected from the group consisting of hydrogen, hydroxy, amino, nitro, iodo, bromo, fluoro, chloro, (C₁ -C₆) alkyl, (C₁ -C₆) alkoxy, (C₃ -C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof, and a cyclodextrin. In some embodiments, the cyclodextrin comprises one or more of: hydroxypropyl-β-cyclodextrin, hyroxypropyl-y-cyclodextrin, and sulfobutyl ether- β-cyclodextrin. In some embodiments, the formulation is an oral formulation, such as a tablet or capsule. In some embodiments, the formulation is a parenteral formulation, such as in intravenous or intraperitoneal injection.

A pharmaceutical composition comprising a compound of formula (Ia) 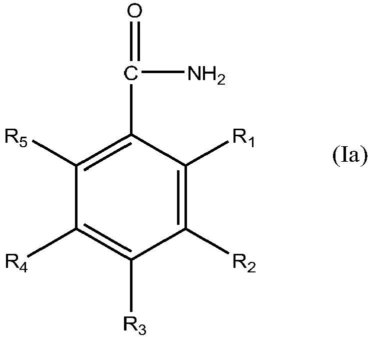 wherein R₁, R₂, R₃, R₄, and R₅ are, independently selected from the group consisting of hydrogen, hydroxy, amino, nitro, iodo, bromo, fluoro, chloro, (C₁ -C₆) alkyl, (C₁ -C₆) alkoxy, (C₃ -C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof, and a co-solvent. In some embodiments, the co-solvent is selected from the group of: ethanol, glycofurol, glycerin formal, benzyl alcohol, PEG 400, propylene glycol, and N, N-dimethyl acetamide (DMA). In some embodiments, the formulation is an oral formulation, such as a tablet or capsule. In some embodiments, the formulation is a parenteral formulation, such as in intravenous or intraperitoneal injection.

A method for treating a condition selected from cancer, and a viral condition, comprising treating a subject suspected of having said condition with a pharmaceutical composition comprising a compound of formula (Ia) 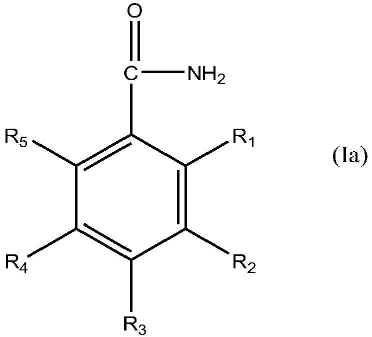 wherein R₁, R₂, R₃, R₄, and R₅ are, independently selected from the group consisting of hydrogen, hydroxy, amino, nitro, iodo, bromo, fluoro, chloro, (C₁ -C₆) alkyl, (C₁-C₆) alkoxy, (C₃-C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof, and one or more of the group consisting of: a cyclodextrin, a surfactant, and a co-solvent. In some embodiments, the compound of formula Ia is administered orally. In some embodiments, the compound of formula Ia is administered parenterally. In some embodiments, the cyclodextrin is selected from the group of hydroxypropyl-β-cyclodextrin, hyroxypropyl-γ-cyclodextrin, and sulfobutyl ether- β-cyclodextrin. In some embodiments, the surfactant is selected from the group of: Polysorbate 80, Polysorbate 20, Cremophor EL, Cremophor RH40, Poloxamer 118, and Solutol HS-15. In some embodiments, the co-solvent is selected from the group of: ethanol, glycofurol, glycerin formal, benzyl alcohol, PEG 400, propylene glycol, and N, N-dimethyl acetamide (DMA). In some embodiments, the cancer is a member of the group consisting of wherein the cancer is selected from adrenal cortical cancer, anal cancer, aplastic anemia, bile duct cancer, bladder cancer, bone cancer, bone metastasis, CNS tumors, peripheral CNS cancer, breast cancer, Castleman's Disease, cervical cancer, childhood Non-Hodgkin's lymphoma, colon and rectum cancer, endometrial cancer, esophagus cancer, Ewing's family of tumors, eye cancer, gallbladder cancer, gastrointestinal carcinoid tumors, gastrointestinal stromal tumors, gestational trophoblastic disease, hairy cell leukemia, Hodgkin's disease, Kaposi's sarcoma, kidney cancer, laryngeal and hypopharyngeal cancer, acute lymphocytic leukemia, acute myeloid leukemia, children's leukemia, chronic lymphocytic leukemia, chronic myeloid leukemia, liver cancer, lung cancer, lung carcinoid tumors, Non-Hodgkin's lymphoma, male breast cancer, malignant mesothelioma, multiple myeloma, myelodysplastic syndrome, myeloproliferative disorders, nasal cavity and paranasal cancer, nasopharyngeal cancer, neuroblastoma, oral cavity and oropharyngeal cancer, osteosarcoma, ovarian cancer, pancreatic cancer, penile cancer, pituitary tumor, prostate cancer, retinoblastoma, rhabdomyosarcoma, salivary gland cancer, sarcoma (adult soft tissue cancer), melanoma skin cancer, non-melanoma skin cancer, stomach cancer, testicular cancer, thymus cancer, thyroid cancer, uterine sarcoma, vaginal cancer, vulvar cancer, Waldenstrom's macroglobulinemia and cancers of viral origin. In some embodiments, the formulation is an oral formulation, such as a tablet or capsule. In some embodiments, the formulation is a parenteral formulation, such as in intravenous or intraperitoneal injection.

Some embodiments described herein provide a kit comprising a compound of formula (Ia) 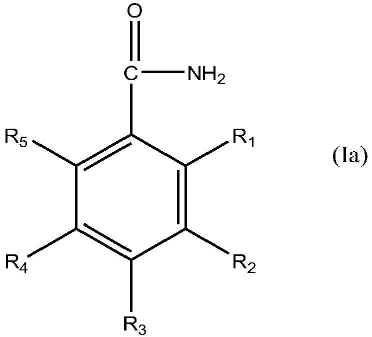 wherein R₁, R₂, R₃, R₄, and R₅ are, independently selected from the group consisting of hydrogen, hydroxy, amino, nitro, iodo, bromo, fluoro, chloro, (C₁-C₆) alkyl, (C₁-C₆) alkoxy, (C₃-C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof, and a cyclodextrin, a surfactant, a co-solvent, or mixtures therof. In some embodiments, the cyclodextrin is selected from the group of hydroxypropyl-β-cyclodextrin, hyroxypropyl-γ-cyclodextrin, and sulfobutyl ether- β-cyclodextrin. In some embodiments, the surfactant is selected from the group of: Polysorbate 80, Polysorbate 20, Cremophor EL, Cremophor RH40, Poloxamer 118, and Solutol HS-15. In some embodiments, the co-solvent is selected from the group of: ethanol, glycofurol, glycerin formal, benzyl alcohol, PEG 400, propylene glycol, and N, N-dimethyl acetamide (DMA). In some embodiments, the formulation is an oral formulation, such as a tablet or capsule. In some embodiments, the formulation is a parenteral formulation, such as in intravenous or intraperitoneal injection.

Some embodiments described herein provide an aqueous solution comprising a compound of formula (Ia): 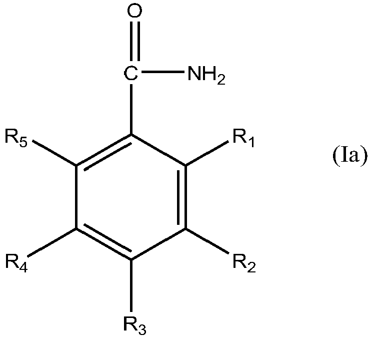
wherein R₁, R₂, R₃, R₄, and R₅ are, independently selected from the group consisting of hydrogen, hydroxy, amino, nitro, iodo, bromo, fluoro, chloro, (C₁-C₆) alkyl, (C₁-C₆) alkoxy, (C₃-C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof, and a cyclodextrin, a surfactant, a co-solvent, or mixtures thereof.

Some embodiments described herein provide a unit dosage comprising a compound of formula (Ia): 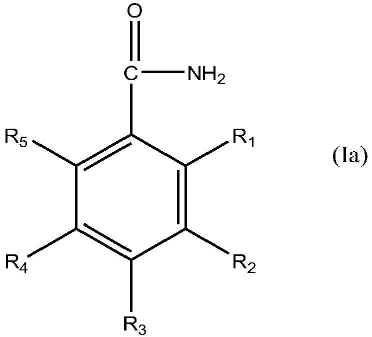
wherein R₁, R₂, R₃, R₄, and R₅ are, independently selected from the group consisting of hydrogen, hydroxy, amino, nitro, iodo, bromo, fluoro, chloro, (C₁-C₆) alkyl, (C₁-C₆) alkoxy, (C₃-C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof, and at least one pharmaceutically acceptable solubilizer. In some embodiments, the solubilizer comprises a surfactant and optionally one or more of a cyclodextrin, a co-solvent, a lipid or mixtures thereof. In some embodiments, the solubility of the compound is at least about 1.5 times the solubility of that compound in pure water. In some embodiments, the solubility of the compound is at least about 2 times the solubility of that compound in pure water. In some embodiments, the solubility of the compound is at least about 5 times the solubility of that compound in pure water. In some embodiments, the compound is at least about 10 times the solubility of that compound in pure water. In some embodiments, the compound is at least about 50 times the solubility of that compound in pure water. In some embodiments the surfactant is one or more compounds of formula II or III: 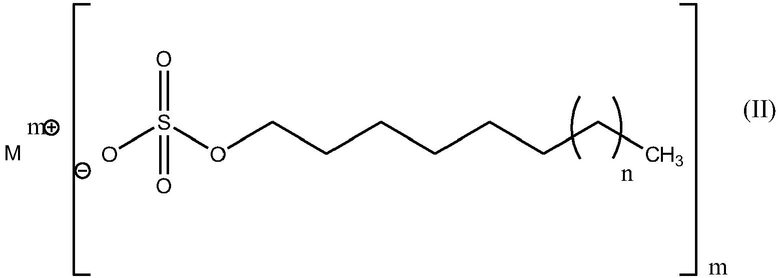 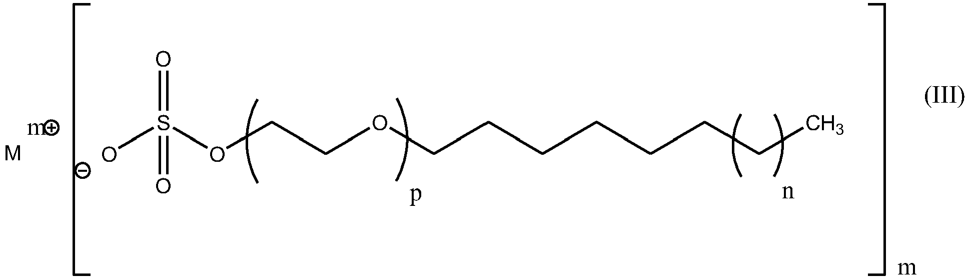
wherein M is a metal ion having a positive charge m+, m is an integer of value 1, 2 or 3 and n is an integer of value 1 to 11 and p is a value of 1 to 10. In some embodiments, the surfactant comprises one or more poloxamer, polysorbate, polyethoxylated triglyceride or polyethoxylated fatty acid.
The unit dosage of 35, wherein the surfactant is selected from the group of: Polysorbate 80, Polysorbate 20, Cremophor EL, Cremophor RH40, Poloxamer 118, and Solutol HS-15. In some embodiments, the unit dosage formulation is an oral formulation, such as a tablet or capsule. In some embodiments, the unit dosage formulation is a parenteral formulation, such as in intravenous or intraperitoneal injection.

A parenteral pharmaceutical composition comprising a compound of formula (Ia) 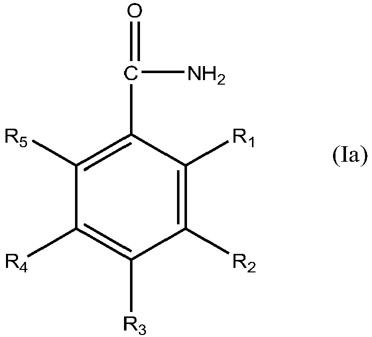 wherein R₁, R₂, R₃, R₄, and R₅ are, independently selected from the group consisting of hydrogen, hydroxy, amino, nitro, iodo, bromo, fluoro, chloro, (C₁-C₆) alkyl, (C₁-C₆) alkoxy, (C₃-C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof; a cyclodextrin; and water. In some embodiments, the cyclodextrin is selected from the group of hydroxypropyl-β-cyclodextrin, hyroxypropyl-γ-cyclodextrin, and sulfobutyl ether- β-cyclodextrin. In some embodiments, the composition further comprises a co-solvent. In some embodiments, the co-solvent is selected from the group of: ethanol, glycofurol, glycerin formal, benzyl alcohol, PEG 400, propylene glycol, and N, N-dimethyl acetamide (DMA).

### Definitions

"nitrobenzamide compound(s)" means a compound of the formula (Ia) 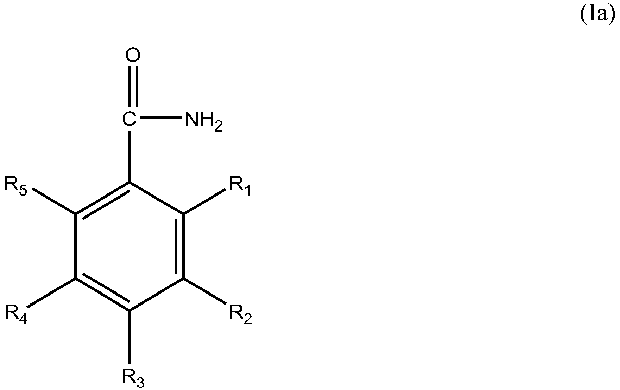 wherein R₁, R₂, R₃, R₄, and R₅ are, independently selected from the group consisting of hydrogen, hydroxy, amino, nitro, bromo, fluoro, chloro,₌iodo, (C₁-C₆) alkyl, (C₁-C₆) alkoxy, (C₃-C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof. R₁, R₂, R₃, R₄, and R₅ can also be a halide such as chloro, fluoro, or bromo.

"Solubility" generally means the amount of a compound dissolved in a solvent. Suitable solvents include aqueous and non-aqueous solvents.

A compound is "dissolved" when it is "in solution", and does not spontaneously come out of solution to form a separate phase. In order to be dissolved, the compound need not dissociate completely on a molecular level, but must remain in solution so as to be effective in treatment of a disease or condition. A dissolved compound may be present in a micellar, emulsified, or liposomal form. A solution with dissolved compound will generally be clear.

"Poor solubility" means a small amount of compound dissolved in a solvent. Poor solubility is not an absolute term, but depends on the amount of the compound that is needed for effective treatment of a disease or condition. A compound will be poorly soluble if its solubility is lower than is desired in order for an effective treatment of a disease or condition.

"Enhanced solubility" means higher solubility than for the nitrobenzamide compound alone.

The term "treating" and its grammatical equivalents as used herein include attempting to achieve a therapeutic benefit and/or a prophylactic benefit. By therapeutic benefit is meant eradication or amelioration of the underlying disorder being treated. For example, in a cancer patient, therapeutic benefit includes eradication or amelioration of the underlying cancer. Also, a therapeutic benefit is achieved with the eradication or amelioration of one or more of the physiological symptoms associated with the underlying disorder such that an improvement is observed in the patient, notwithstanding the fact that the patient may still be afflicted with the underlying disorder. For prophylactic benefit, a method of the invention may be performed on, or a composition of the invention administered to a patient at risk of developing cancer, or to a patient reporting one or more of the physiological symptoms of such conditions, even though a diagnosis of the condition may not have been made.

Enhanced solubility in water can be useful because many bodily fluids such as blood are water based (aqueous), and therefore, a more water soluble drug can have higher bioavailability. While the exact solubility of a compound in pure water is not the same as in an aqueous solution such as blood, a composition's solubility in pure water is often a good indication of solubility in other aqueous solutions.

The present invention provides pharmaceutical compositions containing nitrobenzamide compounds that have enhanced solubility in water.

In a preferred embodiment of the invention, the pharmaceutical composition of the invention will have a solubility of aromatic benzamide in aqueous solution greater than 1.5 times the solubility of the aromatic benzamide compound in pure water; preferably the solubility is greater than 2 times higher, more preferably the solubility is greater than 5 times higher, even more preferably the solubility is greater that 10 times higher, and most preferably the solubility is greater than 50 times higher.

While generally high drug solubility is desired, is would be appreciated by persons of ordinary skill in the art that there are other considerations in creating a pharmaceutical composition such as viscosity, stability, potential toxicity, etc. that may result a composition with lower solubility being more desirable for a particular therapy or delivery method as long as the amount of available drug is enough for the application. The present invention allows the pharmaceutical formulator the ability to optimize these factors.

The present invention provides for the use of pharmaceutical compositions containing nitrobenzamide compounds for the treatment of various cancers, including leukemia, breast cancer, ovarian cancer, lung cancer, bladder cancer, prostate cancer, pancreatic cancer, and cervical cancer, as well as other cancer types such as other breast cancers including a ductal carcinoma in a mammary gland, other forms of leukemia including acute promyleocytic leukemia in peripheral blood, ovarian cancer, lung cancer, bladder cancer, prostate cancer, pancreatic cancer, and cervical cancer, as well as other cancer types described herein.

It has been reported that nitrobenzamide compounds have selective cytotoxicity upon malignant cancer cells but not upon nonmalignant cancer cells. *See* Rice et at., Proc. Natl. Acad. Sci. USA 89:7703-7707 (1992). In one embodiment, the pharmaceutical compositions of the present invention may exhibit more selective toxicity towards tumor cells than non-tumor cells.

It has been reported that the tumorgenicity of nitrobenzamide and nitrososbenzamide compounds is enhanced when buthionine sulfoximine BSO is co-administered to cancer cells. *See* Mendeleyev et al., Biochemical Pharmacol. 50(5):705-714 (1995). Buthionine sulfoximine (BSO) inhibits gamma-glutamylcysteine sythetase, a key enzyme in the biosynthesis of glutathione, which is responsible in part for cellular resistance to chemotherapy. See Chen et al., Chem Biol Interact. Apr 24;111-112:263-75 (1998). The invention also provides pharmaceutical compounds useful for treating cancer via the administration of a nitrobenzamide compound in combination with BSO.

One aspect of the present invention is pharmaceutical compositions comprising a nitrobenzamide compound and an oligosaccharide. As used herein, an oligosaccharide is a compound with two or more monosaccharides, or sugar units, which are covalently connected, usually through glycosidic linkages. Oligosaccharides usually have less than 20 monosaccharides, typically having 3 to 6 monosaccharides. The oligosaccharides can be either a linear chain or branched. The monosaccharide can be, for example, a triose, tetrose, pentose, hexose or heptose. Preferred monosaccharides are glyceraldehyde, dihydroxyacetone, erythrose, threose, arabinose, lyxose, ribose, deoxyribose, xylose, ribulose, xylulose, allose, altose, galactose, glucose, gulose, idose, mannose, tagatose, fructose, mannoheptulose, and sedoheptulose. The oligosaccharide can be, for instance, a maltodextrin. A preferred embodiment of an oligosaccharide is a cyclic oligosaccharide, such as cyclodextrin.

Cyclodextrins are cyclic carbohydrates that are derived from starch. The unmodified cyclodextrins differ by the number of glucopyranose units joined together in the cylindrical structure. The parent cyclodextrins contain 6, 7, or 8 glucopyranose units and are referred to as .alpha.-, .beta.-, and .gamma.-cyclodextrin respectively. Each cyclodextrin subunit has secondary hydroxyl groups at the 2 and 3-positions and a primary hydroxyl group at the 6-position. The cyclodextrins may be pictured as hollow truncated cones with hydrophilic exterior surfaces and hydrophobic interior cavities. In aqueous solutions, these hydrophobic cavities provide a haven for hydrophobic organic compounds, which can fit all, or part of their structure into these cavities. This process, known as inclusion complexation, may result in increased apparent aqueous solubility and stability for the complexed drug.

Chemical modification of the parent cyclodextrins (usually at the hydroxyl moieties) has resulted in derivatives with sometimes improved safety while retaining or improving the complexation ability of the cyclodextrin. Some preferred derivatives of cyclodextrins are; the 2-hydroxypropyl derivatives, e.g. hydroxypropyl-β-cyclodextrin (Kleptose® from Roquette) (HPBCD) and hyroxypropyl-γ-cyclodextrin (Cavamax W8® from Wacker), and the sulfoalkyl ether derivatives (SAE-CDs), e.g. sulfobutyl ether- β-cyclodextrin (Captisol® from Cydex) (SBEBCD).

The SAE-CDs are a class of negatively charged cyclodextrins, which vary in the nature of the alkyl spacer, the salt form, the degree of substitution and the starting parent cyclodextrin.

The anionic sulfobutyl ether substituent dramatically improves the aqueous solubility of the parent cyclodextrin. Reversible, non-covalent, complexation of drugs with the Captisol cyclodextrin generally allows for increased solubility and stability of drugs in aqueous solutions.

The preferred weight ratio of cyclodextrin to compound (Ia) is from 1:100 to 5,000:1. A preferred formulation is 1 g BA to 25 g cyclodextrin. About 25% cyclodextrin is most preferred. Also, 40% cyclodextrin is suitable.

Another aspect of the present invention is a pharmaceutical composition comprising a nitrobenzamide compound and a surfactant, which has enhanced solubility over that of the nitrobenzamide compound alone.

Surfactants are compounds that have surface active properties. Surfactants are amphiphilic molecules which are manufactured by chemical processes or purified from natural sources or processes. These can be anionic, cationic, nonionic, and zwitterionic. Typical surfactants are described in Emulsions: Theory and Practice, Paul Becher, Robert E. Krieger Publishing, Malabar, Fla., 1965; Pharmaceutical Dosage Forms: Dispersed Systems Vol. I, Martin M. Rigear, Surfactants and U.S. Pat. No. 5,595,723.

The pharmaceutical compositions of the present invention comprising surfactants can result in emulsions, suspensions, or other preparations, for example, liposomal preparations, may be used. With respect to liposomal preparations, any known methods for preparing liposomes for treatment of a condition may be used. See, for example, Bangham et al., J. Mol. Biol, 23: 238-252 (1965) and Szoka et al., Proc. Natl Acad. Sci 75: 4194-4198 (1978), incorporated herein by reference. Ligands may also be attached to the liposomes to direct these compositions to particular sites of action. The surfactants of the present invention can also be a wetting agent (e.g., lecithin, lysolecithin and/or a long-chain fatty alcohol).

One group of preferred surfactants for the present invention are non-ionic surfactants. Examples of useful non-ionic surfactants are Poloxamers or pluronics, which are synthetic block copolymers of ethylene oxide and propylene oxide having the general structure: H(OCH₂ CH₂)ₐ (OCH₂CH₂CH₂)_{b} (OCH₂CH₂)ₐ OH.

The following variants based on the values of a and b are commercially available from BASF Performance Chemicals (Parsippany, N.J.) under the trade name Pluronic and which consist of the group of surfactants designated by the CTFA (Cosmetic, toiletry, and fragrance association) name of Poloxamer 108, 188, 217, 237, 238, 288, 338, 407, 101, 105, 122, 123, 124, 181, 182, 183, 184, 212, 231, 282, 331, 401, 402, 185, 215, 234, 235, 284, 333, 334, 335, and 403. For the most commonly used poloxamers 124, 188, 237, 338 and 407 the values of a and b are 12/20, 79/28, 64/37, 141/44 and 101/56, respectively.

The pharmaceutical compositions of the present invention may also comprise the surfactant Solutol HS-15 which is a polyethylene glycol 660 hydroxystearate manufactured by BASF.

The pharmaceutical compositions of the present invention also comprise surfactants selected from a group of non-ionic surfactants including, without limitation thereto, polyoxyethylene sorbitan fatty acid esters such as Polysorbates 20, 60 and 80; polyoxyethylene alkyl ethers such as Brij's (e.g., BRIJ 97 or BRIJ 98 from ICI Surfactants, Cremophors (such as ***Cremophor*** RH or ***Cremophor*** EL), Volpo (e.g., VOLPO 10 and VOLPO 20 from Croda, Inc.,) and equivalents thereof.

Hydrophile-lipophile balance: An empirical formula used to index surfactants. Its value varies from 1-45 and in the case of non-ionic surfactants from about 1-20. In general for lipophilic surfactants the HLB is less than 10 and for hydrophilic ones the HLB is greater than 10. Suitable formulations include Polysorbate 80 and 20 at 100% and 10%, cremophor solutions at 10%, and Solutol at 25% and 30%.

Preferred surfactants for the present invention are polyethylene sorbitan monooleate (Polysorbate 80), polyoxyethylene [20] sorbitan monolaurate (Polysorbate 20), Cremophor EL (BASF), Cremophor RH40 (BASF), Poloxamer 118, and Solutol HS-15 (BASF).

The pharmaceutical compositions of the present invention may also comprise co-solvents. Co-solvents are at least partially miscible with water and can result in increased solubility of the nitrobenzamide compound.

In a preferred embodiment, the co-solvent comprises ethanol, glycofurol, glycerin formal, benzyl alcohol, PEG 400, propylene glycol, or N, N-dimethyl acetamide (DMA).

The preferred weight ratio of co-solvent to compound (Ia) in the pharmaceutical composition is from 1:100 to 10,000:1. Most preferred range is about 1:50 to about 1:250.

It is desirable that the pharmaceutical compositions of the invention are biocompatible, meaning that they are capable of performing functions within or upon a living organism in an acceptable manner, without undue toxicity or physiological or pharmacological effects.

The pharmaceutical compositions of the present comprise mixtures of the solubilizers including combinations of cyclodextrins, surfactants, and/or co-solvents. These mixtures can comprise cyclodextrins and surfactants, cyclodextrins and co-solvents, surfactants and co-solvents, and mixtures of cyclodextrins, surfactants, and co-solvents. The compositions can also comprise mixtures of each of the types of solubilizer such as more than one type of cyclodextrin, more than one type of surfactant, and/or more than one type of co-solvent.

Preferred mixtures of solubilizers include ethanol and PEG 400, ethanol and PEG 400 and benzyl alcohol, glycofurol and PEG 400, DMA and PEG 400, DMA, ethanol, and PEG 400, DMA and Solutol HS-15, Polysorbate 80 and ethanol, Polysorbate 20 and ethanol, Polysorbate 80 and glycofurol, Polysorbate 20 and glycofurol, and Polysorbate 80 and ethanol and PEG 400.

### Formulations, Routes of Administration, and Effective Doses

Another aspect of the invention is the use of the pharmaceutical compositions as an anti-viral or an anti-tumor treatment. The pharmaceutical compositions of the present invention may be provided as a prodrug and/or may be allowed to interconvert to a nitrosobenzamide form *in vivo* after administration. That is, either the nitrobenzamide form and/or the nitrosobenzamide form, or pharmaceutically acceptable salts may be used in developing a formulation for use in the present invention. Further, in some embodiments, the compound may be used in combination with one or more other compounds or in one or more other forms. For example a formulation may comprise both the nitrobenzamide compound and acid forms in particular proportions, depending on the relative potencies of each and the intended indication. The two forms may be formulated together, in the same dosage unit e.g. in one cream, suppository, tablet, capsule, or packet of powder to be dissolved in a beverage; or each form may be formulated in a separate unit, e.g., two creams, two suppositories, two tablets, two capsules, a tablet and a liquid for dissolving the tablet, a packet of powder and a liquid for dissolving the powder, etc.

The pharmaceutical compositions of the present invention can be combined with other active ingredients. The two compounds and/or forms of a compound may be formulated together, in the same dosage unit e.g. in one cream, suppository, tablet, capsule, or packet of powder to be dissolved in a beverage; or each form may be formulated in separate units, e.g., two creams, suppositories, tablets, two capsules, a tablet and a liquid for dissolving the tablet, a packet of powder and a liquid for dissolving the powder, etc.

The term "pharmaceutically acceptable salt" means those salts which retain the biological effectiveness and properties of the compounds used in the present invention, and which are not biologically or otherwise undesirable. For example, a pharmaceutically acceptable salt does not interfere with the beneficial effect of the compound of the invention in treating a cancer or a virus.

Typical salts are those of the inorganic ions, such as, for example, sodium, potassium, calcium and magnesium ions. Such salts include salts with inorganic or organic acids, such as hydrochloric acid, hydrobromic acid, phosphoric acid, nitric acid, sulfuric acid, methanesulfonic acid, p-toluenesulfonic acid, acetic acid, fumaric acid, succinic acid, lactic acid, mandelic acid, malic acid, citric acid, tartaric acid or maleic acid. In addition, if the compounds used in the present invention contain a carboxy group or other acidic group, it may be converted into a pharmaceutically acceptable addition salt with inorganic or organic bases. Examples of suitable bases include sodium hydroxide, potassium hydroxide, ammonia, cyclohexylamine, dicyclohexyl-amine, ethanolamine, diethanolamine and triethanolamine.

For oral administration, the pharmaceutical compounds of the present invention can be formulated readily by combining the active compound(s) with pharmaceutically acceptable carriers well known in the art. Such carriers enable the compounds of the invention to be formulated as tablets, including chewable tablets, pills, dragees, capsules, lozenges, hard candy, liquids, gels, syrups, slurries, powders, suspensions, elixirs, wafers, and the like, for oral ingestion by a patient to be treated. Such formulations can comprise pharmaceutically acceptable carriers including solid diluents or fillers, sterile aqueous media and various non-toxic organic solvents. Generally, the compounds of the invention will be included at concentration levels ranging from about 0.5%, about 5%, about 10%, about 20%, or about 30% to about 50%, about 60%, about 70%, about 80% or about 90% by weight of the total composition of oral dosage forms, in an amount sufficient to provide a desired unit of dosage.

Aqueous suspensions of the pharmaceutical composition of the invention may contain pharmaceutically acceptable excipients, such as a suspending agent (e.g., methyl cellulose), as well as coloring agents, preservatives, flavoring agents, and the like.

Compounds of this invention may also be integrated into foodstuffs, e.g., cream cheese, butter, salad dressing, or ice cream to facilitate solubilization, administration, and/or compliance in certain patient populations.

Pharmaceutical preparations for oral use can be obtained as a solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; flavoring elements, cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinyl pyrrolidone (PVP). If desired, disintegrating agents may be added, such as the cross-linked polyvinyl pyrrolidone, agar, or alginic acid or a salt thereof such as sodium alginate. The compounds may also be formulated as a sustained release preparation.

Dragee cores can be provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations that can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for administration.

For injection, the inhibitors of the present invention may be formulated in aqueous solutions, preferably in physiologically compatible buffers such as Hank's solution, Ringer's solution, or physiological saline buffer. Such compositions may also include one or more excipients, for example, preservatives, solubilizers, fillers, lubricants, stabilizers, albumin, and the like. Methods of formulation are known in the art, for example, as disclosed in Remington's Pharmaceutical Sciences, latest edition, Mack Publishing Co., Easton, PA. These compounds may also be formulated for transmucosal administration, buccal administration, for administration by inhalation, for parental administration, for transdermal administration, and rectal administration.

In addition to the formulations described previously, the compounds may also be formulated as a depot preparation. Such long acting formulations may be administered by implantation or transcutaneous delivery (for example subcutaneously or intramuscularly), intramuscular injection or use of a transdermal patch. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

Pharmaceutical compositions suitable for use in the present invention include compositions wherein the active ingredients are present in an effective amount, i.e., in an amount effective to achieve therapeutic and/or prophylactic benefit in at least one of the cancers described herein. The actual amount effective for a particular application will depend on the condition or conditions being treated, the condition of the subject, the formulation, and the route of administration, as well as other factors known to those of skill in the art. Determination of an effective amount of a nitrobenzamide compound is well within the capabilities of those skilled in the art, in light of the disclosure herein, and will be determined using routine optimization techniques.

### THERAPEUTIC USE OF PARP INHIBITORS

### Cancer types

The invention provides methods to treat several specific cancers or tumors. For example, cancer types include adrenal cortical cancer, anal cancer, aplastic anemia, bile duct cancer, bladder cancer, bone cancer, bone metastasis, Adult CNS brain tumors, Children CNS brain tumors, breast cancer, Castleman Disease, cervical cancer, Childhood Non-Hodgkin's lymphoma, colon and rectum (colorectal) cancer, endometrial cancer, esophagus cancer, Ewing's family of tumors, eye cancer, gallbladder cancer, gastrointestinal carcinoid tumors, gastrointestinal stromal tumors, gestational trophoblastic disease, Hodgkin's disease, Kaposi's sarcoma, kidney cancer, laryngeal and hypopharyngeal cancer, acute lymphocytic leukemia, acute myeloid leukemia, children's leukemia, chronic lymphocytic leukemia, chronic myeloid leukemia, liver cancer, lung cancer, lung carcinoid tumors, Non-Hodgkin's lymphoma, male breast cancer, malignant mesothelioma, multiple myeloma, myelodysplastic syndrome, nasal cavity and paranasal cancer, nasopharyngeal cancer, neuroblastoma, oral cavity and oropharyngeal cancer, osteosarcoma, ovarian cancer, pancreatic cancer, penile cancer, pituitary tumor, prostate cancer, retinoblastoma, rhabdomyosarcoma, salivary gland cancer, sarcoma (adult soft tissue cancer), melanoma skin cancer, non-melanoma skin cancer, stomach cancer, testicular cancer, thymus cancer, thyroid cancer, uterine sacrcoma, vaginal cancer, vulvar cancer, Waldenstrom's macroglobulinemia, cancers of viral origin and virus-associated cancers.

Carcinoma of the thyroid gland is the most common malignancy of the endocrine system. Carcinoma of the thyroid gland include differentiated tumors (papillary or follicular) and poorly differentiated tumors (medullary or anaplastic). Carcinomas of the vagina include squamous cell carcinoma, adenocarcinoma, melanoma and sarcoma. Testicular cancer is broadly divided into seminoma and nonseminoma types.

Thymomas are epithelial tumors of the thymus, which may or may not be extensively infiltrated by nonneoplastic lymphocytes. The term thymoma is customarily used to describe neoplasms that show no overt atypia of the epithelial component. A thymic epithelial tumor that exhibits clear-cut cytologic atypia and histologic features no longer specific to the thymus is known as a thymic carcinoma (also known as type C thymoma).

The methods provided by the invention may comprise the administration of the benzamide compounds in combination with other therapies. The choice of therapy that can be co-administered with the compositions of the invention will depend, in part, on the condition being treated. For example, for treating acute myleoid leukemia, a benzamide compound of some embodiments of the invention can be used in combination with radiation therapy, monoclonal antibody therapy, chemotherapy, bone marrow transplantation, gene therapy, immunotherapy, or a combination thereof.

### Breast Cancer

In one aspect, the invention provides a method of treating breast cancer, preferably a ductal carcinoma in duct tissue in a mammary gland.

Several types of breast cancer exist that may be treated by the methods provided by the invention. A lobular carcinoma in situ and a ductal carcinoma in situ are breast cancers that have developed in the lobules and ducts, respectively, but have not spread to the fatty tissue surrounding the breast or to other areas of the body. An infiltrating (or invasive) lobular and a ductal carcinoma are cancers that have developed in the lobules and ducts, respectively, and have spread to either the breast's fatty tissue and/or other parts of the body. Other cancers of the breast that would benefit from treatment by the methods provided by the invention are medullary carcinomas, colloid carcinomas, tubular carcinomas, and inflammatory breast cancer.

Treatments available for breast cancer patients are surgery, immunotherapy, radiation therapy, chemotherapy, endocrine therapy, or a combination thereof. A lumpectomy and a mastectomy are two possible surgical procedures available for breast cancer patients.

Chemotherapy utilizes anti-tumor agents to prevent cancer cells from multiplying, invading, metastasizing and killing a patient. Several drugs are available to treat breast cancer, including cytotoxic drugs such as doxorubicin, cyclophosphamide, methotrexate, paclitaxel, thiotepa, mitoxantrone, vincristine, or combinations thereof. Endocrine therapy may be an effective treatment where the remaining breast tissue retains endocrine sensitivity. Agents administered for this therapy include tamoxifen, megestrol acetate, aminoglutethimide, fluoxymesterone, leuprolide, goserelin, and prednisone.

The methods provided by the invention can provide a beneficial effect for breast cancer patients, by administration of a nitrobenzamide compound or a combination of administration of a nitrobenzamide compound and surgery, radiation therapy, chemotherapy, or endocrine therapy.

### Ovarian cancer

In another aspect, the invention provides a method of treating ovarian cancer, including epithelial ovarian tumors. Preferably, the invention provides a method of treating an ovarian cancer selected from the following: an adenocarcinoma in the ovary and an adenocarcinoma that has migrated from the ovary into the abdominal cavity. Surgery, immunotherapy, chemotherapy, hormone therapy, radiation therapy, or a combination thereof are some possible treatments available for ovarian cancer. Some possible surgical procedures include debulking, and a unilateral or bilateral oophorectomy and/or a unilateral or bilateral salpigectomy.

Anti-cancer drugs that may be used include cyclophosphamide, etoposide, altretamine, and ifosfamide. Hormone therapy with the drug tamoxifen may be used to shrink ovarian tumors. Radiation therapy may be external beam radiation therapy and/or brachytherapy.

The methods provided by the invention can provide a beneficial effect for ovarian cancer patients, by administration of a nitrobenzamide compound or a combination of administration of a nitrobenzamide compound and surgery, radiation therapy, chemotherapy endocrine therapy, or a combination thereof.

### Cervical Cancer

In another aspect, the invention provides a method of treating cervical cancer, preferably an adenocarcinoma in the cervix epithelial. Two main types of this cancer exist: squamous cell carcinoma and adenocarcinomas. The former constitutes about 80-90% of all cervical cancers and develops where the ectocervix (portion closest to the vagina) and the endocervix (portion closest to the uterus) join. The latter develop in the mucous-producing gland cells of the endocervix. Some cervical cancers have characteristics of both of these and are called adenosquamous carcinomas or mixed carcinomas.

The chief treatments available for cervical cancer are surgery, immunotherapy, radiation therapy and chemotherapy. Some possible surgical options are cryosurgery, a hysterectomy, and a radical hysterectomy. Radiation therapy for cervical cancer patients includes external beam radiation therapy or brachytherapy. Anti-cancer drugs that may be administered as part of chemotherapy to treat cervical cancer include cisplatin, carboplatin, hydroxyurea, irinotecan, bleomycin, vincrinstine, mitomycin, ifosfamide, fluorouracil, etoposide, methotrexate, and combinations thereof.

The methods provided by the invention can provide a beneficial effect for cervical cancer patients, by administration of a nitrobenzamide compound or a combination of administration of a nitrobenzamide compound and surgery, radiation therapy, chemotherapy, or a combination thereof.

### Prostate Cancer

In one other aspect, the invention provides methods to treat prostate cancer, preferably a prostate cancer selected from the following: an adenocarcinoma or an adenocarinoma that has migrated to the bone. Prostate cancer develops in the prostate organ in men, which surrounds the first part of the urethra. The prostate has several cell types but 99% of tumors are adenocarcinomas that develop in the glandular cells responsible for generating seminal fluid.

Surgery, immunotherapy, radiation therapy, cryosurgery, hormone therapy, and chemotherapy are some treatments available for prostate cancer patients. Possible surgical procedures to treat prostate cancer include radical retropubic prostatectomy, a radical perineal prostatectomy, and a laparoscopic radical prostatectomy. Some radiation therapy options are external beam radiation, including three dimensional conformal radiation therapy, intensity modulated radiation therapy, and conformal proton beam radiation therapy. Brachytherapy (seed implantation or interstitial radiation therapy) is also an available method of treatment for prostate cancer. Cryosurgery is another possible method used to treat localized prostate cancer cells.

Hormone therapy, also called androgen deprivation therapy or androgen suppression therapy, may be used to treat prostate cancer. Several methods of this therapy are available including an orchiectomy in which the testicles, where 90% of androgens are produced, are removed. Another method is the administration of luteinizing hormone-releasing hormone (LHRH) analogs to lower androgen levels. The LHRH analogs available include leuprolide, goserelin, triptorelin, and histrelin. An LHRH antagonist may also be administered, such as abarelix.

Treatment with an anti-androgen agent, which blocks androgen activity in the body, is another available therapy. Such agents include flutamide, bicalutamide, and nilutamide. This therapy is typically combined with LHRH analog administration or an orchiectomy, which is termed a combined androgen blockade (CAB).

Chemotherapy may be appropriate where a prostate tumor has spread outside the prostate gland and hormone treatment is not effective. Anti-cancer drugs such as doxorubicin, estramustine, etoposide, mitoxantrone, vinblastine, paclitaxel, docetaxel, carboplatin, and prednisone may be administered to slow the growth of prostate cancer, reduce symptoms and improve the quality of life.

The methods provided by the invention can provide a beneficial effect for prostate cancer patients, by administration of a nitrobenzamide compound or a combination of administration of a nitrobenzamide compound and surgery, radiation therapy, chemotherapy, hormone therapy, or a combination thereof.

### Pancreatic Cancer

Some embodiments provide methods of treating pancreatic cancer, preferably a pancreatic cancer selected from the following: an epitheliod carcinoma in the pancreatic duct tissue and an adenocarcinoma in a pancreatic duct.

The most common type of pancreatic cancer is an adenocarcinoma, which occurs in the lining of the pancreatic duct. The possible treatments available for pancreatic cancer are surgery, immunotherapy, radiation therapy, and chemotherapy. Possible surgical treatment options include a distal or total pancreatectomy and a pancreaticoduodenectomy (Whipple procedure).

Radiation therapy may be an option for pancreatic cancer patients, specifically external beam radiation where radiation is focused on the tumor by a machine outside the body. Another option is intraoperative electron beam radiation administered during an operation.

Chemotherapy may be used to treat pancreatic cancer patients. Appropriate anti-cancer drugs include 5-fluorouracil (5-FU), mitomycin, ifosfamide, doxorubicin, streptozocin, chlorozotocin, and combinations thereof.

The methods provided by the invention can provide a beneficial effect for pancreatic cancer patients, by administration of a nitrobenzamide compound or a combination of administration of a nitrobenzamide compound and surgery, radiation therapy, or chemotherapy.

### Bladder Cancer

Some embodiments provide methods of treating bladder cancer, preferably a transitional cell carcinoma in urinary bladder. Bladder cancers are urothelial carcinomas (transitional cell carcinomas) or tumors in the urothelial cells that line the bladder. The remaining cases of bladder cancer are squamous cell carcinomas, adenocarcinomas, and small cell cancers. Several subtypes of urothelial carcinomas exist depending on whether they are noninvasive or invasive and whether they are papillary, or flat. Noninvasive tumors are in the urothelium, the innermost layer of the bladder, while invasive tumors have spread from the urothelium to deeper layers of the bladder's main muscle wall. Invasive papillary urothelial carcinomas are slender finger-like projections that branch into the hollow center of the bladder and also grow outward into the bladder wall. Non-invasive papillary urothelial tumors grow towards the center of the bladder. While a non-invasive, flat urothelial tumor (also called a flat carcinoma in situ) is confined to the layer of cells closest to the inside hollow part of the bladder, an invasive flat urothelial carcinoma invades the deeper layer of the bladder, particularly the muscle layer.

To treat bladder cancer, surgery, radiation therapy, immunotherapy, chemotherapy, or a combination thereof may be applied. Some possible surgical options are a transurethral resection, a cystectomy, or a radical cystectomy. Radiation therapy for bladder cancer may include external beam radiation and brachytherapy.

Immunotherapy is another method that may be used to treat a bladder cancer patient. Typically this is accomplished intravesically, which is the administration of a treatment agent directly into the bladder by way of a catheter. One method is Bacillus Calmete-Guerin (BCG) where a bacterium sometimes used in tuberculosis vaccination is given directly to the bladder through a catheter. The body mounts an immune response to the bacterium, thereby attacking and killing the cancer cells.

Another method of immunotherapy is the administration of interferons, glycoproteins that modulate the immune response. Interferon alpha is often used to treat bladder cancer.

Anti-cancer drugs that may be used in chemotherapy to treat bladder cancer include thitepa, methotrexate, vinblastine, doxorubicin, cyclophosphamide, paclitaxel, carboplatin, cisplatin, ifosfamide, gemcitabine, or combinations thereof.

The methods provided by the invention can provide a beneficial effect for bladder cancer patients, by administration of a nitrobenzamide compound or a combination of administration of a nitrobenzamide compound and surgery, radiation therapy, immunotherapy, chemotherapy, or a combination thereof.

### Acute Myeloid Leukemia

Some embodiments provide methods of treating acute myeloid leukemia (AML), preferably acute promyleocytic leukemia in peripheral blood. AML begins in the bone marrow but can spread to other parts of the body including the lymph nodes, liver, spleen, central nervous system, and testes. It is acute meaning it develops quickly and may be fatal if not treated within a few months. AML is characterized by immature bone marrow cells usually granulocytes or monocytes, which continue to reproduce and accumulate.

AML maybe treated by immunotherapy, radiation therapy, chemotherapy, bone marrow or peripheral blood stem cell transplantation, or a combination thereof. Radiation therapy includes external beam radiation and may have side effects. Anti-cancer drugs that may be used in chemotherapy to treat AML include cytarabine, anthracycline, anthracenedione, idarubicin, daunorubicin, idarubicin, mitoxantrone, thioguanine, vincristine, prednisone, etoposide, or a combination thereof.

Monoclonal antibody therapy may be used to treat AML patients. Small molecules or radioactive chemicals may be attached to these antibodies before administration to a patient in order to provide a means of killing leukemia cells in the body. The monoclonal antibody, gemtuzumab ozogamicin, which binds CD33 on AML cells, may be used to treat AML patients unable to tolerate prior chemotherapy regimens.

Bone marrow or peripheral blood stem cell transplantation may be used to treat AML patients. Some possible transplantation procedures are an allogenic or an autologous transplant.

The methods provided by the invention can provide a beneficial effect for leukemia patients, by administration of a nitrobenzamide compound or a combination of administration of a nitrobenzamide compound and surgery, radiation therapy, chemotherapy, or transplantation therapy.

There are other types of leukemia's that can also be treated by the methods provided by the invention including but not limited to, Acute Lymphocytic Leukemia, Acute Myeloid Leukemia, Chronic Lymphocytic Leukemia, Chronic Myeloid Leukemia, Hairy Cell Leukemia, Myelodysplasia, and Myeloproliferative Disorders.

### Lung Cancer

Some embodiments provide methods to treat lung cancer. The most common type of lung cancer is non-small cell lung cancer (NSCLC), which accounts for approximately 80-85% of lung cancers and is divided into squamous cell carcinomas, adenocarcinomas, and large cell undifferentiated carcinomas. Small cell lung cancer accounts for 15-20% of lung cancers.

Treatment options for lung cancer include surgery, immunotherapy, radiation therapy, chemotherapy, photodynamic therapy, or a combination thereof. Some possible surgical options for treatment of lung cancer are a segmental or wedge resection, a lobectomy, or a pneumonectomy. Radiation therapy may be external beam radiation therapy or brachytherapy.

Some anti-cancer drugs that may be used in chemotherapy to treat lung cancer include cisplatin, carboplatin, paclitaxel, docetaxel, gemcitabine, vinorelbine, irinotecan, etoposide, vinblastine, gefitinib, ifosfamide, methotrexate, or a combination thereof. Photodynamic therapy (PDT) may be used to treat lung cancer patients.

The methods described herein can provide a beneficial effect for lung cancer patients, by administration of a nitrobenzamide compound or a combination of administration of a nitrobenzamide compound and surgery, radiation therapy, chemotherapy, photodynamic therapy, or a combination thereof.

### Skin Cancer

Some embodiments provide methods of treating skin cancer. There are several types of cancer that start in the skin. The most common types are basal cell carcinoma and squamous cell carcinoma, which are non-melanoma skin cancers. Actinic keratosis is a skin condition that sometimes develops into squamous cell carcinoma. Non-melanoma skin cancers rarely spread to other parts of the body. Melanoma, the rarest form of skin cancer, is more likely to invade nearby tissues and spread to other parts of the body. Different types of treatment are available for patients with non-melanoma and melanoma skin cancer and actinic keratosis including surgery, radiation therapy, chemotherapy and photodynamic therapy. Some possible surgical options for treatment of skin cancer are mohs micrographic surgery, simple excision, electrodesiccation and curettage, cryosurgery, laser surgery. Radiation therapy may be external beam radiation therapy or brachytherapy. Other types of treatments that are being tested in clinical trials are biologic therapy or immunotherapy, chemoimmunotherapy, topical chemotherapy with fluorouracil and photodynamic therapy.

The methods provided by the invention can provide a beneficial effect for skin cancer patients, by administration of a nitrobenzamide compound or a combination of administration of a nitrobenzamide compound and surgery, radiation therapy, chemotherapy, photodynamic therapy, or a combination thereof.

### Eye Cancer, Retinoblastoma

Some embodiments provide methods to treat eye retinoblastoma. Retinoblastoma is a malignant tumor of the retina. Although retinoblastoma may occur at any age, it most often occurs in younger children, usually before the age of 5 years. The tumor may be in one eye only or in both eyes. Retinoblastoma is usually confined to the eye and does not spread to nearby tissue or other parts of the body. Treatment options that attempt to cure the patient and preserve vision include enucleation (surgery to remove the eye), radiation therapy, cryotherapy, photocoagulation, immunotherapy, thermotherapy and chemotherapy. Radiation therapy may be external beam radiation therapy or brachytherapy.

The methods provided by the invention can provide a beneficial effect for eye retinoblastoma patients, by administration of a nitrobenzamide compound or a combination of administration of a nitrobenzamide compound and surgery, radiation therapy, cryotherapy, photocoagulation, thermotherapy and chemotherapy, or a combination thereof.

### Eye Cancer, Intraocular Melanoma

Some embodiments provide methods to treat intraocular (eye) melanoma. Intraocular melanoma, a rare cancer, is a disease in which cancer cells are found in the part of the eye called the uvea. The uvea includes the iris, the ciliary body, and the choroid. Intraocular melanoma occurs most often in people who are middle aged. Treatments for intraocular melanoma include surgery, immunotherapy, radiation therapy and laser therapy. Surgery is the most common treatment of intraocular melanoma. Some possible surgical options are iridectomy, iridotrabeculectomy, iridocyclectomy, choroidectomy, enucleation and orbital exenteration. Radiation therapy may be external beam radiation therapy or brachytherapy. Laser therapy may be an intensely powerful beam of light to destroy the tumor, thermotherapy or photocoagulation.

The methods provided by the invention can provide a beneficial effect for intraocular melanoma patients, by administration of a nitrobenzamide compound or a combination of administration of a nitrobenzamide compound and surgery, radiation therapy and laser therapy, or a combination thereof.

### Endometrium Cancer

Some embodiments provide methods of treating endometrium cancer. Endometrial cancer is a cancer that starts in the endometrium, the inner lining of the uterus. Some of the examples of the cancer of uterus and endometrium include, but are not limited to, adenocarcinomas, adenoacanthomas, adenosquamous carcinomas, papillary serous adenocarcinomas, clear cell adenocarcinomas, uterine sarcomas, stromal sarcomas, malignant mixed mesodermal tumors, and leiomyosarcomas.

The methods provided by the invention can provide a beneficial effect for endometrium cancer patients, by administration of a nitrobenzamide compound or a combination of administration of a nitrobenzamide compound and surgery, radiation therapy, chemotherapy, gene therapy, photodynamic therapy, antiangiogenesis therapy, and immunotherapy, or a combination thereof.

### Liver Cancer

Some embodiments provide methods to treat primary liver cancer (cancer that begins in the liver). Primary liver cancer can occur in both adults and children. Different types of treatments are available for patients with primary liver cancer. These include surgery, immunotherapy, radiation therapy, chemotherapy and percutaneous ethanol injection. The types of surgery that may be used are cryosurgery, partial hepatectomy, total hepatectomy and radiofrequency ablation. Radiation therapy may be external beam radiation therapy, brachytherapy, radiosensitizers or radiolabel antibodies. Other types of treatment include hyperthermia therapy and immunotherapy.

The methods provided by the invention can provide a beneficial effect for liver cancer patients, by administration of a nitrobenzamide compound or a combination of administration of a nitrobenzamide compound and surgery, radiation therapy, chemotherapy, percutaneous ethanol injection, hyperthermia therapy and immunotherapy, or a combination thereof.

### Kidney Cancer

Some embodiments provide methods to treat kidney cancer. Kidney cancer (also called renal cell cancer or renal adenocarcinoma) is a disease in which malignant cells are found in the lining of tubules in the kidney. Kidney cancer may be treated by surgery, radiation therapy, chemotherapy and immunotherapy. Some possible surgical options to treat kidney cancer are partial nephrectomy, simple nephrectomy and radical nephrectomy. Radiation therapy may be external beam radiation therapy or brachytherapy. Stem cell transplant may be used to treat kidney cancer.

The methods provided by the invention can provide a beneficial effect for kidney cancer patients, by administration of a nitrobenzamide compound or a combination of administration of a nitrobenzamide compound and surgery, radiation therapy, chemotherapy, immunotherapy and stem cell transplant, or a combination thereof.

### Thyroid Cancer

Some embodiments provide methods of treating thyroid cancer. Thyroid cancer is a disease in which cancer (malignant) cells are found in the tissues of the thyroid gland. The four main types of thyroid cancer are papillary, follicular, medullary and anaplastic. Thyroid cancer may be treated by surgery, immunotherapy, radiation therapy, hormone therapy and chemotherapy. Surgery is the most common treatment of thyroid cancer. Some possible surgical options for treatment of thyroid cancer are lobectomy, near-total thyroidectomy, total thyroidectomy and lymph node dissection. Radiation therapy may be external radiation therapy or may required intake of a liquid that contains radioactive iodine. Hormone therapy uses hormones to stop cancer cells from growing. In treating thyroid cancer, hormones can be used to stop the body from making other hormones that might make cancer cells grow.

The methods provided by the invention can provide a beneficial effect for thyroid cancer patients, by administration of a nitrobenzamide compound or a combination of administration of a nitrobenzamide compound and surgery, surgery, radiation therapy, hormone therapy and chemotherapy, or a combination thereof.

### AIDS Related Cancers

### AIDS-Related Lymphoma

Some embodiments provide methods of treating AIDS-related lymphoma. AIDS-related lymphoma is a disease in which malignant cells form in the lymph system of patients who have acquired immunodeficiency syndrome (AIDS). AIDS is caused by the human immunodeficiency virus (HIV), which attacks and weakens the body's immune system. The immune system is then unable to fight infection and diseases that invade the body. People with HIV disease have an increased risk of developing infections, lymphoma, and other types of cancer. Lymphomas are cancers that affect the white blood cells of the lymph system. Lymphomas are divided into two general types: Hodgkin's lymphoma and non-Hodgkin's lymphoma. Both Hodgkin's lymphoma and non-Hodgkin's lymphoma may occur in AIDS patients, but non-Hodgkin's lymphoma is more common. When a person with AIDS has non-Hodgkin's lymphoma, it is called an AIDS-related lymphoma. Non-Hodgkin's lymphomas may be indolent (slow-growing) or aggressive (fast-growing). AIDS-related lymphoma is usually aggressive. The three main types of AIDS-related lymphoma are diffuse large B-cell lymphoma, B-cell immunoblastic lymphoma and small non-cleaved cell lymphoma.

Treatment of AIDS-related lymphoma combines treatment of the lymphoma with treatment for AIDS. Patients with AIDS have weakened immune systems and treatment can cause further damage. For this reason, patients who have AIDS-related lymphoma are usually treated with lower doses of drugs than lymphoma patients who do not have AIDS. Highly-active antiretroviral therapy (HAART) is used to slow progression of HIV Medicine to prevent and treat infections, which can be serious, is also used. AIDS-related lymphomas may be treated by chemotherapy, immunotherapy, radiation therapy and high-dose chemotherapy with stem cell transplant. Radiation therapy may be external beam radiation therapy or brachytherapy. AIDS-related lymphomas can be treated by monoclonal antibody therapy.

The methods provided by the invention can provide a beneficial effect for AIDS-related lymphoma patients, by administration of a nitrobenzamide compound or a combination of administration of a nitrobenzamide compound and chemotherapy, radiation therapy and high-dose chemotherapy, or a combination thereof.

### Kaposi's Sarcoma

Some embodiments provide methods of treating Kaposi's sarcoma. Kaposi's sarcoma is a disease in which cancer cells are found in the tissues under the skin or mucous membranes that line the mouth, nose, and anus. Classic Kaposi's sarcoma usually occurs in older men of Jewish, Italian, or Mediterranean heritage. This type of Kaposi's sarcoma progresses slowly, sometimes over 10 to 15 years. Kaposi's sarcoma may occur in people who are taking immunosuppressants. Kaposi's sarcoma in patients who have Acquired Immunodeficiency Syndrome (AIDS) is called epidemic Kaposi's sarcoma. Kaposi's sarcoma in people with AIDS usually spreads more quickly than other kinds of Kaposi's sarcoma and often is found in many parts of the body. Kaposi's sarcoma may be treated with surgery, chemotherapy, radiation therapy and immunotherapy. External radiation therapy is a common treatment of Kaposi's sarcoma. Some possible surgical options to treat Kaposi's Sarcoma are local excision, electrodessication and curettage, and cryotherapy.

The methods provided by the invention can provide a beneficial effect for Kaposi's sarcoma, by administration of a nitrobenzamide compound or a combination of administration of a nitrobenzamide compound and surgery, chemotherapy, radiation therapy and immunotherapy, or a combination thereof.

### Viral-Induced Cancers

Some embodiments provide methods of treating viral-induced cancers. Several common viruses are clearly or probable causal factors in the etiology of specific malignancies. These viruses either normally establish latency or few can become persistent infections. Oncogenesis is probably linked to an enhanced level of viral activation in the infected host, reflecting heavy viral dose or compromised immune control. The major virus-malignancy systems include hepatitis B virus (HBV), hepatitis C virus (HCV), and hepatocellular carcinoma; human lymphotropic virus-type 1 (HTLV-1) and adult T-cell leukemia/lymphoma; and human papilloma virus (HPV) and cervical cancer. In general, these malignancies occur relatively early in life, typically peaking in middle-age or earlier.

### Virus-Induced Hepatocellular Carcinoma

The causal relationship between both HBV and HCV and hepatocellular carcinoma or liver cancer is established through substantial epidemiologic evidence. Both appear to act via chronic replication in the liver by causing cell death and subsequent regeneration. Different types of treatments are available for patients with liver cancer. These include surgery, immunotherapy, radiation therapy, chemotherapy and percutaneous ethanol injection. The types of surgery that may be used are cryosurgery, partial hepatectomy, total hepatectomy and radiofrequency ablation. Radiation therapy may be external beam radiation therapy, brachytherapy, radiosensitizers or radiolabel antibodies. Other types of treatment include hyperthermia therapy and immunotherapy.

The methods provided by the invention can provide a beneficial effect for virus induce hepatocellular carcinoma patients, by administration of a nitrobenzamide compound or a combination of administration of a nitrobenzamide compound and surgery, radiation therapy, chemotherapy, percutaneous ethanol injection, hyperthermia therapy and immunotherapy, or a combination thereof.

### Viral-Induced Adult T cell leukemia/lymphoma

The association between HTLV-1 and Adult T cell leukemia (ATL) is firmly established. Unlike the other oncogenic viruses found throughout the world, HTLV-1 is highly geographically restricted, being found primarily in southern Japan, the Caribbean, west and central Africa, and the South Pacific islands. Evidence for causality includes the monoclonal integration of viral genome in almost all cases of ATL in carriers. The risk factors for HTLV-1-associated malignancy appear to be perinatal infection, high viral load, and being male sex.

Adult T cell leukemia is a cancer of the blood and bone marrow. The standard treatments for adult T cell leukemia/lymphoma are radiation therapy, immunotherapy, and chemotherapy. Radiation therapy may be external beam radiation therapy or brachytherapy. Other methods of treating adult T cell leukemia/lymphoma include immunotherapy and high-dose chemotherapy with stem cell transplantation.

The methods provided by the invention can provide a beneficial effect for Adult T cell leukemia patients, by administration of a nitrobenzamide compound or a combination of administration of a nitrobenzamide compound and radiation therapy, chemotherapy, immunotherapy and high-dose chemotherapy with stem cell transplantation, or a combination thereof.

### Viral-Induced Cervical Cancer

Infection of the cervix with human papillomavirus (HPV) is the most common cause of cervical cancer. Not all women with HPV infection, however, will develop cervical cancer. Cervical cancer usually develops slowly over time. Before cancer appears in the cervix, the cells of the cervix go through changes known as dysplasia, in which cells that are not normal begin to appear in the cervical tissue. Later, cancer cells start to grow and spread more deeply into the cervix and to surrounding areas. The standard treatments for cervical cancers are surgery, immunotherapy, radiation therapy and chemotherapy. The types of surgery that may be used are conization, total hysterectomy, bilateral salpingo-oophorectomy, radical hysterectomy, pelvic exenteration, cryosurgery, laser surgery and loop electrosurgical excision procedure. Radiation therapy may be external beam radiation therapy or brachytherapy.

The methods provided by the invention can provide a beneficial effect for adult cervical cancer, by administration of a nitrobenzamide compound or a combination of administration of a nitrobenzamide compound and radiation therapy, chemotherapy, or a combination thereof.

### CNS cancers

Brain and spinal cord tumors are abnormal growths of tissue found inside the skull or the bony spinal column, which are the primary components of the central nervous system (CNS). Benign tumors are non-cancerous, and malignant tumors are cancerous. The CNS is housed within rigid, bony quarters (i.e., the skull and spinal column), so any abnormal growth, whether benign or malignant, can place pressure on sensitive tissues and impair function. Tumors that originate in the brain or spinal cord are called primary tumors. Most primary tumors are caused by out-of-control growth among cells that surround and support neurons. In a small number of individuals, primary tumors may result from specific genetic disease (e.g., neurofibromatosis, tuberous sclerosis) or from exposure to radiation or cancer-causing chemicals. The cause of most primary tumors remains a mystery.

The first test to diagnose brain and spinal column tumors is a neurological examination. Special imaging techniques (computed tomography, and magnetic resonance imaging, positron emission tomography) are also employed. Laboratory tests include the EEG and the spinal tap. A biopsy, a surgical procedure in which a sample of tissue is taken from a suspected tumor, helps doctors diagnose the type of tumor.

Tumors are classified according to the kind of cell from which the tumor seems to originate. The most common primary brain tumor in adults comes from cells in the brain called astrocytes that make up the blood-brain barrier and contribute to the nutrition of the central nervous system. These tumors are called gliomas (astrocytoma, anaplastic astrocytoma, or glioblastoma multiforme) and account for 65% of all primary central nervous system tumors. Some of the tumors are, but not limited to, Oligodendroglioma, Ependymoma, Meningioma, Lymphoma, Schwannoma, and Medulloblastoma.

### Neuroepithelial Tumors of the CNS

Astrocytic tumors, such as astrocytoma,; anaplastic (malignant) astrocytoma, such as hemispheric, diencephalic, optic, brain stem, cerebellar; glioblastoma multiforme; pilocytic astrocytoma, such as hemispheric, diencephalic, optic, brain stem, cerebellar; subependymal giant cell astrocytoma; and pleomorphic xanthoastrocytoma. Oligodendroglial tumors, such as oligodendroglioma; and anaplastic (malignant) oligodendroglioma. Ependymal cell tumors, such as ependymoma,; anaplastic ependymoma; myxopapillary ependymoma; and subependymoma. Mixed gliomas, such as mixed oligoastrocytoma; anaplastic (malignant) oligoastrocytoma; and others (e.g. ependymo-astrocytomas). Neuroepithelial tumors of uncertain origin, such as polar spongioblastoma; astroblastoma; and gliomatosis cerebri. Tumors of the choroid plexus, such as choroid plexus papilloma; and choroid plexus carcinoma (anaplastic choroid plexus papilloma). Neuronal and mixed neuronal-glial tumors, such as gangliocytoma; dysplastic gangliocytoma of cerebellum (Lhermitte-Duclos); ganglioglioma; anaplastic (malignant) ganglioglioma; desmoplastic infantile ganglioglioma, such as desmoplastic infantile astrocytoma; central neurocytoma; dysembryoplastic neuroepithelial tumor; olfactory neuroblastoma (esthesioneuroblastoma. Pineal Parenchyma Tumors, such as pineocytoma; pineoblastoma; and mixed pineocytoma/pineoblastoma. Tumors with neuroblastic or glioblastic elements (embryonal tumors), such as medulloepithelioma; primitive neuroectodermal tumors with multipotent differentiation, such as medulloblastoma; cerebral primitive neuroectodermal tumor; neuroblastoma; retinoblastoma; and ependymoblastoma.

### Other CNS Neoplasms

Tumors of the Sellar Region, such as pituitary adenoma; pituitary carcinoma; and craniopharyngioma. Hematopoietic tumors, such as primary malignant lymphomas; plasmacytoma; and granulocytic sarcoma. Germ Cell Tumors, such as germinoma; embryonal carcinoma; yolk sac tumor (endodermal sinus tumor); choriocarcinoma; teratoma; and mixed germ cell tumors. Tumors of the Meninges, such as meningioma; atypical meningioma; and anaplastic (malignant) meningioma. Non-menigothelial tumors of the meninges, such as Benign Mesenchymal; Malignant Mesenchymal; Primary Melanocytic Lesions; Hemopoietic Neoplasms; and Tumors of Uncertain Histogenesis, such as hemangioblastoma (capillary hemangioblastoma). Tumors of Cranial and Spinal Nerves, such as schwannoma (neurinoma, neurilemoma); neurofibroma; malignant peripheral nerve sheath tumor (malignant schwannoma), such as epithelioid, divergent mesenchymal or epithelial differentiation, and melanotic. Local Extensions from Regional Tumors; such as paraganglioma (chemodectoma); chordoma; chodroma; chondrosarcoma; and carcinoma. Metastatic tumours, Unclassified Tumors and Cysts and Tumor-like Lesions, such as Rathke cleft cyst; Epidermoid; dermoid; colloid cyst of the third ventricle; enterogenous cyst; neuroglial cyst; granular cell tumor (choristoma, pituicytoma); hypothalamic neuronal hamartoma; nasal glial herterotopia; and plasma cell granuloma.

Chemotherapeutics available are, but not limited to, alkylating agents such as, Cyclophosphamide, Ifosphamide, Melphalan, Chlorambucil, BCNU, CCNU, Decarbazine, Procarbazine, Busulfan, and Thiotepa; antimetabolites such as, Methotraxate, 5-Fluorouracil, Cytarabine, Gemcitabine (Gemzar®), 6-mercaptopurine, 6-thioguanine, Fludarabine, and Cladribine; anthracyclins such as, daunorubicin. Doxorubicin, Idarubicin, Epirubicin and Mitoxantrone; antibiotics such as, Bleomycin; camptothecins such as, irinotecan and topotecan; taxanes such as, paclitaxel and docetaxel; and platinums such as, Cisplatin, carboplatin, and Oxaliplatin.

The treatments are surgery, radiation therapy, immunotherapy, hyperthermia, gene therapy, chemotherapy, and combination of radiation and chemotherapy. Doctors also may prescribe steroids to reduce the swelling inside the CNS.

The methods provided by the invention can provide a beneficial effect for adult cervical cancer, by administration of a nitrobenzamide compound or a combination of administration of a nitrobenzamide compound and radiation therapy, chemotherapy, or a combination thereof.

### PNS Cancers

The peripheral nervous system consists of the nerves that branch out from the brain and spinal cord. These nerves form the communication network between the CNS and the body parts. The peripheral nervous system is further subdivided into the somatic nervous system and the autonomic nervous system. The somatic nervous system consists of nerves that go to the skin and muscles and is involved in conscious activities. The autonomic nervous system consists of nerves that connect the CNS to the visceral organs such as the heart, stomach, and intestines. It mediates unconscious activities.

Acoustic neuromas are benign fibrous growths that arise from the balance nerve, also called the eighth cranial nerve or vestibulocochlear nerve. These tumors are non-malignant, meaning that they do not spread or metastasize to other parts of the body. The location of these tumors is deep inside the skull, adjacent to vital brain centers in the brain stem. As the tumors enlarge, they involve surrounding structures which have to do with vital functions. In the majority of cases, these tumors grow slowly over a period of years.

The malignant peripheral nerve sheath tumor (MPNST) is the malignant counterpart to benign soft tissue tumors such as neurofibromas and schwannomas. It is most common in the deep soft tissue, usually in close proximity of a nerve trunk. The most common sites include the sciatic nerve, brachial plexus, and sarcal plexus. The most common symptom is pain which usually prompts a biopsy. It is a rare, aggressive, and lethal orbital neoplasm that usually arises from sensory branches of the trigeminal nerve in adults. Malignant PNS tumor spreads along nerves to involve the brain, and most patients die within 5 years of clinical diagnosis. The MPNST may be classified into three major categories with epithelioid, mesenchymal or glandular characteristics. Some of the MPNST include but not limited to, Subcutaneous malignant epithelioid schwannoma with cartilaginous differentiation, Glandular malignant schwannoma, Malignant peripheral nerve sheath tumor with perineurial differentiation, Cutaneous epithelioid malignant nerve sheath tumor with rhabdoid features, Superficial epithelioid MPNST, Triton Tumor (MPNST with rhabdomyoblastic differentiation), Schwannoma with rhabdomyoblastic differentiation. Rare MPNST cases contain multiple sarcomatous tissue types, especially osteosarcoma, chondrosarcoma and angiosarcoma. These have sometimes been indistinguishable from the malignant mesenchymoma of soft tissue.

Other types of PNS cancers include but not limited to, malignant fibrous cytoma, malignant fibrous histiocytoma, malignant meningioma, malignant mesothelioma, and malignant mixed Müllerian tumor.

The treatments are surgery, radiation therapy, immunotherapy, chemotherapy, and combination of radiation and chemotherapy.

The methods provided by the invention can provide a beneficial effect for PNS cancers, by administration of a nitrobenzamide compound or a combination of administration of a nitrobenzamide compound and radiation therapy, chemotherapy, or a combination thereof.

### Oral Cavity and Oropharyngeal Cancer

Management of patients with central nervous system (CNS) cancers remains a formidable task. Cancers such as, hypopharyngeal cancer, laryngeal cancer, nasopharyngeal cancer, oropharyngeal cancer, and the like, have been treated with surgery, immunotherapy, chemotherapy, combination of chemotherapy and radiation therapy. Etoposide and actinomycin D, two commonly used oncology agents that inhibit topoisomerase II, fail to cross the blood-brain barrier in useful amounts.

The methods provided by the invention can provide a beneficial effect for Oral Cavity and Oropharyngeal cancer, by administration of a nitrobenzamide compound or a combination of administration of a nitrobenzamide compound and radiation therapy, chemotherapy, or a combination thereof.

### Stomach Cancer

Stomach cancer is the result of cell changes in the lining of the stomach. There are three main types of stomach cancers: lymphomas, gastric stromal tumors, and carcinoid tumors. Lymphomas are cancers of the immune system tissue that are sometimes found in the wall of the stomach. Gastric stromal tumors develop from the tissue of the stomach wall. Carcinoid tumors are tumors of hormone-producing cells of the stomach.

The causes of stomach cancer continue to be debated. A combination of heredity and environment (diet, smoking, etc) are all thought to play a part. Common approaches to the treatment include surgery, immunotherapy, chemotherapy, radiation therapy, combination of chemotherapy and radiation therapy or biological therapy.

The methods provided by the invention can provide a beneficial effect for stomach cancer, by administration of a nitrobenzamide compound or a combination of administration of a nitrobenzamide compound and radiation therapy, chemotherapy, or a combination thereof.

### Testicular cancer

Testicular cancer is cancer that typically develops in one or both testicles in young men. Cancers of the testicle develop in certain cells known as germ cells. The 2 main types of germ cell tumors (GCTs) that occur in men are seminomas (60%) and nonseminomas (40%). Tumors can also arise in the supportive and hormone-producing tissues, or stroma, of the testicles. Such tumors are known as gonadal stromal tumors. The 2 main types are Leydig cell tumors and Sertoli cell tumors. Secondary testicular tumors are those that start in another organ and then spread to the testicle. Lymphoma is the most common secondary testicular cancer.

Common approaches to the treatment include surgery, immunotherapy, chemotherapy, radiation therapy, combination of chemotherapy and radiation therapy or biological therapy. Several drugs are typically used to treat testicular cancer: Platinol (cisplatin), Vepesid or VP-16 (etoposide) and Blenoxane (bleomycin sulfate). Additionally, Ifex (ifosfamide), Velban (vinblastine sulfate) and others may be used.

The methods provided by the invention can provide a beneficial effect for stomach cancer, by administration of a nitrobenzamide compound or a combination of administration of a nitrobenzamide compound and radiation therapy, chemotherapy, or a combination thereof.

### Thymus Cancer

The thymus is a small organ located in the upper/front portion of your chest, extending from the base of the throat to the front of the heart. The thymus contains 2 main types of cells, thymic epithelial cells and lymphocytes. Thymic epithelial cells can give origin to thymomas and thymic carcinomas. Lymphocytes, whether in the thymus or in the lymph nodes, can become malignant and develop into cancers called Hodgkin disease and non-Hodgkin lymphomas. The thymus also contains another much less common type of cells called Kulchitsky cells, or neuroendocrine cells, which normally release certain hormones. These cells can give rise to cancers, called carcinoids or carcinoid tumors that often release the same type of hormones, and are similar to other tumors arising from neuroendocrine cells elsewhere in the body.

Common approaches to the treatment include surgery, immunotherapy, chemotherapy, radiation therapy, combination of chemotherapy and radiation therapy or biological therapy. Anticancer drugs that have been used in the treatment of thymomas and thymic carcinomas are doxorubicin (adriamycin), cisplatin, ifosfamide, and corticosteroids (prednisone). Often, these drugs are given in combination to increase their effectiveness. Combinations used to treat thymic cancer include cisplatin, doxorubicin, etoposide and cyclophosphamide, and the combination of cisplatin, doxorubicin, cyclophosphamide, and vincristine.

The methods provided by the invention can provide a beneficial effect for stomach cancer, by administration of a nitrobenzamide compound or a combination of administration of a nitrobenzamide compound and radiation therapy, chemotherapy, or a combination thereof.

### EXAMPLES

### Example 1

### Baseline solubility of 4-iodo-3-nitrobenzamide in water, acid, base, and sodium chloride

Excess 4-iodo-3-nitrobenzamide ("BA") was equilibrated overnight (>16 hr) at 25° C in purified water, 0.01M HCl (pH2), 0.01M NaOH (pH 13), and 0.9% NaCl. Following dilution, the solubility of the drug was measured by HPLC. The drug solubility results are shown in Table 1.

**Table 1**

| **Media** | **Solubility (mg/ml)** |
|---|---|
| Purified Water | 0.182 |
| 0.01 M HCl (pH 2) | 0.179 |
| 0.01M NaOH (pH 13) | 0.181 |
| 0.9% NaCl | 0.164 |
| 5% Glucose (5GW) | 0.173 |

As can be seen from Table 1, the solubility of BA in water without any solubilizer is ≤ 0.2 mg/ml. For purposes of calculating enhancement of solubility of BA in water, 0.2 mg/ml is taken as the baseline solubility of BA, against which various solubilizers are tested.

### Example 2

### Solubility of 4-iodo-3-nitrobenzamide in water with cyclodextrin

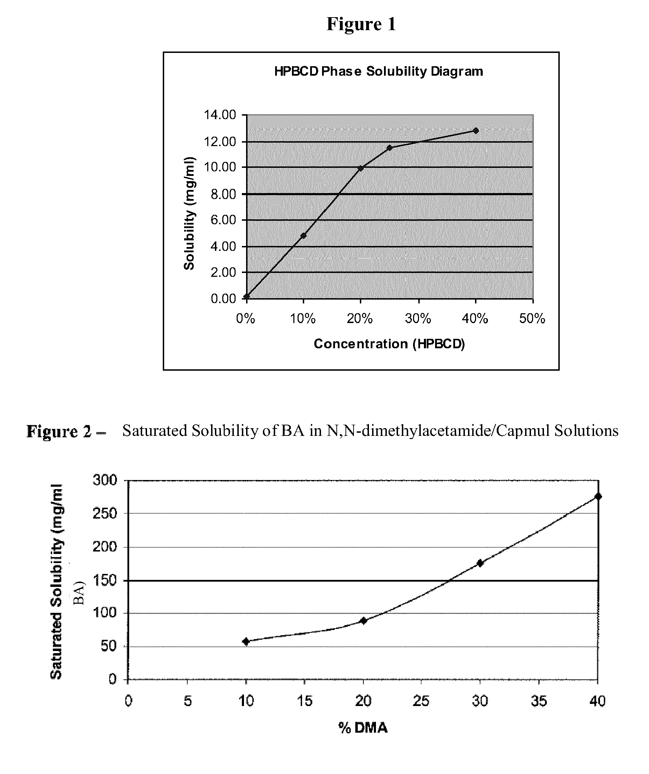

Excess 4-iodo-3-nitrobenzamide was equilibrated overnight (>16 hr) at 25° C in solutions of purified water containing various concentrations of cyclodextrins including hydroxypropyl-β-cyclodextrin (Kleptose® from Roquette) (HPBCD), sulfobutyl ether- β-cyclodextrin (Captisol® from Cydex) (SBEBCD), and Hyroxypropyl-γ-cyclodextrin (Cavamax W8® from Wacker) (HPGCD). Following dilution, the solubility of the drug was measured by HPLC. The drug solubility results are shown in Table 2. A plot of drug solubility versus concentration of HPBCD is shown in Figure 1.

**Table 2**

| **Solubilizer** | **Level** | **Solubility (mg/ml)** | **Enhancement** |
|---|---|---|---|
| None | 0% | 0.182 | 1.0 |
| HPBCD | 10% | 4.860 | 26.7 |
| HPBCD | 20% | 9.965 | 54.8 |
| HPBCD | 25% | 11.465 | 63.0 |
| HPBCD | 40% | 12.769 | 70.2 |
| HPGCD | 25% | 0.272 | 1.5 |
| SBEBCD | 25% | 11.036 | 60.6 |

### Example 3

### Solubility of 4-iodo-3-nitrobenzamide in water with surfactants

Excess 4-iodo-3-nitrobenzamide was equilibrated overnight (>16 hr) at 25° C in purified water containing various concentrations of the surfactants: polyethylene sorbitan monooleate (Polysorbate 80), polyoxyethylene [20] sorbitan monolaurate (Polysorbate 20), Cremophor EL (BASF), Cremophor RH40 (BASF), Poloxamer 118, and Solutol HS-15 (BASF). Following dilution, the solubility of the drug was measured by HPLC. The drug solubility results are shown in Table 3.

**Table 3**

| **Solubilizer** | **Level** | **Solubility (mg/ml)** | **Enhancement** |
|---|---|---|---|
| Polysorbate 80 | 100% | 16.89 | 92.8 |
| Polysorbate 20 | 100% | 10.85 | 59.6 |
| Polysorbate 80 | 10% | 3.12 | 17.2 |
| Polysorbate 20 | 10% | 3.66 | 20.1 |
| Cremophor EL | 10% | 3.50 | 19.2 |
| Cremophor RH40 | 10% | 3.37 | 18.5 |
| Poloxamer 118 | 10% | 0.43 | 2.4 |
| Solutol HS 15 | 25% | 6.55 | 36.0 |
| Solutol HS 15 | 30% | 7.46 | 41.0 |

### Example 4

### Solubility of 4-iodo-3-nitrobenzamide in water with cosolvents

Excess 4-iodo-3-nitrobenzamide was equilibrated overnight (>16 hr) at 25° C in solutions of purified water containing various co-solvents including ethanol, glycofurol, glycerin formal, benzyl alcohol, N, N, dimethylacetamide (DMA), polyethylene glycol (PEG) 400, and propylene glycol. Following dilution, the solubility of the drug was measured by HPLC. The drug solubility results are shown in Table 4.

**Table 4**

| **Solubilizer** | **Level** | **Solubility (mg/ml)** | **Enhancement** |
|---|---|---|---|
| Ethanol | 100% | 14.06 | 77.2 |
| Glycofurol | 100% | 14.97 | 82.3 |
| Glycerin Formal | 100% | 15.14 | 83.2 |
| Benzyl alcohol | 100% | 15.52 | 85.3 |
| DMA | 100% | 71.46 | 392.6 |
| Ethanol | 50% | 5.72 | 31.4 |
| Glycofurol | 50% | 9.85 | 54.1 |
| Glycerin Formal | 50% | 3.45 | 19.0 |
| PEG 400 | 50% | 7.66 | 42.1 |
| Propylene glycol | 50% | 2.24 | 12.3 |
| DMA | 5% | 1.39 | 7.6 |

### Example 5

### Solubility of 4-iodo-3-nitrobenzamide in mixed systems

Excess 4-iodo-3-nitrobenzamide was equilibrated overnight (>16 hr) at 25° C in solutions in purified water containing mixtures of solubilizers. Following dilution, the solubility of the drug was measured by HPLC. The drug solubility results are shown in Table 5.

**Table 5**

| **Solubisers** | **Solubility (mg/ml)** | **Enhancement** |
|---|---|---|
| 10% EtOH/40% PEG 400 | 3.416 | 18.8 |
| 10% EtOH/10% Polysorbate 80 | 2.3 | 11.5 |
| 10% EtOH/40% PEG 400,/5%Benzyl Alcohol | 3.111 | 17.1 |
| 10% glycofurol, 40% PEG 400 | 6.121 | 33.6 |
| 5% DMA/40% PEG 400 | 6.05 | 33.2 |
| 5% DMA/5% EtOH/40% PEG 400 | 8.01 | 44.0 |
| 5% DMA/25% Solutol HS-15 | 8.32 | 45.7 |
| 50% Polysorbate 80/50% EtOH | 18.60 | 102.2 |
| 50% Polysorbate 20/50% EtOH | 16.31 | 89.6 |
| 5% Polysorbate 80/5% ETOH | 2.23 | 12.3 |
| 5% Polysorbate 20/5% ETOH | 2.21 | 12.1 |
| 5% Polysorbate 80/5% glycofurol | 2.39 | 13.1 |
| 5% Polysorbate 20/5% glycofurol | 2.00 | 11.0 |
| 5% Polysorbate 80/10% EtOH/ 40% PEG 400 | 1.23 | 6.7 |

### Example 6

### FORMULATION STUDIES

Addition of Tonicity Adjusters: Initial studies on 10 ml volumes of solution investigated the use of sodium chloride as a tonicity adjuster since it was felt that its inclusion may reduce the level of hemolysis associated with the use of hydroxypropyl-β-cyclodextrin (HPβCD).

Osmolality Measurements: Osmolality determinations where carried out using a Roebling Freezing Point Depression Osmometer.

Since the osmolality of a 25% HPβCD solution was already 249 mOsmol/kg addition of tonicity adjusters results in the formation of a hypertonic solution (see Table 6). However addition of small quantities of tonicity adjuster may be employed to reduce the level of chemical lysis associated with some formulation excipients. Solutions that are slightly hypertonic are usually satisfactorily tolerated on intravenous administration.

pH Determination: The pH of formulations was measured to determine if these would be suitable to provide a well-tolerated formulation.

Compounding Studies (30 ml or 50 ml lots) are summarized in Table 6:

**Table 6: Formula**

| **Material** | **Percentage Formula** |
|---|---|
| BA | 1.0% w/v |
| Kleptose HPB | 25%w/v |
| Sodium Chloride | 0.6% w/v |
| Purified water | to 100% v/v |

Two trials were carried out:
1. 300 mg of BA was added to 30 ml of a 25% HPBCD (containing 0.6% saline) and mixed using a magnetic stirrer. The drug substance dissolved in 4 hours. This is deemed to be an unacceptably long time for a viable manufacturing process and it was decided to evaluate a procedure involving dissolving the drug substance directly in the HPBCD.
2. In a second study 500 mg of BA dissolved in a 50 ml of a 25% HPBCD vehicle (no saline) in approx 90 minutes. The dissolution rate of BA was deemed to be a more acceptable time and provides some prospect for a viable larger scale process. Further excipients could be added after the drug substance has been dissolved.

Taken together these observations suggest that saline may reduce the (kinetic) drug solubility possibly as a consequence of the ionic strength. Further evidence of a reduction in solubility in the presence of saline was obtained from the filtration study (below).

Filtration: The solutions described above could be satisfactorily filtered through a 0.2 µm syringe filter.

Surprisingly when solution #2 described above was filtered through a 0.2 µm PVDF disk filter precipitation occurred. This suggests that the inclusion of saline has resulted in reduction in BA solubility.

Alternative tonicity adjusters (e.g. glucose and buffers) were therefore investigated.

Tonicity adjustment with Glucose (10 ml - 25 ml volumes): Solutions of BA in 25% HPBCD were prepared containing various levels of glucose and 10 mM buffer (disodium hydrogen phosphate dodecahydrate, sodium dihydrogen phosphate dihydrate). The pH and Osmolality were determined as described previously. Results are provided in Table 7.

Solutions (approx 25 ml volumes) of the glucose and phosphate buffered solutions could be satisfactorily filtered through a 0.2 µm PVDF syringe filter.

The use of a phosphate buffer system may provide an advantage compared to an unbuffered system since a more stable pH is obtained. A further advantage is that the 10 mM phosphate buffer level provides a formulation that is virtually isotonic with blood.

### Hemolysis Studies

Summary of Procedure: The procedure involves adding 1 ml of sheep's blood to a sample (0.1 ml and 0.25 ml) of the formulation. The samples are vortex mixed and diluted with 0.9% saline, mixed for a further time and then centrifuged (1500 r.p.m.) and observed for evidence of a red color indicating the presence of hemoglobin caused by lysis. 0.1 ml of the supernatant is diluted with 2 ml of 0.9% saline and centrifuged for a further 5 minutes. The solution is then compared to a positive control (0.9% saline providing no hemolysis) and a negative control (water to provide complete hemolysis).

Results: None of the phosphate buffered or glucose adjusted solutions demonstrated evidence of hemolysis. Although a very faint pink tinge was observed in the samples, the intensity was similar to the positive saline control. During use of this procedure it has been noted that the lot of sheep's blood used can influence the degree of color in the positive control.

**Table 7: Summary of Results for Glucose and Phosphate Buffered Systems**

| Vehicle | Osmolality (mOsmol/kg) | pH | Hemolysis |
|---|---|---|---|
| 25% HPβCD (no tonicity adjusters or buffers) | 249 | 8.54 | N |
| 25% HPβCD + 2.5% glucose + no buffer | 466 | 8.03 | N |
| 25% HPβCD + 5% glucose + no buffer | 703 | 8.18 | N |
| 25% HPβCD + no glucose + 10 mM phosphate buffer | 296 | 7.45 | N |
| 25% HPβCD + 2.5% glucose + 10 mM phosphate buffer | 503 | 7.44 | N |
| 25% HPβCD + 5% glucose + 10 mM phosphate buffer | 746 | 7.40 | N |
| 25% HPβCD + 0.45% saline | 617 | ND | ND |
| 25% HPβCD + 0.6% saline | 494 | ND | ND |
| 25% HPβCD + 0.9% saline | 430 | ND | ND |

| | | | |
|---|---|---|---|
| Shaded region is currently the preferred formulation. ND = Not Determined (precipitation on filtration of saline solutions). | | | |

Conclusion: The 10 mM phosphate buffered solution is especially interesting, as: (a) The pH is similar to that of blood; (b) The pH of solutions should remain more stable on storage; (c) The osmotic pressure of the phosphate buffered formulation (296 mOsmol/kg) is similar to blood; (d) There is no need to include a separate tonicity adjuster to achieve isotonicity; (e) The phosphate buffered formulations without tonicity adjusters appear to be filterable without risk of precipitation; (f) The use of saline (causing precipitation) and glucose (potentially decomposing to form 5-HMF on autoclaving) could be avoided; It has been reported that Phosphate is allegedly not complexed by CDs hence solubility should not be affected.

### Example 7

### Oral Formulation Studies

Six capsule (oral) formulations were developed using micronized BA. Four formulations of capsules containing granulated BA were prepared as follows:
- Micronized BA without surfactants
- Micronized BA with sodium lauryl sulfate (SLS) as surfactant
- Micronized BA with Poloxamer 188 as surfactant
- Micronized BA with Polysorbate 80 as surfactant

Two formulations of capsules containing hot-melt extruded (amorphous) BA were then prepared:
- 1. Hot-melt extrusion formulation with a 30:70 BA:polymer ratio; each capsule contained 100 mg of BA
- 2. Hot-melt extrusion formulation with a 10:90 BA:polymer ratio; each capsule contained 33.3 mg of BA

Pregelatinized starch, Pharmatose DCL 21 (anhydrous lactose), microcrystalline cellulose (MCC), Polyvinylpyrrolidone K30 (Povidone K30), sodium starch glycolate, magnesium stearate and sodium lauryl sulfate were obtained from commercial sources. Four formulations were prepared by the process of wet granulation. An initial dry blending of all intra-granular materials was carried out prior to granulation. A summary of the formulations for the granulated capsules is set forth in table 7-1, below.

**Table 7-1: Granule Formulations**

| | **FT06212** | **FT06213** | **FT06214** | **FT06215** |
|---|---|---|---|---|
| Intra-granular % w/w | | | | |
| BA (micronized) | 40.0 | 40.0 | 40.0 | 40.0 |
| Pregelatinized starch | 32.5 | 31.5 | 22.5 | 31.5 |
| Microcrystalline Cellulose | 20.0 | 20.0 | 20.0 | 20.0 |
| Polyvinylpyrrolidone K30 | 2.5 | 2.5 | 2.5 | 2.5 |
| Sodium Starch Glycollate | 2.0 | 2.0 | 2.0 | 2.0 |
| Sodium Lauryl Sulfate | - | 1.0 | - | - |
| Poloxamer 188 | - | - | 10.0 | - |
| Polysorbate 80 | - | - | - | 1.0 |

| Extra-granular % w/w | | | | |
|---|---|---|---|---|
| Sodium Starch Glycollate | 2.0 | 2.0 | 2.0 | 2.0 |
| Magnesium Stearate | 1.0 | 1.0 | 1.0 | 1.0 |
| Total | 100 | 100 | 100 | 100 |

Dry Blending: The weighted, micronized BA was screened through a 500 µm sieve, then mixed with the intra-granular excipients. This resulted in an even-colored bright yellow blend. The blending time for each formulation was 5.00 minutes.

Moisture analysis of Dry Blends was evaluated by loss-on-drying (LOD) analysist. The percentage of moisture loss for each formulation is given in Table 7-2, below.

| Granule Batches | Calculated Equilibrium Moisture Content (EMC) (%) | Moisture Loss on Drying (%) |
|---|---|---|
| | | |
| **FT06212** | 4.00 | 4.48 |
| **FT06213** | 3.94 | 4.39 |
| **FT06214** | 3.11 | 3.32 |
| **FT06215** | 3.94 | 4.41 |

Wet granulation: The micronization process performed on BA appeared to result in tackiness (cohesiveness). A further test revealed that the BA did not readily flow through a funnel and formed clumps when exposed to air. Therefore, wet-granulation was used to improve processing. Granulation took place by adding the granulation fluid slowly while mixing the intra-granular mixture of API and excipients. Table 7-3 shows the addition of granulation fluid.

| | **FT06212** | **FT06213** | **FT06214** | **FT06215** |
|---|---|---|---|---|
| Total amount of granulation fluid (g) | 13.586 | 14.158 | 4.752 | *T-7.095 #W=12.347 |
| Total fluid addition time (min:sec) | 20:30 | 19:00 | 6:00 | 20:00 |
| Total extra mixing time (min:sec) | 00:00 | 01:30 | 00:56 | 00:30 |

| | | | | |
|---|---|---|---|---|
| *T = Tween 80 (6.05% w/w in water) #W = Water | | | | |

Screening of Granules: Granules were screened through a 500 µm sieve onto stainless steel trays.

Drying of Granules: Thee of the screened granule batches were placed in an oven to dry; formulation FT06214 was left to dry overnight at room temperature. Table 7-4 shows the granule drying properties.

**Table 7-4: Granule Drying Properties**

| | **FT06212** | **FT06213** | **FT06214** | **FT06215** |
|---|---|---|---|---|
| Granule drying temperature (°C) | 60 | 60 | 25 | 60 |
| Total drying time (hr:min) | 00:45 | 00:45 | 16:00 | 00:46 |

Moisture Analysis of Granules: The moisture content of the four batches of dried granules was determined. Results are listed in Table 7-5:

**Table 7-5: Granule Moisture Content**

| | **FT06212** | **FT06213** | **FT06214** | **FT06215** |
|---|---|---|---|---|
| Dry granule moisture content (%) | 2.13 | 1.43 | 1.84 | 2.03 |
| Target moisture content (%) | 4.48 | 4.39 | 3.32 | 4.41 |

**Table 7-6: Summary of Results of the Granulation Process**

| | **FT06212** | **FT06213** | **FT06214** | **FT06215** |
|---|---|---|---|---|
| Intra- granular | | | | |
| Dry blend mixing time (min:sec) | 5:00 | 5:00 | 5:00 | 5:00 |
| Dry blend actual % moisture | 4.48 | 4,39 | 3.32 | 4.41 |
| Theoretical % moisture content | 4.00 | 3.94 | 3.32 | 4.41 |
| Total amount of fluid added (g) | 13.586 | 14.158 | 4.752 | 19.442 |
| Total granulation mixing time (min:sec) | 20:30 | 20:00 | 06:56 | 21:30 |
| Total fluid addition time (min:sec) | 20:30 | 19:00 | 06:00 | 20:00 |
| Granule drying temperature (°C) | 60 | 60 | 25 | 60 |
| Dry granule moisture content (%) | 2.13 | 1.43 | 1.84 | 2.03 |
| Total drying time (hr:min) | 00:45 | 00:45 | 16:00 | 00:46 |
| Dry granule % yield | 84.74 | 86.95 | 83.94 | 83.00 |

| Extra -granular | | | | |
|---|---|---|---|---|
| Total Batch Weight (g) | 33.393 | 34.556 | 33.448 | 32.772 |
| % Batch Yield | 83.48 | 86.39 | 83.62 | 81.93 |

Addition of Extra Materials: In order to enhance disintegration, an extra amount of disintegrant was added to the prepared granules; the mixture was blended for 10 minutes using a Turbula^{®} mixer at a speed of 42 rpm. The lubricant was then added as the last excipient and blended with the mixer for 2 minutes at the same speed.

Each of the four batches of granules was characterized using the tests described below.

Angle of Repose: When particulate materials, such as powders and granules, are poured through a funnel from an elevated position, they form an angular heap. The values of the diameter and height of the heap are used to determine the angle of repose. Cohesive materials with poor flowability tend to consolidate when poured; and this cohesion results in high values for the angle of repose. Values above 50° indicate poor flowability; and angles close to 25° indicate excellent flowability.

Density determination: This is the mass of the particulate material divided by its volume. The volume occupied by a particulate material in a measuring cylinder is its aerated bulk density (AD). When the material is subjected to tapping, the tap density (TD) is obtained. Both of these density measurements aid in estimating the settling patterns of materials and the volumes occupied by the formulations. In this study, a 250 mg fill is required to fill a size 0 capsule. These capsules have a maximum fill volume of 0.68 ml. Table 7-8, below, shows that 250 mg of the formulations would easily fit into size 0 capsules.

Compressibility Using Carr's Index: The reduction in the volume of a particulate material in a defined space is a measure of its compressibility. This can be influenced by particle size, shape, density and moisture content. The rate of volume reduction can be deduced from the aerated and tap density content. The rate of volume reduction can be deduced from the aerated and tap density (equation 1); and it is indicative of flowability. Materials that show erratic patterns of densification are typically cohesive and do have high values of Carr's index. Table 7-7, below, provides and estimation of flowability based on Carr's index. Equation 1: % Compressibility = 100% × (Tapped Density - Aerated Density)/Tapped Density

**Table 7-7: Carr's Index as a flow descriptor**

| % Carr's Index | Flow Description |
|---|---|
| 5-15 | Free-flowing granules |
| 12-16 | Free-flowing powdered granules |
| 17-21 | Fair-flowing powdered granules |
| 22-28 | Very fluid powders |
| 28-35 | Fluid cohesive powders |
| 35-38 | Fluid cohesive powders |
| > 40 | Extremely cohesive powders |

**Table 7-8: Results of Physical Characterization of Granules**

| | **FT06212** | **FT06213** | **FT06214** | **FT06215** |
|---|---|---|---|---|
| Aerated density (AD) (g/cm³) | 0.66 | 0.70 | 0.69 | 0.58 |
| Tapped density TD (g/cm³) | 0.78 | 0.80 | 0.75 | 0.68 |
| Carr's Index | 15.4 | 12.5 | 8.0 | 14.7 |
| Capsule fill volume (ml) AD | 038 | 0.36 | 0.36 | 0.43 |
| % volume occupied in size 0 capsules based on AD | 55.9 | 52.9 | 52.9 | 63.2 |
| Angle of repose (°) | 49 | 50 | 50 | 50 |

Capsule Filling: Each formulation was filled into size 0 Swedish orange capsules with the aid of a manual Torpac^{®} capsule filling device and a Perry Accofil^{®} filling gun. For each batch, 20 capsules were randomly sampled and weighed individually. The results are recorded in Table 7-9.

**Table 7-9: Capsule Filling**

| **Capsules** | **FT06212** | **FT06213** | **FT06214** | **FT06215** |
|---|---|---|---|---|
| Target weight (g) | 0.346 | 0.346 | 0.346 | 0.346 |
| Mean weight (g) | 0.354 | 0.350 | 0.341 | 0.345 |
| RSD (%) | 1.68 | 2.35 | 1.59 | 1.98 |
| Min (g) | 0.343 | 0.334 | 0.333 | 0.332 |
| Max (g) | 0.363 | 0.359 | 0.352 | 0.356 |

Disintegration Test: Three capsules were selected from each formulation batch and subjected to disintegration in acid media (pH 2.53) at a temperature of 37°C in a disintegration bath. The time for each capsule's contents to completely disintegrate is recorded in Table 7-10, below:

**Table 7-10: Disintegration Test**

| **Capsules** | Disintegration Time (min:sec) |
|---|---|
| FT06212 Capsule 1 | 1:32 |
| FT06212 Capsule 2 | 2:04 |
| FT06212 Capsule 3 | 2:15 |
| FT06213 Capsule 1 | 2:09 |
| FT06213 Capsule 2 | 2:40 |
| FT06213 Capsule 3 | 1:40 |
| | |
| FT06214 Capsule 1 | 7:23 |
| FT06214 Capsule 2 | 2:45 |
| FT06214 Capsule 3 | 1:40 |
| | |
| FT06215 Capsule 1 | 2:33 |
| FT06215 Capsule 2 | 2:33 |
| FT06215 Capsule 3 | 2:33 |

All of the capsules disintegrated within an acceptable time for immediate release oral dosage form.

### Hot Melt Extrusion Formulation

The conversion of the API (BA) from crystalline to amorphous form was achieved by the process of hot-melt extrusion. A thermoplastic excipient (polymer) is required as a solvent/carrier and also to enhance stability of the amorphous state. Miscibility of the polymer with the API can be estimated using solubility parameters. Generally cationic polymers are miscible with anionic drug substances. In this case BA has a polarity of 12.3 MPa, as opposed to 5.1 mPa for Eudragit E 100^{®} polymer. Two formulations were developed at the ratios of 70:30 and 90:10 (Eudragit^{®} polymer:BA). One parameter used to determine compatibility of API and polymer is the hydrogen attractive force, which is 11.2 cm³ for BA and 8.7 cm³, the difference between which is 2.5 cm³. Generally a difference in hydrogen attractive force of less than 7 cm³ is acceptable. The process parameters for hot-melt extrusion are shown in Table 7-11, below.

**Table 7-11: Extrusion Parameters**

| | **FT06216 (70:30)** | **FT06230 (90:10)** |
|---|---|---|
| Dry blending of API and Polymer (min) | 10 | 10 |
| Dry blending speed (rpm) | 40 | 40 |
| Melt Temperature (°C) | 165-170 | 170 |
| Torque (kPa) | 18-26 | 19-28 |
| Extrusion screw speed (rpm) | 100 | 100 |

Hot-Melt Formulations: The hot-melt formulations were of even color (slightly pale yellow). However formulation FT06216 did not readily flow through a funnel and formed large aggregates. Tests were carried out to assess the density and flowability of the supplied API:Polymer matrices and the results are given in Table 7-12, below.

**Table 7-12: Physical characterization of API:Polymer matrices as supplied**

| **Tests** | **FT06216 (70:30)** | **FT06230 (90:10)** |
|---|---|---|
| Angle of repose (°) | 52 | 48 |
| Aerated bulk density (g/ml) | 0.44 | 0.48 |
| Tap density (g/ml) | 0.53 | 0.58 |
| Moisture Content (%) | 2.22 | 0.85 |

The angle of repose indicates a poor flow for the polymer and API matrices.

Formulation development for Hot-Melt Extrudates: A 2.4 g batch of the 30:70 BA:polymer matrix was prepared and assessed. The formulation and powder properties are shown in the following tables 7-13 and 7-14. The BA:polymer matrix was screened through a 500 µm sieve. The weighed API:polymer matrix was then weighted and added to the other excipients with the exception of magnesium stearate. The mixture was subjected to dry blending for 15 minutes at 42 rpm using the Turbula^{®} mixer. The lubricant was finally added and blended into the rest of the formulation for 2 minutes at 42 rpm.

**Table 7-13: Hot-melt formulation 30:70 (FT06216c) with DCL 21**

| **Materials** | **% w/w** |
|---|---|
| BA:Eudragit E | 82.5 |
| Pharmatose DCL 21 | 12.5 |
| Sodium starch glycolate | 4.0 |
| Magnesium stearate | 1.0 |
| Total | 100 |

**Table 7-14: Density determination of formulation FT06216c**

| | **FT06216 (70:30)** |
|---|---|
| Aerated bulk density (AD) g/ml | 0.51 |
| Tapped density (TD) g/ml | 0.67 |
| Capsule fill volume (ml) | 0.78 |
| % volume occupied in a size 0 capsule (based on AD) | 114.71 |

Due to the small batch size of this formulation it was impossible to carry out an angle of repose test. In comparison to the results in column 2 in Table 7-12, the aerated and tapped density has been improved as shown in Table 7-14. The result also shows that the size 0 capsule should not theoretically be suitable to contain the 400 mg required to achieve the target dose strength for the formulation. However with the aid of manual tapping, it was possible to fill the capsules with the required 400 mg. Trial runs were carried out using the Perry Accofil^{®} vacuumetric powder/granule dispenser and the Torpac^{®} encapsulation device. The tests showed reproducibility in fill weights of 400 mg.

**Disintegration Test on Hot Melt Trial Formulation Capsules:** The disintegration test involved hand-filling 400 mg of the formulation blend filled into three size 0 capsules. The filled capsules were subjected to disintegration tests in two different fluid media-water and 0.1 M hydrochloric acid (pH 4.08). None of the capsules disintegrated in water or 0.1 M HCl in 2 hours.

The formulation was then altered slightly, with the addition of 1% sodium lauryl sulfate (SLS). Capsule disintegration tests were carried out in an acid medium at pH 2.53 and the capsules disintegrated. The disintegration test was then repeated in the acid media at pH 2.53 with the initial formulation and without the addition of SLS. These capsules also disintegrated. These results are shown in Table 7-15.

**Table 7-15: Hot-Melt Formulation 30:70 (FT06216d) with DCL 21**

| **Materials** | **% w/w** |
|---|---|
| BA:Eudragit E | 82.5 |
| Pharmatose DCL 21 | 12.0 |
| Sodium starch glycolate | 4.0 |
| Sodium lauryl sulfate | 1.0 |
| Magnesium stearate | 0.5 |
| Total | 100 |

**Table 7-16: Disintegration in acid media pH 2.53**

| **Hot-melt Extrusion Formulation** | **Disintegration time (min:sec)** |
|---|---|
| Formulation 90:10 without SLS (min:sec) FT06216c | 11:09 |
| Formulation 90:10 with SLS (min:sec) FT06216d | 09:36 |

The disintegration time was slower for the granulated capsule formulations, but were still within the required time of 15 minutes.

**Final formulations for the hot-melt extrusion matrices:** Based on the findings of the disintegration studies, the formulation was altered slightly to enhance disintegration. This was done to prevent disintegration from being the rate-limiting step to dissolution and *in vivo* bioavailability. Details of the final two hot-melt extrusion formulations are shown below:

**Table 7-17: Melt Extrusion Formulation (FT06216) (30:70)**

| **Materials** | **Unit Dose (mg)** | **% w/w** |
|---|---|---|
| BA/Eudragit E | 333.3 | 83.3 |
| Pharmatose DCL 21 | 28.7 | 7.2 |
| Sodium starch glycolate | 32.0 | 8.0 |
| Sodium lauryl sulfate | 4.0 | 1.0 |
| Magnesium stearate | 2.0 | 0.5 |
| Total | 400 | 100 |

The formulation detailed in Table 7-17 shows one capsule to contain 99.99 mg of BA.

**Table 7-18: Melt Extrusion Formulation (FT06230) (10:90)**

| **Materials** | **Unit Dose (mg)** | **% w/w** |
|---|---|---|
| BA/Eudragit E | 333.3 | 83.3 |
| Pharmatose DCL 21 | 28.7 | 7.2 |
| Sodium starch glycolate | 32.0 | 8.0 |
| Sodium lauryl sulfate | 4.0 | 1.0 |
| Magnesium stearate | 2.0 | 0.5 |
| Total | 400 | 100 |

The formulation detailed in Table 7-18 shows one capsule contains 33.33 mg of BA. Therefore a 100 mg dose would require administration of three (3) capsules.

Physical characterization for the hot-melt extrusion formulations: The angle of repose and density for each of the formulations were determined according to the methods described above. The results are recorded in Table 7-19.

**Table 7-19: Summary of the physical properties of the final hot-melt extrusion formulations**

| | **FT06216** | **FT06230** |
|---|---|---|
| Aerated density AD (g/cm³) | 0.49 | 0.51 |
| Tapped density TD (g/cm³) | 0.57 | 0.63 |
| Carr's index (%) | 14.0 | 19.0 |
| Capsule fill volume (ml) AD | 0.82 | 0.78 |
| Angle of repose (°) | 52 | 48 |

Formulation FT06216 (30:70) shows poor flowability, but good packing ability. The ease of plug formation has been demonstrated for the filling of this formulation into size 0 capsules reproducibly. Formulation FT06230 displays good flowability and compressibility; and therefore reproducibility in fill volume is expected and was achieved with 400 mg in each capsule.

**Table 7-20: Capsule Disintegration Test**

| | **Disintegration Time (min:sec)** |
|---|---|
| FT06216 Capsule 1 | 3:12 |
| FT06216 Capsule 2 | 2:54 |
| | |
| FT06230 Capsule 1 | 2:16 |
| FT06230 Capsule 2 | 1:52 |
| FT06230 Capsule 3 | 2:10 |

The disintegration profiles of the granulated and melt extrusion formulation are similar and indicate that rapid disintegration is likely to occur in a low pH environment. The addition of surface active agents (surfactants) such as SLS, Poloxamer or polysorbate, increasing the amount of disintegrant and the use of more hydrophilic diluent has enhanced disintegration of the formulations.

Conclusion: The granulated formulations produced granules of acceptable flow quality, which were reproducibly filled into size 0 capsules. The addition of surface active agents to some of the encapsulated formulations did enhance densification, and the percentage compressibility of the granules. The disintegration of the hot-melt extrusion formulations was improved by the increase in the amount of disintegrant, optimization of the diluent and addition of a surface active agent.

### Example 8

### Oral Doses of BA

Four capsule formulations containing 100 mg of BA (4-iodo-3-nitrobenzamide) were developed. These formulations included:
- Capsules containing solid granulated BA
- Liquid-containing capsules comprising BA
- Semi-solid (aqueous miscible base) filled into hard gelatin capsules
- Semi-solid (aqueous dispersible oil base) filled into hard gelatin capsules

BA (4-iodo-3-nitrobenzamide) has a molecular weight of 292 and a melting point of 159°C. Excipients included partially pregelatinized starch, microcrystalline cellulose, polyvinylpyrrolidone, sodium starch glycolate, sodium lauryl sulfate (SLS), magnesium stearate, polyethylene glycol 400, glycerol, propylene glycol monocaprylate, N,N-dimethylacetamide (DMA), polyethylene glycol 3350 and lauroryl macrogolglyceride. Each excipient was obtained from commercial sources.

The Granulated capsule formulation is shown in Table 8-1, below:

**Table 8-1: Formulation of Granulated Capsule**

| **Material** | **Trade Name** | **Unit Dose (mg)** | **% w/w** |
|---|---|---|---|
| **Intragranular** | | | |
| BA (API) | | 100.00 | 40.00 |
| Partially pregelatinized starch | Starch 1500 | 78.75 | 31.50 |
| Microcrystalline cellulose | Avicel PH 101 | 50.00 | 20.00 |
| Polyvinylpyrrolidone | Povidone K30 | 6.25 | 2.50 |
| Sodium starch glycolate | Explotab | 5.00 | 2.00 |
| SLS | Empicol LZD | 2.50 | 1.00 |

| **Extragranular** | | | |
|---|---|---|---|
| Sodium starch glycolate | Explotab | 5.00 | 2.00 |
| Magnesium stearate | Magnesium stearate | 2.50 | 1.00 |
| | Total | 250.00 | 100.00 |

The formulation was filled into size 0 Swedish orange gelatin capsules.

A 50 g batch of BA was manufactured. The granules were manufactured using the following process:
- The BA active was sieved through a 1000 µm sieve and then the required quantity of sieved material was dispensed;
- The required quantities of Starch 1500, followed by BA, Avicel PH101, Povidone K30, Explotab and Empicol were placed in a food processor and mixed for 5 minutes with a metal blade;
- 16.38 g of water were added over 10 minutes during mixing;
- Granules were dried in an oven at 60°C for 5 hours.

Granules with an even yellow color were produced. These granules had a water content of 4.07%. The theoretical water content was calculated as 4.0%, therefore the granules were dry.

The addition of extragranular materials and the filling of 100 capsules were carried out using the following process:
- The Explotab was added to the granules and mixed with a blender in a container;
- The magnesium stearate was mixed with a portion of the granules and then added to the container; this was mixed at 42 rpm for 2 minutes;
- 100 capsules were filled using a Perry Accofil and Torpac capsule filler. A 3/16 inch filling gun was used to fill 250 mg of the formulation into each capsule.

**Liquid Filled Capsule:** The following method was used to measure the saturated solubility of BA in various solvents:
- Approximately 4 ml of the solution under test were placed in an 8 ml glass vial with a magnetic stir bar;
- An excess of BA was added to each 8 ml glass vial; the sample was placed in an ultrasonic bath for 5 minutes to ensure an excess of BA was present; and more BA was added if required;
- The samples were placed in a 25°C water bath and stirred overnight (at least 16 h);
- The samples were diluted as required and the absorbance measured at 320 nm; the oil based samples were diluted with dichloromethane and the other samples were diluted with acetonitrile; and
- The saturated solubility was calculated from the A^{1%}_{1cm} value for BA and the dilution factor of the solution.

**Solubility enhancement study results:** Details of the materials evaluated in the solubility enhancement study are shown in Table 8-2.

**Table 8-2: Materials Assessed During Solubility Enhancement Study**

| **Material** | **Trade Name/Abbreviation** | **Supplier** |
|---|---|---|
| Diethylene glycol monoethyl ether | Transcutol HP | Gattefosse |
| Propylene glycol monocaprylate | Capmul PG-8 | Abitec |
| N-methyl-2-pyrrolidone | NMP | BDH |
| Polyethylene glycol 400 | PEG 400 | Clariant |
| Oleoyl macrogolglyceride | Labrafil M1944CS | Gattefosse |
| Polyethylene glycol-15-hydroxystearate | Solutol HS-15 | BASF |
| Vitamin E TPGS | Vit E TPGS | Eastman |
| N,N-dimethylacetamide | DMA | Sigma |
| Glycerol | N/A | Fisher |

The results of the solutions tested are shown in Table 8-3

**Table 8-3: Solubility Study Results**

| **Solution** | **BA Solubility (mg/ml)** |
|---|---|
| 100 % NMP | 319 |
| 40 % DMA in Capmul | 276 |
| 25 % w/v Solutol in PEG 400 | 224 |
| 25 % w/v Vit E TPGS in PEG 400 | 204 |
| 20 % v/v glycerol, 3% v/v water in PEG 400 | 192 |
| 100 % Transcutol | 192 |
| 100 % PEG 400 | 186 |
| 30 % v/v DMA in Capmul | 176 |
| 20 % v/v DMA in Capmul | 89 |
| 40 % v/v Transcutol in Capmul | 73 |
| 10 % v/v DMA in Capmul | 57 |
| 100 % Capmul | 32 |
| 100 % Labrafil | 18 |

These results indicate that there were a few formulation options. The material with the best solubility, 100 % NMP, was filled into capsules; but this caused the capsules to crack, and is therefore not a suitable material for a solid gelatin capsule. Such a solution may, however, be suitable for other oral formulations.

The Solutol in PEG 400 and Vit E TPGS in PEG 400 both increased the solubility fo the solution; but both formed cloudy suspensions. These may be suitable if used with another solvent; but they were not evaluated further in this study.

It was decided to make capsules of a PEG 400 formulation and a DMA/Capmul formulation. The PEG 400 formulation is water-miscible and the DMA/Capmul formulation is water immiscible. Both are likely to have different oral bioavailabilities.

**Polyethylene Glycol 400 Formulation:** PEG 400 is hygroscopic and likely to cause the migration of water from capsule shells into the fill, causing the capsules to become brittle. 0.5 ml of PEG 400 was filled into a HPMC capsule; but this cracked and is therefore not suitable for this formulation. When 0.5 ml of PEG 400 was filled into a gelatin capsule, it did not crack; therefore, gelatin capsules are better suited for this formulation. Water and glycerol were added to the formulation to try prevent the capsules from becoming brittle. A level of 5 %v/v glycerol was chosen for the final formulation, because the supplier literature for the capsules did not recommend levels of glycerol above this level. Water was added to a level of 3 %v/v. It was thought that the addition of water and glycerol might help to prevent the capsules from becoming brittle. This formulation is described in Table 8-4.

**Table 8-4: Polyethylene Glycol 400 Formulation**

| **Material** | **Unit Dose (mg)** | **%w/v** |
|---|---|---|
| BA (API) | 100.00 | 17.544 |
| 3 %v/v water | | |
| 5 %v/v glycerol | q.s. to 0.57 ml | q.s. to 100% |
| 92 %v/v polyethylene glycol 400 | | |

The formulation was filled into size 0 Swedish orange capsules.

**N,N-dimethylacetamide/Capmul PG-8 Formulation:** Four different levels of DMA in Capmul were tested during the saturated solubility study. A graph was plotted of the results and is shown in Figure 2. From the graph shown in Fig. 2, a formulation with 35 %v/v DMA in Capmul PG-8 was chosen. This should have a saturated solubility of approximately 220 mg/ml of BA.

A placebo formulation containing 30 %v/v DMA in Capmul was put into HPMC capsules. The capsules became brittle and cracked, which indicates that the formulation is not suitable for HPMC capsules. A placebo formulation containing 40% DMA in Capmul filled into gelatin capsules did not cause any brittleness or cracking; therefore gelatin capsules were judged to be suitable for use in the formulation. Details of the formulation are shown in Table 8-5.

**Table 8-5: N,N-dimethylacetamide/Capmul PG-8 Formulation**

| **Material** | **Unit Dose (mg)** | **%w/v** |
|---|---|---|
| BA (API) | 100.00 | 20.00 |
| 35 %v/v N,N-dimethylacetamide | | |
| 65 %v/v Capmul PG-8 | q.s. to 0.50 ml | q.s. to 100 % |

The formulation was filled into size 0 Swedish orange capsules.

Capsule Manufacture--Polyethylene Glycol 400 Formulation: A 100 ml batch of the formulation shown in Table 5, batch number FT06113, was manufactured. The following process was used:
- The glycerol (6.30 g is equivalent to 5 ml, density = 1.26 g/ml) was weighed into a
- 100 ml volumetric flask.
- 3 ml of water was added using a glass bulb pipette.
- PEG 400 was added, made up to volume and mixed by shaking.
- The BA was weighed into a 100 ml glass bottle and the PEG solution added to approximately 85 ml.
- The resulting mixture was mixed with a magnetic stirrer and heated to 50°C until all of the active had dissolved.
- The solution was transferred to a 100 ml volumetric flask, allowed to cool, then made up to volume with the PEG solution and shaken to mix.
- 100 capsules were each filled with 0.57 ml of the solution using a Finnpipette.
- The capsules were sealed by painting gelatin banding solution around the join between the body and cap of the capsules. Details of the banding solution are shown below.
- The capsules were left until the banding solution dried.

N,N-dimethylacetamide/Capmul PG-8 Formulation: A 75 ml batch of the formulation shown in Table 6, batch number FT06114, was manufactured. The following process was used:
- 28 ml of DMA was placed in a 100 ml measuring cylinder and Capmul added to 80 ml.
- This was shaken to mix.
- The BA was weighed into a 100 ml glass bottle and the Capmul solution added
- to approximately 60 ml.
- It was mixed with a magnetic stirrer and heated to 50°C until all of the active had
- dissolved.
- The solution was allowed to cool, transferred to a 100 ml measuring cylinder, made up
- to volume with the Capmul solution and shaken to mix.
- 100 capsules were each filled with 0.50 ml of the solution using a Finnpipette.
- The capsules were sealed by painting gelatin banding solution around the join between the body and cap of the capsules. Details of the banding solution are shown below.
- The capsules were left until the banding solution dried.

The formulation of the banding solution used to seal the capsules is shown in Table 8-6:

**Table 8-6: Gelatin Banding Solution**

| **Material** | **%w/w** | **g/10 g** |
|---|---|---|
| Tween 80V | 0.78 | 0.078 |
| Gelatin shells (Swedish Orange) | 21.26 | 2.126 |
| Water | 77.96 | 7.796 |

A 10 g mixture of ganding solution was made up using the following procedure:
- The Tween 80 and 2.353 g of water were mixed in a glass vial.
- The gelatin shells were broken up in a food processor and the required weight added.
- It was mixed with a magnetic stirrer and by shaking for 3 minutes.
- The mixture was allowed to stand for approximately 2 hours, and then the remaining water was added.
- It was mixed for 5 minutes with a magnetic stirrer.
- When required, the mixture was heated to 50°C and mixed with a magnetic stirrer.

Capsules were sealed by painting the sealing solution around the join between the cap and body of the capsules with a fine paint brush. The capsules were left for at least 30 minutes for the banding solution to dry.

**Semi-Solid (Aqueous Miscible Base) Filled into a Capsule:** Table 8-7 shows the formulation for the semi-solid (aqueous miscible base) formulation, batch number FT06096.

**Table 8-7: Formulation of Semi-Solid (aqueous miscible base) Capsule**

| **Material** | **Unit Dose (mg)** | **%w/w** |
|---|---|---|
| BA (API) | 100.00 | 20.00 |
| Polyethylene glycol 3350 | 400.00 | 80.00 |
| **Total** | 500.00 | 100.00 |

The formulation was filled into size 0 Swedish orange capsules. A 100 g batch was manufactured according to the following procedure:
- The polyethylene glycol was heated to approximately 66°C whilst stirring with a magnetic stirrer until it melted.
- The active was added and mixed with a Silverson homogeniser for 15 minutes, after this time an even yellow suspension was formed.
- 500 mg of the formulation was filled into each capsule using a Finnpipette and a Torpac capsule filler.

During the filling the temperature was maintained above 65°C to keep the polyethylene glycol molten. The mixing process caused the heat to increase and a maximum temperature of 95°C was obtained. At this temperature most of the active appeared to dissolve in the polyethylene glycol, but came out of solution when the mixture was cooled.

All of the 100 capsules were available for use in the animal study.

**Semi-Solid (Aqueous Dispersible Oil Base) Filled into a Capsule:** Table 8-8 shows the formulation for the semi-solid (aqueous dispersal oil base) formulation, batch number FT06095.

**Table 8-8: Formulation of Semi-Solid (Aqueous-dispersible oil base) Capsule**

| **Material** | **Unit Dose (mg)** | **%w/w** |
|---|---|---|
| BA (API) | 100.00 | 20.00 |
| Gelucire 44/14 | 400.00 | 80.00 |
| **Total** | 500.00 | 100.00 |

The formulation was filled into size 0 Swedish orange capsules. A 100 g batch was made using the following process:
- The Gelucire was heated to approximately 60°C whilst stirring with a magnetic stirrer until it melted.
- The Gelucire was cooled to 50°C before the active was added.
- The BA was added and mixed with a Silverson homogenizer for 15 minutes, after this time an even yellow suspension was formed.
- 500 mg of the formulation was filled into each capsule using a Finnpipette and a Torpac capsule filler.

During the mixing a maximum temperature of 67 °C was obtained. All of the 100 capsules filled were available for use in the animal study.

**Discussion:** All four target capsule types were successfully manufactured. During the solubility study to identify suitable liquid fill formulations two different solvents were identified. One was a water miscible polyethylene glycol 400 based formulation and the other a water immiscible N, N-dimethylacetamide in propylene glycol monocaprylate (Capmul PG-8) formulation.

The two semi-solid formulations were both prepared without any issues. The aqueous miscible formulation contained polyethylene glycol 3350. The aqueous dispersible oil base formulation contained lauroryl macrogolglyceride (Gelucire 44/14). All of the formulations are suitable for progressing to the bioavailability studies.

### Example 9

### BA Oral Bioavailability Study Summary

Self emulsifying lipid formulations (SELF's) are lipid vehicles which are prepared using an oil base, surfactants, co-surfactants and co-solvents. They can function as delivery systems and solubility enhancers. The self-emulsification occurs when the lipid formulations come into contact with an aqueous media or the gastrointestinal fluid. Agitation of the mixture of aqueous media and SELF sample results in the formation of micelles, which aids the transportation of drug substance across the lipid bilayers.

SELF samples can be freshly prepared and are also available as pre-concentrates. The digestive path of the SELF sample is influenced by the triglyceride chain (the number of carbon atoms in a linear sequence) of the selected oil. Medium chain triglycerides (MCT) are absorbed within the portal system while the long chain triglycerides (LCT) are absorbed in the lymphatic.

A SELF kit designed as a preformulation screening tool to enable the selection of suitable self emulsifying formulations that are miscible with the drug and show maximum solubility enhancement was supplied by Gattefosse. This consisted of three semi-solids and five liquid formulations.

The aim of this work is to evaluate the propensity of SELF samples to self emulsify in water and also to assess the improvement of BA solubility in these lipid systems. An initial miscibility test was carried out with each SELF sample and the diluting solvent acetonitrile. The self emulsifying lipids supplied were used to carry out solubility enhancement evaluation on the BA (API).

**Table 9-1: Materials used**

| **Material** | **Supplier** | **Function** | **Formulation Type** |
|---|---|---|---|
| BA | Bipar | API | Crystalline |

| **Self Emulsifying Lipids (SELF's)** | | | |
|---|---|---|---|
| 27A | Gattefosse | Vehicle/ So lubility Enhancer | Semi-solid |
| 27B | Gattefosse | Vehicle / Solubility Enhancer | Semi-solid |
| 27C | Gattefosse | Vehicle / Solubility Enhancer | Liquid |
| 27D | Gattefosse | Vehicle / Solubility Enhancer | Liquid |
| 27E | Gattefosse | Vehicle / Solubility Enhancer | Liquid |
| 27F | Gattefosse | Vehicle / Solubility Enhancer | Liquid |
| 27G | Gattefosse | Vehicle / Solubility Enhancer | Semi-solid |
| 27H | Gattefosse | Vehicle / Solubility Enhancer | Liquid |

| **Oils** | | | |
|---|---|---|---|
| Castor Oil | Sigma-Aldrich | Vehicle | Liquid |
| Corn Oil | Sigma | Vehicle | Liquid |
| Miglyol 812 | Sasol | Vehicle | Liquid |

| **Bioavailability Enhancers (Surfactants)** | | | |
|---|---|---|---|
| Capryol PGMC | Gatefosse | Surfactant | Liquid |
| Cremphor ELP | BASF | Surfactant | Semi-solid |
| Gelucire 44/14 | Gattefosse | Surfactant | Semi-solid |
| Maisine-35 | Gattefosse | Surfactant | Liquid |
| Polyethylene glycol 400 | Clairant | Surfactant | Liquid |
| Vitamin E TPGS | Eastman | Surfactant | Semi-solid |

| Others | | | |
|---|---|---|---|
| Sweedish orange size 0 hard gelatine capsules | Capsugel | Drug delivery device | Solid |
| Acetonitrile | Fisher Scientific | Solvent | Liquid |

Solvent Miscibility Test: Equal portions of each SELF sample and neat acetonitrile were accurately measured into a clear screw cap bottle using a pipette and then swirled for 1 minute using the Heidolph relax top a vortex mixer. The mixtures thus produced-a continuous phase of a visibly clear solution-are miscible.

Saturated Solubility Test for Liquid SELF Samples: 2 ml of each liquid SELF was measured into 8 ml screw cap glass bottle using a Finnpipette. Aliquots of 50 mg of the API were then added until the mixture appeared to be saturated. The mixtures were sonicated for 30 minutes, and then observed to see if saturation had been maintained. Aliquots of 25 mg of the API were further added to non-saturated samples while stirring; these were sonicated for a further 30 minutes. This process was repeated until saturation was attained and maintained after sonication.

The liquid samples were then placed in the Variomatic water bath at 25°C and left to stir for two nights. Each of the SELF samples was purged with nitrogen gas after use to minimize oxidation.

The saturated liquid samples were centrifuged at a speed of 4000 rpm for 10 minutes at 25°C.

Saturated Solubility Test for Semi-Solid SELF Samples: Prior to dispensing, semi-solid lipids were melted in a dry oven at 60°C. The semi-solids were dispensed into screw cap bottles; aliquots of the API were added to attain and maintain saturation. The saturated semi-solids were left stirring at 60°C.

The following day the stirrer was switched off but heating was maintained at 60°C for 1 hour. This was to allow the dispersed solid particles to sediment. 0.1 ml was taken from the top clear layer of each of the saturated semi-solid samples and diluted with acetonitrile. For samples that showed no sedimentation, due to the saturated API solubilizing over time, more API was added and the samples were left to stir for one more night.

**UV Analysis:** The saturated samples and the lipid vehicles were diluted with acetonitrile at the same ratio. The samples were diluted with acetonitrile to obtain an absorbance between 0.1 to 1. The absorbance of the diluted lipid vehicles were used as the baseline for the absorbance readings of the active sample. The diluted samples were scanned at a fixed wavelength of 320 nm. The concentration of the API was calculated using the E¹₁ value, derived from the API concentration in acetonitrile.

BIOAVAILABILITY RESULTS OF ENCAPSULATED GRANULE FORMULATION: Oral bioavailability studies were carried out on ten dogs, five female and five male. This involved administering 60 mg per kg of the granulated capsules and 33 mg per kg of the hot melt formulation. These bioavailability results were compared with that of previous granulated capsule formulation (Phase I). Table 9-2 shows the mean percentage bioavailability of each formulation and the number of dogs that attained the target percentage bioavailability.

**Table 9-2 Percentage bioavailability derived from dog study**

| **Formulations** | **Study Phase** | **% Bioavailability (F)** | **Number of Dogs > 20% F** |
|---|---|---|---|
| Granulated capsule with SLS | **II** | 17.4 ± 10.7 | 5 |
| Granulated capsule | **I** | 11.5 ± 7.9 | 1 |
| Granulated capsule with Poloxamer 188 | **II** | 8.2 ± 5.7 | 0 |
| Granulated capsule with Tween 80 | **II** | 2.2 ± 1.4 | 0 |
| Hot melt extrusion 30:90 | **II** | 2.1 ± 1.2 | 0 |
| Hot melt extrusion 10:90 | **II** | 1.3 ± 0.6 | 0 |

The combination of the size reduction of the drug substance BA and the use of sodium lauryl sulfate, an ionic surfactant, has been shown to enhance bioavailability of the drug substance. Increasing the surface area of the drug substance has improved its permeability. Sodium lauryl sulphate is an anionic surfactant and it is amphiphilic in nature, being both hydrophilic and lipophilic. Sodium lauryl sulphate has the chemical structure Na⁺⁻OSO₃(CH₂)₁₁CH₃.

The polymeric carbon chain of sodium lauryl soleplate provides it lipophilicity while the soleplate head provides its hydrophilicity. It is this amphiphilic nature of SLS that enables it to enhance hydration and permeation in a wide group of compounds. The concentration of SLS influences the rate of micelle formation. It has been reported that SLS may promote porosity in the parent drug substance. Increasing the quantity of sodium lauryl sulphate in future formulations should further enhance its ability to 'lock' on to the BA drug substance and promote rapid drug-micelle matrix formation and rapid wetting ability. Incorporating sodium lauryl sulphate into self emulsifying systems may also provide the potential for increased wettability and permeability.

### Solubility Enhancement with Pre-Concentrate self emulsifying lipids

Solvent Miscibility Test: Mscibility test was carried out as described above; the results are detailed in Table 9-3.

**Table 9-3: Acetonitrile miscibility with Gattefosse supplied SELF samples**

| **SELF's** | **Miscibility** | **Appearance** |
|---|---|---|
| 27A | Yes | Clear |
| 27B | Yes | Slightly cloudy |
| 27C | No | Clear/ Phase separation |
| 27D | Yes | Clear |
| 27E | Yes | Clear |
| 27F | Yes | Clear |
| 27G | Yes | Clear |
| 27H | No | Clear/Phase separation |

Miscibility test samples 27A, D,E,F and G showed immediate miscibility that was maintained over 48 hours. Sample 27B showed evidence of turbidity when shaken, but this cleared up once shaking ceased. Samples 27C and H appeared clear when shaken, but separated into two phases when left to stand. Based on these observations, the lipid samples that passed the miscibility test were used for further API analysis.

**Test for Self Emulsification:** 0.15 ml was taken from each placebo lipid delivery system and was dispensed into 200 ml of water while stirring, the results are shown in Table 9-4.

**Table 9-4: self emulsification of SELF's in water**

| **Self emulsifying lipids** | **Appearance in water** | **Comments** |
|---|---|---|
| 27A | Clear | Micro emulsion formed |
| 27B | Cloudy / Golden tinge | Emulsion formed |
| 27C | Cloudy / Golden tinge | Emulsion formed |
| 27D | Clear | Micro emulsion formed |
| 27E | Cloudy / Golden tinge | Emulsion formed |
| 27F | Clear | Micro emulsion formed |
| 27G | Clear | Micro emulsion formed |
| 27H | Clear | Micro emulsion formed |

Saturated Solubility: Saturated solubility was carried out on the six SELF samples (Gattefosse) that were miscible with acetonitrile. These samples were stirred over two nights. Results of this study are shown in Table 9-5.

**Table 9-5: Concentration of BA in solvents after 2 nights**

| **Samples** | **Rank** | **Mean Conc. mg/ml** | **Solubility Enhancement vs. water** |
|---|---|---|---|
| Water | 7 | 0.20 | N/A |
| 27B | 6 | 105 | 524 |
| 27E | 5 | 107 | 675 |
| 27G | 4 | 139 | 695 |
| 27D | 3 | 140 | 699 |
| 27F | 2 | 161 | 804 |
| 27A | 1 | 215 | 1074 |

The Gattefosse self emulsifying lipids were first analysed for solubility enhancement. Based on the initial results, it was necessary to prepare some more self emulsifying lipids. The formulae for the freshly prepared self emulsifying lipids are detailed in table 9-6.

Preparation of self emulsifying Lipids. The self emulsifying lipids were prepared by a single pot process. The mixture of oil, surfactants and co-surfactants were homogenised at high speed, until the whole content became frothy, the samples were then sonicated for 30 minutes at 60°C. Further sonication was carried out to remove remaining bubbles.

**Table 9-6: Freshly prepared self emulsifying lipids**

| **SELF's** | **Excipients of the self emulsifying lipids** | | | | | |
|---|---|---|---|---|---|---|
| | **OIL** | **%** | **Surfactant** | **%** | **Co-surfactant** | **%** |
| F1 | Miglyol 812 | 30 | Solutol HS | 50 | PEG 400 | 20 |
| | | | | | | |
| F2 | Corn Oil | 30 | Solutol HS | 47.5 | PEG400 | 20 |
| | | | | | SLS | 2.5 |
| | | | | | | |
| F3 | Castor Oil | 50 | Solutol HS | 40 | Tween 80 | 10 |
| | | | | | | |
| F4 | Miglyol 812 | 37.5 | Gelucire 1/44 | 50 | Tween 80 | 10 |
| | | | | | SLS | 2.5 |
| | | | | | | |
| F5 | Miglyol 812 | 40 | Vitamin E TPGS | 40 | Capryol PGMC | 20 |
| | | | | | | |
| F6 | Castor Oil | 40 | Maisine | 30 | Cremphor ELP | 27.5 |
| | | | | | SLS | 2.5 |
| | | | | | | |
| F7 | Miglyol 812 | 27.5 | Solutol HS | 50 | PEG 400 | 20 |
| | | | | | SLS | 2.5 |

Test for self emulsification of freshly prepared SELF Sample. Approximately 0.15 ml of the placebo self emulsifying lipids were dispensed into 200 ml of water while stirring. The freshly prepared self emulsifying lipids spontaneously emulsified in water. The mixture of formulation F1 in water remained clear, this may indicate nano-particle size scale emulsion and the formation of a microemulsion. All the other formulations in water showed an opaque tinge of gold colour when observed under light.

Miscibility assessment of the freshly prepared self emulsifying lipids in acetonitrile. Equal amounts of the freshly prepared SELF samples and acetonitrile were dispensed into a 20 ml clear screw cap bottle. The mixtures were swirled for 1 minute using a heidolph relax top. The observations are recorded in table 9-7.

**Table 9-7: Miscibility of SELF samples in acetonitrile**

| **SELF Samples** | **Acetonitrile** | **Appearance** |
|---|---|---|
| F1 | Yes | Clear |
| F2 | Yes | Slightly cloudy |
| F3 | Yes | Clear |
| F4 | Yes | Clear |
| F5 | Yes | Clear |
| F6 | Yes | Clear |
| F7 | Yes | Clear |

Due to the potential instability of sample F2 its use for solubility enhancement was discontinued.

Saturated solubility of freshly prepared lipid samples: Saturated solubility was carried out on each of the freshly prepared semi-solid lipid samples as described above. The results in table 9-8 are based on equilibration of saturated samples over two nights.

**Table 9-8 Solubility enhancement of freshly prepared semi-solid lipid samples**

| **Samples** | **Rank** | **Mean Conc. mg/ml** | **Solubility Enhancement in comparison with water** |
|---|---|---|---|
| Water | 7 | 0.20 | N/A |
| F4 | 6 | 42 | 210 |
| F5 | 5 | 75 | 375 |
| F6 | 4 | 76 | 380 |
| F3 | 3 | 87 | 435 |
| F7 | 2 | 135 | 675 |
| F1 | 1 | 185 | 925 |

Solubility Enhancement Data: The solubility enhancement of the API in all the tested samples were calculated in comparison with the concentration of API in water and have been ranked in order of increment (see table 9-9).

**Table 9-9 Concentration of BA in saturated samples after 2 nights**

| **Samples** | **Rank** | **Mean Conc. mg/ml** | **Solubility Enhancement** |
|---|---|---|---|
| Water | 13 | 0.20 | N/A |
| F4 | 12 | 42 | 212 |
| F5 | 11 | 75 | 374 |
| F6 | 10 | 76 | 380 |
| F3 | 9 | 87 | 435 |
| 27B | 8 | 105 | 524 |
| 27E | 7 | 107 | 532 |
| F7 | 6 | 135 | 675 |
| 27G | 5 | 139 | 695 |
| 27D | 4 | 140 | 699 |
| 27F | 3 | 161 | 804 |
| F1 | 2 | 185 | 924 |
| 27A | 1 | 215 | 1074 |

Good solubility enhancement has been achieved with some of Gattefose samples and the in-house SELF samples. The use of sodium lauryl sulphate (SLS) in the formulation of the self emulsifying lipids did not have the desired effect probably due to the particle size of the powdered SLS. Optimising the preparation methods of the lipids could further enhance solubility.

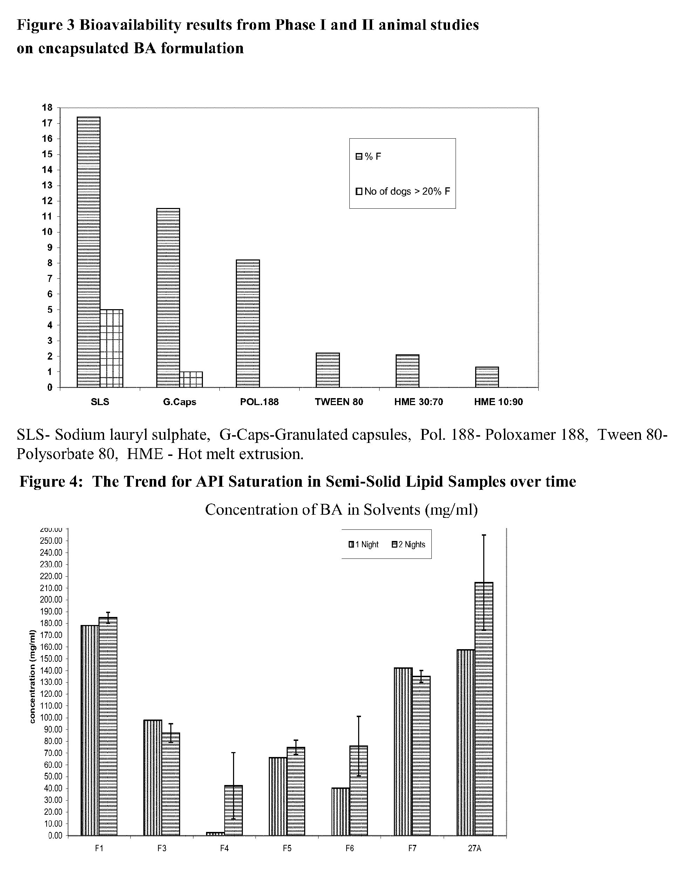

Data Analysis: A comparison was made between the concentration of saturated samples equilibrated for one night and those for two nights. On observation there appeared to be a phase separation of samples F3 and F7, this usually indicates that the emulsion is unstable and may impact on the concentration of the API over time. The graph in FIG. 4 not only highlights the effect of stability of the delivery agents on concentration, it also indicates the length of time required to obtain saturation.

Saturated samples of the semi-solids were analysed at each time point using a UV spectrophotometer; after the first and then the second night of saturation. Samples F1, F4, F5, F6 and 27A showed increase in API concentration over the 2 nights. However samples F3 and F7 showed slight reduction in concentration. This may be attributed to the instability of these two self emulsifying lipids; phase separation may result in a reduced amount of API in the top layer of the saturated mixture. Sample F1 showed the highest concentration after the first night of equilibration, this may indicate that sample 27A has more of a slow release effect on the API than F1. It also implies that equilibration over two nights may be more suitable for lipid vehicles.

Evaluation of Capsule Filling Efficiency: A selected number of the lipid systems were evaluated to ascertain the amount that will fill into a size zero capsule. The derived density is then applied to calculate the capsule fill volume for a total fill weight of 0.6g. The results of this study are set forth in Table 9-10.

**Table 9-10 Filling capacity of Size 0 capsules with self emulsifying lipids**

| **Materials** | **Actual Fill Volume (ml)** | **Actual Fill Weight (g)** | **Density (g/ml)** | **Calculated Capsule fill Volume (ml)** |
|---|---|---|---|---|
| PEG | 0.45 | 0.5315 | 1.18 | 0.51 |
| 400 | | | | |
| | | | | |
| F7 | 0.6 | 0.5503 | 0.92 | 0.65 |
| | | | | |
| 27G | 0.6 | 0.5815 | 0.97 | 0.62 |
| | | | | |
| 27D | 0.6 | 0.6207 | 1.04 | 0.57 |
| | | | | |
| 27F | 0.6 | 0.6196 | 1.03 | 0.58 |
| | | | | |
| F1 | 0.6 | 0.5803 | 0.97 | 0.62 |
| | | | | |
| 27A | 0.6 | 0.6008 | 1.001 | 0.6 |

The data in table 9-10 reveal that all the tested lipid systems would fill into a size zero capsule at 0.6 g this is based on approximately 100 % fill volume. The addition of the API may alter these values.

Estimation of API content in the lipid systems: The API content in a size zero capsule was determined for each of the two formulations that showed suitable solubility enhancement for a 100 mg unit capsule dose. The derived density was applied to estimate the API content in 90 % of the capsule fill volume. The rationale for this is to primarily avoid spillage during formulation batch production and to allow for potential expansion of the semi-solids in the capsule shell due to the cooling effect. The calculated values are detailed in Table 9-11.

**Table 9-11 API content for 90% of the capsule fill volume**

| **Materials** | **Density g/ml** | **90% Capsule fill volume** | **API Conc. mg/ml** | **API Content (mg) per size 0 capsule** |
|---|---|---|---|---|
| F1 | 0.97 | 0.56 | 184.76 | 104 |
| 27A | 1.001 | 0.54 | 214.73 | 116 |

The target drug content for BA API is 100 mg per unit capsule; the results in table 9-11 reveal that the desired dose strength can be achieved with lipid formulations F1, 27A and 27F.

The bioavailability result of the encapsulated granules has demonstrated that the size reduction of the API has slightly enhanced drug absorption.

### Bioavailability results

Bioavailability Results are shown in Tables 9-12 and 9-13, below.

**Table 9-12: Bioavailability Results (First Phase)**

| **Formulation** | **CWZAGV** | **CWZAIS** | Dog ID **CWZAKD** | **CWZBAR** | **Mean ± (S.D.)** |
|---|---|---|---|---|---|
| Semisolid aqueous dispersible base | 12.0 | 6.7 | 4.6 | 14.5 | 9.5 (4.6) |
| Seimisolid aqueous miscible base | 21.2 | 6.6 | 3.6 | 3.1 | 8.6 (8.5) |
| Granulated powder capsules | 20.6 | 6.1 | NA | 7.9 | 11.5(7.9) |
| PEG 400 | 4.6 | 7.3 | 4.6 | 10.3 | 6.7(2.7 |
| N,N-dimethylacetamide PEG Monocaprylate | 15.3 | 4.3 | 2.5 | 20.5 | 10.7 (8.6) |

**Table 9-13: Bioavailability results (Second Phase)**

| **ID** | **Sodium Lauryl** | **Poloxamer** | **Polysorbate 80** | **Hot Melt Extrusion 10:90** | **Hot Melt Extrusion 30:70** |
|---|---|---|---|---|---|
| CTPALK | 11.6 | 0.2 | NC | NC | NC |
| CURAWI | 12.6 | 16.2 | 3.6 | NC | 1.2 |
| CURAWW | 26.2 | 2.3 | 0.8 | 0.5 | 1.3 |
| CUSAIT | 21.2 | 6.7 | 3.6 | NC | NC |
| CUSAKN | 36.3 | 14.7 | 3.8 | 2.1 | 2.4 |
| CUSAND | 25.6 | 2.1 | 0.6 | NC | 2.8 |
| CUZAKY | 5.6 | 9.9 | 0.9 | 1.3 | 1.6 |
| CVSCCL | 21.3 | 5.5 | 2.5 | 1.2 | 3.1 |
| CVSCNH | 0.2 | 10.6 | 1.0 | NC | 0.5 |
| CVSCYM | 13.3 | 14.1 | 3.4 | NC | 4.0 |
| **Mean** | 17.4 | 8.2 | 2.2 | 1.3 | 2.1 |
| SD | 10.7 | 5.7 | 1.4 | 0.6 | 1.2 |

### Example 10

### BA Oral Bioavailability Study Summary

Two oral bioavailability studies for BA were performed in beagle dogs. The in-life phase was conducted at Covance (Kalamazoo, MI) under non-GLP conditions. Plasma samples were collected at pre-determined time points post dose and frozen samples were shipped to Alta Analytical (El Dorado Hills, CA) for determination of BA and its metabolites concentrations using a LC/M/MS assay.

### Formulations

A listing of the formulations tested in this study is given in Table 10-1 below. The liquid for injection was supplied by Aptuit and stored at 5°C. The capsules were supplied by Patheon UK Limited and were stored at ambient temperature.

**Table 10-1: Formulations Tested in Study**

| **Phase** | **Formulation Number** | **Description** | **Bottle Lot No.** | **Paperwork Lot No.** |
|---|---|---|---|---|
| 1 | Liquid | Liquid for injection | PD05101 | NA |
| 2 | 1 | Size reduced | FT06234 | FT06212 |
| 3 | 2 | Sodium lauryl sulfate | FT06235 | FT06213 |
| 4 | 3 | Poloxamer 188 | FT06236 | FT06214 |
| 5 | 4 | Polysorbate 80 | FT06237 | FT06215 |
| 6 | 5 | Hot melt extrusion 10:90 | FT06230 | NA |
| 7 | 6 | Hot melt extrusion 30:70 | FT06216 | NA |
| 8 | 1 | Size reduced | FT06234 | FT06212 |

**Table 10-2: Excipients Used in the Capsule Formulations.**

| **Material** | **Trade Name** | **Supplier** | **Function** | **Formulation Type** |
|---|---|---|---|---|
| Partially Pregelatinized Starch | Starch 1500 | Coloreon | Diluent | Granule |
| Lactose Anhydrous | Pharmatose DCL 21 | DMV | Diluent | Powder |
| Microcrystalline Cellulose | Avicel PH101 | FMC | Diluent | Powder |
| Polymethacrylates | Eudragit | Degussa | Carrier (hot melt extrusion) | Powder |
| Polyvinylpyrrolidinone | PovidoneK30 | BASF | Binder | Granule |
| Sodium Starch Glycollate | Explotab | Penwest | Disintegrant | Granule |
| Magnesium Stearate | N/A | Peter Graven | Lubricant | Powder |
| Sodium Lauryl Sulfate | Empicol LZD | Huntsmann | Surfactant | Granule |
| Poloxamer 188 | Lutrol | Univar | Surfactant | Granule |
| Polysorbate 80 | Tween 80 | ICI | Surfactant | Granule |
| Purified water | N/A | N/A | N/A | N/A |

Four formulations were prepared by the process of wet granulation. A summary of the formulations for the granulated capsules is described in Table 10-3. 4-Iodo-3-nitrobenzamide API is referred as BA.

**Table 10-3: Granule Formulations**

| | **12** | **13** | **14** | **15** |
|---|---|---|---|---|
| **Intra-granular % w/w** | | | | |
| BA (micronized) | 40.0 | 40.0 | 40.0 | 40.0 |
| Pregelatinized Starch | 32.5 | 31.5 | 22.5 | 31.5 |
| Microcrystalline Cellulose PH101 | 20.0 | 20.0 | 20.0 | 20.0 |
| Polyvinylpyrrolidionone K30 | 2.5 | 2.5 | 2.5 | 2.5 |
| Sodium Starch Glycollate | 2.0 | 2.0 | 2.0 | 2.0 |
| Sodium Lauryl Sulfate | N/A | 1.0 | N/A | N/A |
| Poloxamer 188 | N/A | N/A | 10.0 | N/A |
| Polysorbate 80 | N/.A | N/A | N/A | 1.0 |

| **Extra-granular % w/w** | | | | |
|---|---|---|---|---|
| Sodium Starch Glycollate | 2.0 | 2.0 | 2.0 | 2.0 |
| Magnesium Stearate | 1.0 | 1.0 | 1.0 | 1.0 |
| **Total** | 100 | 100 | 100 | 100 |

Each critical process has been identified. 4-Iodo-3-nitrobenzamide API is referred as API BA.

Details of the two hot melt extrusion formulations are shown below in Table 10-4 and Table 10-5.

**Table 10-4: Melt Extrusion Formulation FT06216 (30:70)**

| **Materials** | **Unit Dose (mg)** | **% w/w** |
|---|---|---|
| BA/Eudragit E | 333.3 | 83.3 |
| Pharmatose DCL 21 | 28.7 | 7.2 |
| Sodium starch glycolate | 32.0 | 8.0 |
| Sodium lauryl sulfate | 4.0 | 1.0 |
| Magnesium stearate | 2.0 | 0.5 |
| **Total** | **400** | **100** |

**Table 10-5: Melt Extrusion Formulation FT06230 (10:90)**

| **Materials** | **Unit Dose (mg)** | **% w/w** |
|---|---|---|
| BA/Eudragit | 333.3 | 83.3 |
| Pharmatose DCL 21 | 28.7 | 7.2 |
| Sodium starch glycolate | 32.0 | 8.0 |
| Sodium lauryl sulfate | 4.0 | 1.0 |
| Magnesium stearate | 2.0 | 0.5 |
| **Total** | **400** | **100** |

A listing of the formulations tested in study 7666-113 is given in Table 10-6 below. The liquid for injection was supplied by Aptuit and stored at 5°C. The capsules were supplied by Patheon UK Limited and were stored at ambient temperature.

**Table 10-6: Formulations Tested in Study 7666-113**

| **Phase** | **Formulation Number** | **Description** | **Lot No.** |
|---|---|---|---|
| 1 | Liquid | Liquid for injection | PDO5101 |
| 2 | 1 | Micronized | FT07030 |
| 3 | 2 | Micronized with 1% SLS | FT07031 |
| 4 | 3 | Micronized with 1% SLS | FT07032 |
| 5 | 4 | Semi-solid | FT07026 |

All the formulations containing 100 mg of micronized BA and different levels of sodium lauryl sulfate are described in Table 10-7. Micronized BA was made at Micro technologies by feeding the API through a milling system. The final particle size was below 10 µm.

**Table 10-7: Micronized BA Formulations**

| | **FT07030** | | **FT07031** | | **FT07032** | |
|---|---|---|---|---|---|---|
| **Material** | **mg/cap** | **% w/w** | **mg/cap** | **% w/w** | **mg/cap** | **% w/w** |
| **Intra-granular** | | | | | | |
| BA (micronized) | 100.00 | 40.0 | 100.00 | 40.0 | 100.00 | 40.0 |
| Starch 1500 | 81.25 | 32.50 | 78.75 | 31.50 | 76.25 | 30.50 |
| Avicel PH101 | 50.00 | 20.00 | 50.00 | 20.00 | 50.00 | 20.00 |
| Povidone K30 | 6.25 | 2.50 | 6.25 | 2.50 | 6.25 | 2.50 |
| Sodium starch glycolate | 5.00 | 2.00 | 5.00 | 2.00 | 5.00 | 2.00 |
| Sodium lauryl sulfate | - | - | 2.50 | 1.00 | 5.0 | 2.0 |

| **Extra-granular** | | | | | | |
|---|---|---|---|---|---|---|
| Sodium starch glycolate | 5.00 | 2.00 | 5.00 | 2.00 | 5.00 | 2.00 |
| Magnesium stearate | 2.50 | 1.00 | 2.50 | 1.00 | 2.50 | 1.00 |
| **Total** | 250.00 | 100.00 | 250.00 | 100.00 | 250.00 | 100.00 |

The semi-solid capsule formulation was prepared using a self micronized emulsifying drug delivery system (SMEDDS) kit 27A. A self-micronized emulsifying drug delivery system (SMEDDS) was freshly prepared using waxy solid for the oil phase, a lipid co-surfactant and a surfactant. The oil used consists of a mixture of fatty acids of variable chain lengths; but it is generally classed as a medium-chain triglyceride. The SMEDDS formulation possesses a high percentage of oil; and this may have the effect of bimodal absorption profile, in the form of an initial rapid absorption through the portal system and a residual absorption through the lymphatic system. The formulation for the freshly prepared SMEDDS vehicle is detailed in Table 10-8.

**Table 13: SMEDDS formulation:**

| **Materials** | **Batch Numbers** | **Functions** | **% w/w** | **Bulk Weight (g)** | **Actual Weight (g)** |
|---|---|---|---|---|---|
| Gelucire 44/14 | 105656 | Oil | 75 | 300.00 | 300.59 |
| Labrasol | 32927 | Surfactant | 20 | 80.00 | 80.62 |
| Lauroglycol 90 | 34643 | Co-Surfactant | 5 | 20.00 | 20.30 |
| **Total** | | | **100.00** | **400.00** | **401.51** |

The BA SMEDDS based capsules were manufactured using the freshly prepared SMEDDS formulation as described in Table 10-9. The batch number for the capsule formulation is FT07026.

**Table 10-9. Formulation for BA in SMEDDS FT07026**

| **Materials** | **Functions** | **Batch Number** | **Unit Dose (mg)** | **% w/w** | **Bulk Weight (g)** | **Actual Weight (g)** |
|---|---|---|---|---|---|---|
| BA | API | 06-318 005RRD | 100 | 18.18 | 18.18 | 18.20 |
| SMED FT07025 | Lipid Vehicle | FT07025 | 450 | 81.82 | 81.82 | 81.93 |
| **Total** | | | **550** | **100** | **100** | **100** |

### In-Life Phase (In Vivo Beagle Dogs)

Beagle dogs were transferred from Covance stock colony and acclimated for six days before the test period. During acclimation and the test period, animals were individually housed in wire-mesh enclosures with coated rod-bottom floors. Animals were not commingled for at least 24 hours after dose administration to allow monitoring of any test article-related effects. Animals were fed non-certified Canine Diet #5L03 (PMI Feeds, Inc.) *ad libitum*, except as specified for dose administration.

Each dog received a treatment, either IV injection or orally administered capsules, according to Table 10-10 and 10-11 below:

**Table 10-10: 7666-110 Study Design**

| | Number of Animals | | | Dose Route | Target Dose Level | Target Dose Concentration | Target Dose Volume |
|---|---|---|---|---|---|---|---|
| Phase | Male | Female | Test Article Formulation | | (mg/kg) | (mg/mL) | (mL/kg) |
| 1 | 5 | 5 | BA - Liquid for Injection | IV | 5 | 10 | 0.5 |
| 2 | 5 | 5 | BA - Formulation 1 capsules | Oral | 100 | 100^{a} | NA |
| 3 | 5 | 5 | BA - Formulation 2 capsules | Oral | 60 | 100^{a} | NA |
| 4 | 5 | 5 | BA - Formulation 3 capsules | Oral | 60 | 100^{a} | NA |
| 5 | 5 | 5 | BA - Formulation 4 capsules | Oral | 60 | 100^{a} | NA |
| 6 | 5 | 5 | BA - Formulation 5 capsules | Oral | 33 | 33^{a} | NA |
| 7 | 5 | 5 | BA - Formulation 6 capsules | Oral | 60 | 100^{a} | NA |
| 8 | 5 | 5 | BA - Formulation 1 capsules | Oral | 60 | 100^{a} | NA |
| IV | Intravenous. | | | | | | |
| NA | Not applicable. | | | | | | |
| | Note There was at least a 7 -day washout period between phases. | | | | | | |

| | | | | | | | |
|---|---|---|---|---|---|---|---|
| a mg/capsule. | | | | | | | |

**Table 10-11: 7666-113 Study Design**

| Phase/ Group | Number of Animals | | Test Article Formulation | Dose Route | Target Dose Level | Target Dose Concentration | Target Dose Volume |
|---|---|---|---|---|---|---|---|
| | Male | Female | | | (mg/kg) | (mg/mL) | (mL/kg) |
| 1/1 | 3 | 3 | BA - Liquid for Injection | IV | 5 | 10 | 0.5 |
| 2/1 | 3 | 3 | BA - Formulation 1 capsules | Oral | 100 | 100^{a} | NA |
| 3/1 | 3 | 3 | BA - Formulation 2 capsules | Oral | 100 | 100^{a} | NA |
| 4/1 | 3 | 3 | BA - Formulation 3 capsules | Oral | 100 | 100^{a} | NA |
| 5/1 | 3 | 3 | BA - Formulation 4 capsules | Oral | 100 | 100^{a} | NA |
| IV | Intravenous. | | | | | | |

| | | | | | | | |
|---|---|---|---|---|---|---|---|
| a mg/capsule. Note: There was a washout period of at least 7 days between phases. | | | | | | | |

### Dose Preparation

For intravenous administration, the test article was dosed as supplied without dilution. For oral administrations, capsules were administered as supplied. Any intravenous dose formulation remaining following administration was stored at approximately 5°C.

### Dose Administration

The animals dosed orally were fasted overnight through approximately 4 hours post dose for each phase. Animals were not fasted prior to the intravenous dose administration. Individual doses were calculated based on body weights taken on the day of dosing.

The intravenous dose was administered as supplied via a cephalic vein. After dose administration, but before the needle was removed from the animal, the dose apparatus was flushed with approximately 2 mL of saline. The capsule doses were administered orally, followed by approximately 5 mL of water.

### Sample Collection

Blood (approximately 2 mL) was collected from a jugular vein into tubes containing K₃EDTA anticoagulant pre-dose and at 0.033, 0.083, 0.25, 0.5, 1, 2, 3, 4, 6, 8, 12, 16, and 24 hours post-dose.

### Animal Disposition

Upon completion of the in-life portion of the study, animals were returned to the Covance stock colony.

### LC/MS/MS Analysis

Plasma BA concentrations were determined using an LC/MS/MS assay with a lower limit of quantitation of 5.0 ng/mL at Alta Analytical. The analyte and internal standard (CNBA {4-chloro-3-nitrobenzamide}) were extracted from dog plasma by liquid-liquid extraction using methyl-t-butyl ether (MTBE). After evaporation to dryness and reconstitution, the extracts were analyzed by LC-API/MS/MS. Run times were approximately five minutes.

### Equipment and Apparatus

- SCIEX API 3000^{™} mass spectrometer, (Applied Biosystems/MDS SCIEX)
- Shimadzu LC-10AD HPLC pumps (2) with mixing tee (or equivalent) Shimadzu SCL-10A Controller (or equivalent) HTS-PAL or HTC-PAL Autoinjector (or equivalent)
- IEC Centra GP8R Centrifuge (or equivalent)
- TurboVap^{®} LV Evaporator

### Reagents and Chemicals

The reference substance, 4-Iodo-3-nitrobenzamide (BA), and the internal standard, 4-chloro-3-nitrobenzamide (CNBA), are obtained from Pacific BioDevelopment, LLC. When used, acetonitrile (ACN), methanol (MeOH), methyl-t-butyl ether (MTBE) and H₂O are HPLC grade or equivalent.

The following procedures for the preparation of reagents and solutions are provided as examples only. Other preparations may be substituted provided that the final concentrations remain the same.
- Make-up Reagent / Dilution Solvent: 50:50 MeOH : H₂O Combine equal volumes of MeOH and H₂O. (Store at room temperature);
- 1 M KH₂PO₄ Dissolve 68.05 g KH₂PO₄ in 500 mL of H₂O. (Store at room temperature);
- 1 M K₂HPO₄ Dissolve 87.1 g K₂HPO₄ in 500 mL of H₂O. (Store at room temperature);
- 1 M Potassium Phosphate Buffer pH~6 Combine 400 mL of 1 M KH₂PO₄ and ~60 mL of 1 M K₂HPO₄ to reach pH ~6.0. (Store at room temperature);
- 0.1% formic acid: Combine 1000 mL of H₂O and 1 mL formic acid. (Store at 4°C);
- Reconstitution Solvent: 15:85 ACN: 0.1% formic acid Combine 15 mL of ACN and 85 mL of 0.1% formic acid. (Store at room temperature);
- Mobile Phase A: 0.1% formic acid (FA) Combine 999 mL of H₂O and 1 mL formic acid. (Store at room temperature for mobile phase use only);
- Mobile Phase B: ACN with 0.1 % formic acid Combine 999 mL ACN and 1 mL formic acid. (Store at room temperature);
- (50:50 MeOH:H₂O) with ~0.3% formic acid: Combine 500 mL of MeOH, 500 mL of H₂O and 3 mL formic acid. (Store at room temperature);
- (80:20 ACN:H₂O) with ~0.1% formic acid: Combine 800 mL of ACN, 200 mL of H₂O and 1 mL formic acid. (Store at room temperature);

In this study, six different oral formulations were tested and compared to an IV dose of BA for determination of oral bioavailability. Oral administration to male and female Beagle dogs of BA formulated in sodium lauryl sulfate, Poloxamer 188 or in a size reduction formulation resulted in sustained plasma concentrations for 24 hours following administration. Oral bioavailability of 21.6% ± 10.4% was observed with the sodium lauryl sulfate formulation. Size reduced BA API in combination with the use of sodium lauryl sulfate, an ionic surfactant, appeared to enhance bioavailability of the drug substance.

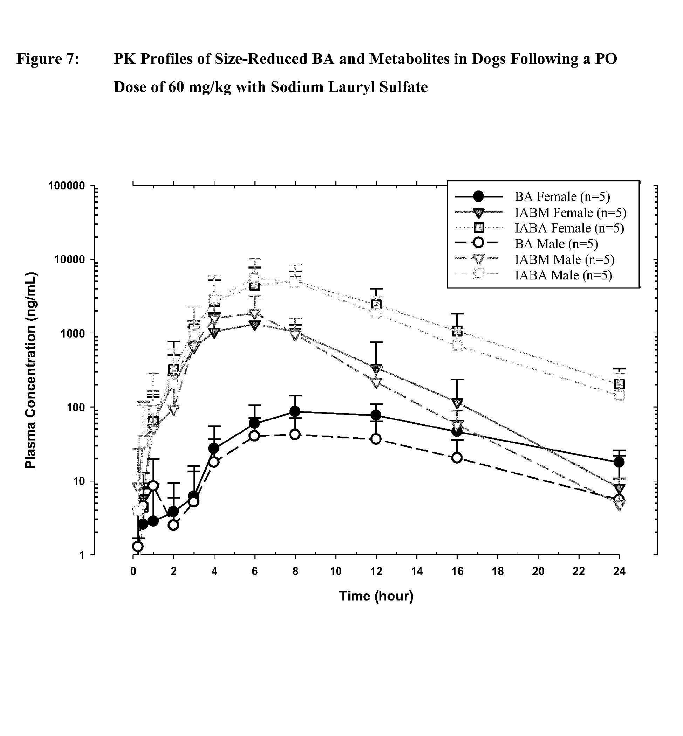

Figure 7 shows the oral bioavailability of different formulations of BA in female and male dogs from study 7666-110. These figures show the PK profiles obtained from male and female dogs given an oral dose of size-reduced BA. BA concentration (circles), IABM concentration (inverted triangles), and IABA concentration (squares). No obvious gender differences in PK profiles of BA and metabolites formed are observed.

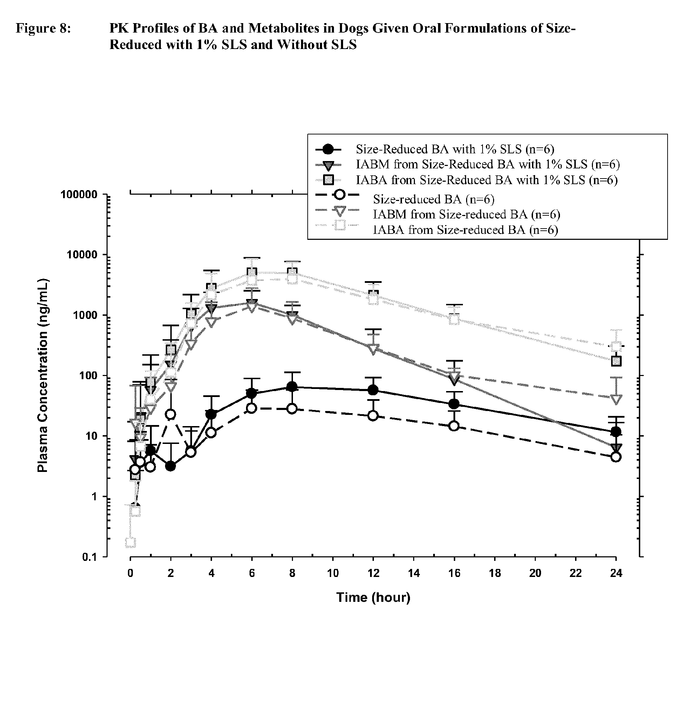

Figure 8 gives the comparison of the PK profiles in dogs (males and females) given an oral dose of either size-reduced BA or size-reduced BA with 1% sodium lauryl sulfate (SLS). The addition of 1% SLS to formulate BA appeared to increase the oral absorption of size-reduced BA providing a larger area under the plasma concentration versus time curve.

In study 7666-113, four different oral formulations (micronized BA with or without SLS, and semi-solid formulation) were tested and compared to an IV dose of BA for determination of oral bioavailability. Oral bioavailability of all formulations was under 6%.

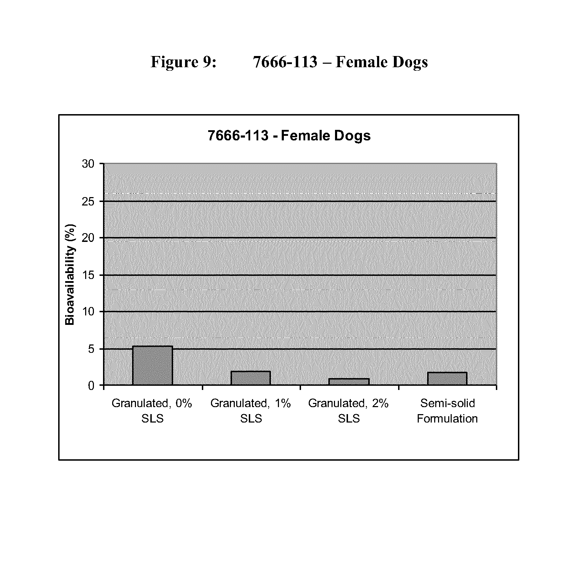

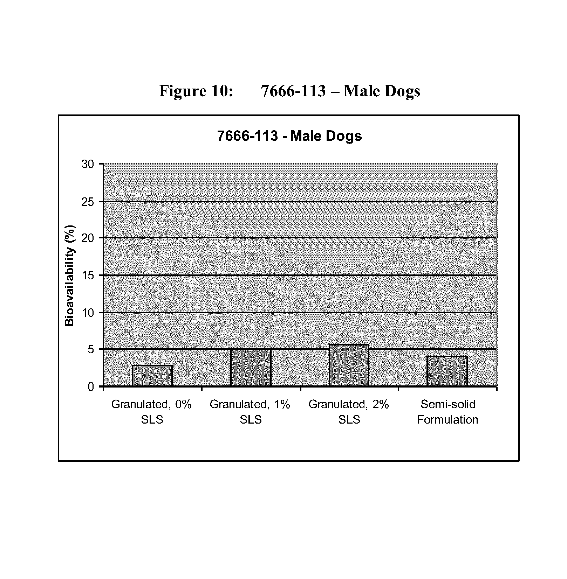

Figures 9 and 10 show the oral bioavailability of different formulations of BA in female and male dogs from study 7666-113.

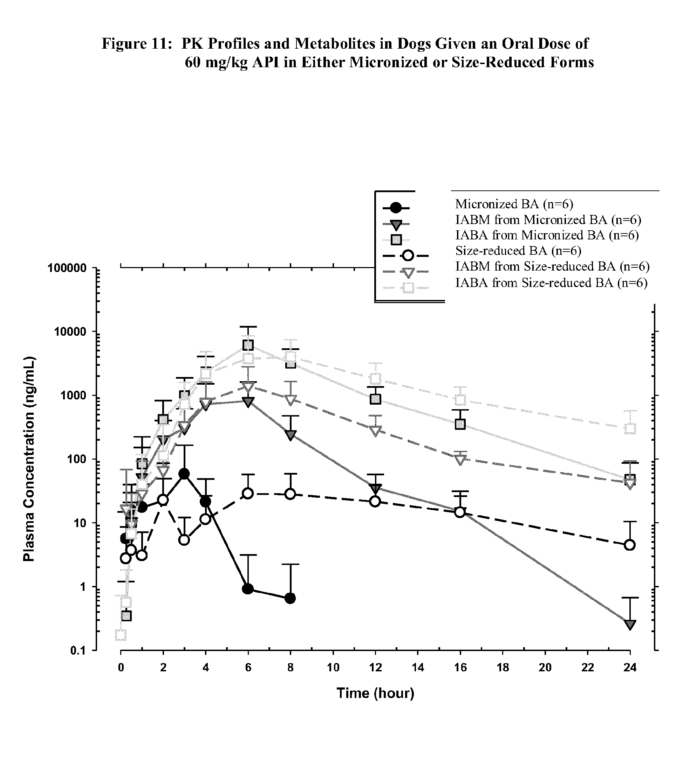

The effects of particle sizes of BA (micronized and size-reduced) on PK profiles are shown in Figure 11. It gives the comparison of the PK profiles in dogs (males and females) given an oral dose of either micronized or size-reduced BA. Data are taken from Covance Studies 7666-110 and 7666-113. For direct comparison, the oral dose of 100 mg/kg from Study 7666-113 was converted to 60 mg/kg with assumption of linear PK with dose.

Exposure from oral dose administration as indicated by AUC of BA was prolonged; and resulted in sustained systemic metabolite concentrations by 24 hours post dose with size-reduced API. Reduction in API particle size by micronization gave higher peak plasma concentration (Cmax) and shorter time to peak concentration (Tmax) indicative of more rapid oral absorption, however decline in BA and metabolite plasma concentration was also rapid.

When AUC values (calculated from dosing to 4 hours, 8 hours, 12 hours, and 24 hours post dose) were compared between oral dosing of size-reduced API and micronized API, it was shown that ratios for size-reduced to micronized API increased from less than one to greater than one with AUCs over a longer duration post dose, reflecting differences in uptake from the GI tract with faster absorption for the smaller micronized API. However, oral bioavailability (comparison of AUC 0-24h) was higher for the size-reduced material. Tmax and Cmax ratios also indicated absorption rate that was slower for the size-reduced material.

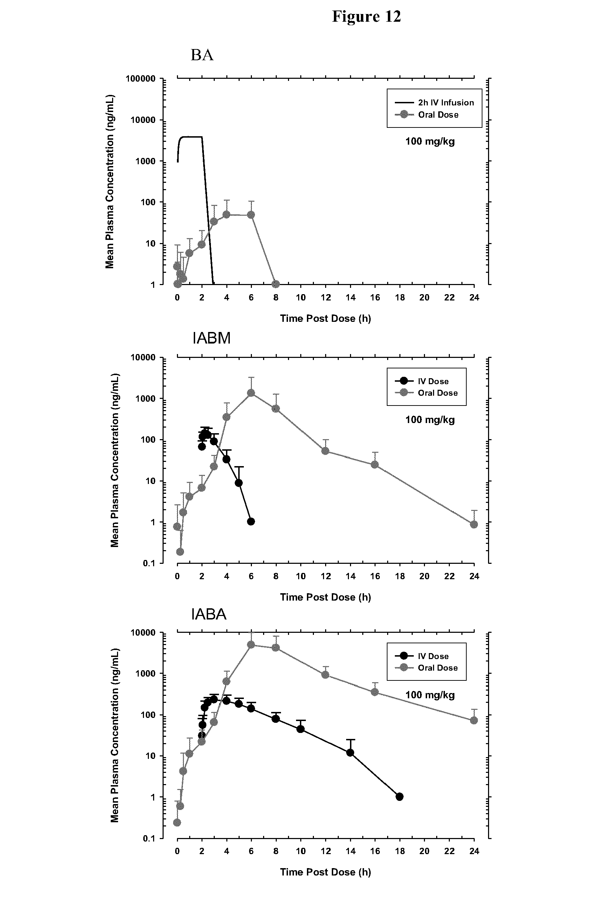

Figure 12 gives the comparison of the PK profiles of BA and its metabolites (IABM, IABA) in dogs given of either an IV infusion or an oral dose of BA (micronized with 2% SLS). Giving BA orally prolonged the exposure of BA and its metabolites in dogs.

In summary, the best oral bioavailability of BA of 21.6% ± 10.4% was observed with the size-reduced API with 1% SLS. Reduction in API particle size by micronization resulted in more rapid oral absorption and rapid decline in BA and metabolite plasma concentration, which led to a lower oral bioavailability compared to size-reduced API.

All of the methods disclosed and claimed herein can be made and executed without undue experimentation in light of the present disclosure. It will be apparent to those of skill in the art that variations may be applied without departing from the concept, spirit and scope of the invention. More specifically, it will be apparent that certain agents that both chemically and physiologically related may be substituted for the agents described herein while the same or similar results would be achieved. All such similar substitutes and modifications apparent to those skilled in the art are deemed to be within the spirit, scope and concept of the invention as defined by the appended claims. While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention.

Specific embodiments of the invention, as defined in the claims of the parent application, are set out in the following paragraphs:
1. A pharmaceutical composition comprising a compound of formula (Ia) 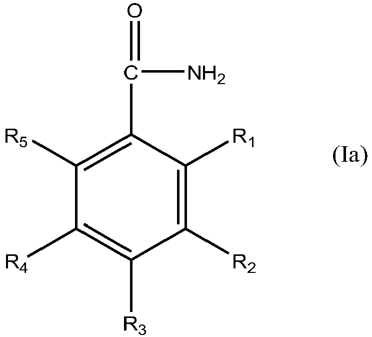 wherein R₁, R₂, R₃, R₄, and R₅ are, independently selected from the group consisting of hydrogen, hydroxy, amino, nitro, iodo, bromo, fluoro, chloro, (C₁ -C₆) alkyl, (C₁ -C₆) alkoxy, (C₃ -C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof, and
   at least one pharmaceutically acceptable solubilizer.
2. The pharmaceutical composition of preceding paragraph 1, wherein the solubilizer comprises a cyclodextrin, a surfactant, a co-solvent, or mixtures of two or more_thereof.
3. The pharmaceutical composition of preceding paragraph 1, wherein the solubility of the compound of formula Ia is at least about 1.5 times the solubility of that compound in pure water.
4. The pharmaceutical composition of preceding paragraph 1, wherein the solubility of the compound of formula Ia is at least about 2 times the solubility of that compound in pure water.
5. The pharmaceutical composition of preceding paragraph 1 wherein the solubility of the compound of formula Ia is at least about 5 times the solubility of that compound in pure water.
6. The pharmaceutical composition of preceding paragraph 1 wherein the solubility of the compound of formula Ia is at least about 10 times the solubility of that compound in pure water.
7. The pharmaceutical composition of preceding paragraph 1 wherein the solubility of the compound of formula Ia is at least about 50 times the solubility of that compound in pure water.
8. A pharmaceutical composition comprising a compound of formula (Ia) 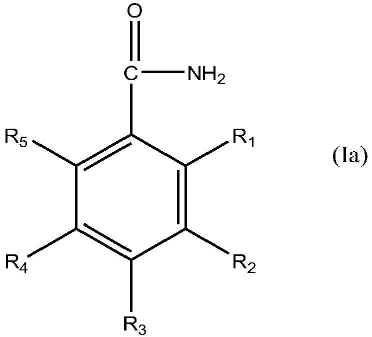 wherein R₁, R₂, R₃, R₄, and R₅ are, independently selected from the group consisting of hydrogen, hydroxy, amino, nitro, iodo, bromo, fluoro, chloro, (C₁ -C₆) alkyl, (C₁ -C₆) alkoxy, (C₃ -C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof, and
   a surfactant.
9. The composition of preceding paragraph 8, wherein the surfactant comprises one or more of: a poloxamer, a polysorbate, a polyethoxylated triglyceride, a polyethoxylated fatty acid or a compound of formula II or III: 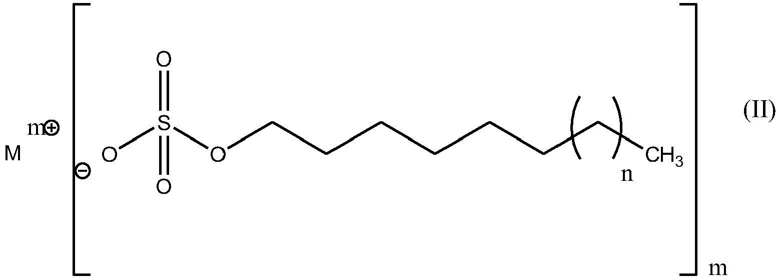 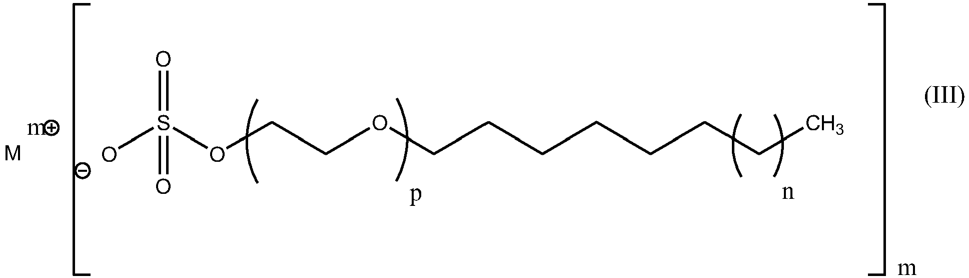 wherein M is a metal ion having a positive charge m+, m is an integer of value 1, 2 or 3 and n is an integer of value 1 to 11 and p is a value of 1 to 10.
10. The composition of preceding paragraph 9, wherein the surfactant comprises one or more of: sodium lauryl sulfate, sodium laureth sulfate, Polysorbate 80, Polysorbate 20, Cremophor EL, Cremophor RH40, Poloxamer 118 or Solutol HS-15.
11. A pharmaceutical composition comprising a compound of formula (Ia) 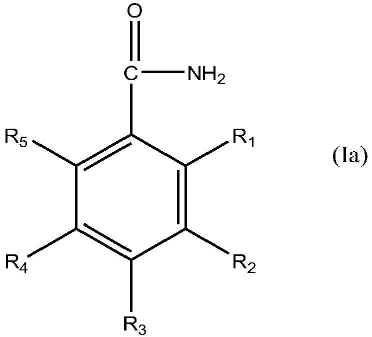 wherein R₁, R₂, R₃, R₄, and R₅ are, independently selected from the group consisting of hydrogen, hydroxy, amino, nitro, iodo, bromo, fluoro, chloro, (C₁ -C₆) alkyl, (C₁ -C₆) alkoxy, (C₃ -C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof, and
   a cyclodextrin.
12. The composition of preceding paragraph 11, wherein the cyclodextrin comprises one or more of:
   hydroxypropyl-β-cyclodextrin, hyroxypropyl-γ-cyclodextrin, and sulfobutyl ether- β-cyclodextrin.
13. A pharmaceutical composition comprising a compound of formula (Ia) 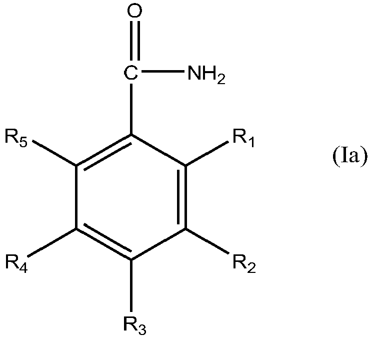 wherein R₁, R₂, R₃, R₄, and R₅ are, independently selected from the group consisting of hydrogen, hydroxy, amino, nitro, iodo, bromo, fluoro, chloro, (C₁ -C₆) alkyl, (C₁ -C₆) alkoxy, (C₃ -C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof, and
   a co-solvent.
14. The composition of preceding paragraph 13 wherein the co-solvent is selected from the group of: ethanol, glycofurol, glycerin formal, benzyl alcohol, PEG 400, propylene glycol, and N, N-dimethyl acetamide (DMA).
15. A method for treating a condition selected from cancer, and a viral condition comprising treating a subject suspected of having said condition with a pharmaceutical composition comprising a compound of formula (Ia) 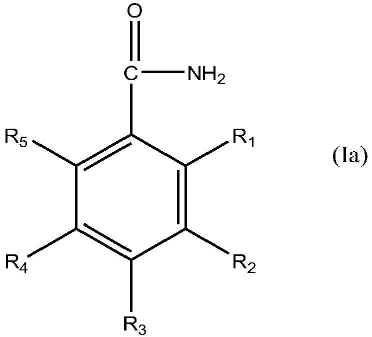 wherein R₁, R₂, R₃, R₄, and R₅ are, independently selected from the group consisting of hydrogen, hydroxy, amino, nitro, iodo, bromo, fluoro, chloro, (C₁ -C₆) alkyl, (C₁ -C₆) alkoxy, (C₃ -C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof, and
   one or more of the group consisting of: a cyclodextrin, a surfactant, and a co-solvent.
16. The method of preceding paragraph 15, wherein said compound of formula Ia is administered orally.
17. The method of preceding paragraph 15, wherein said compound of formula Ia is administered parenterally.
18. The method of preceding paragraph 15, wherein the cyclodextrin is selected from the group of hydroxypropyl-β-cyclodextrin, hyroxypropyl-γ-cyclodextrin, and sulfobutyl ether- β-cyclodextrin.
19. The method of preceding paragraph 15, wherein the surfactant is selected from the group of:
   Polysorbate 80, Polysorbate 20, Cremophor EL, Cremophor RH40, Poloxamer 118, and Solutol HS-15.
20. The method of preceding paragraph 15, wherein the co-solvent is selected from the group of: ethanol, glycofurol, glycerin formal, benzyl alcohol, PEG 400, propylene glycol, and N, N-dimethyl acetamide (DMA).
21. The method of preceding paragraph 15, wherein the condition is cancer and the cancer is a member of the group consisting of wherein the cancer is selected from adrenal cortical cancer, anal cancer, aplastic anemia, bile duct cancer, bladder cancer, bone cancer, bone metastasis, CNS tumors, peripheral CNS cancer, breast cancer, Castleman's Disease, cervical cancer, childhood Non-Hodgkin's lymphoma, colon and rectum cancer, endometrial cancer, esophagus cancer, Ewing's family of tumors, eye cancer, gallbladder cancer, gastrointestinal carcinoid tumors, gastrointestinal stromal tumors, gestational trophoblastic disease, hairy cell leukemia, Hodgkin's disease, Kaposi's sarcoma, kidney cancer, laryngeal and hypopharyngeal cancer, acute lymphocytic leukemia, acute myeloid leukemia, children's leukemia, chronic lymphocytic leukemia, chronic myeloid leukemia, liver cancer, lung cancer, lung carcinoid tumors, Non-Hodgkin's lymphoma, male breast cancer, malignant mesothelioma, multiple myeloma, myelodysplastic syndrome, myeloproliferative disorders, nasal cavity and paranasal cancer, nasopharyngeal cancer, neuroblastoma, oral cavity and oropharyngeal cancer, osteosarcoma, ovarian cancer, pancreatic cancer, penile cancer, pituitary tumor, prostate cancer, retinoblastoma, rhabdomyosarcoma, salivary gland cancer, sarcoma (adult soft tissue cancer), melanoma skin cancer, non-melanoma skin cancer, stomach cancer, testicular cancer, thymus cancer, thyroid cancer, uterine sarcoma, vaginal cancer, vulvar cancer, Waldenstrom's macroglobulinemia and cancers of viral origin.
22. A kit comprising a compound of formula (Ia) 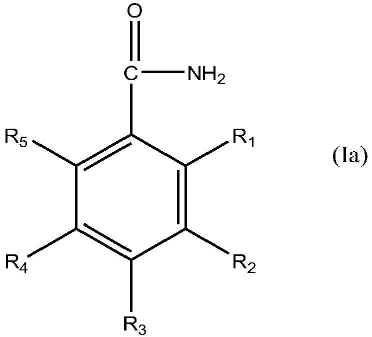 wherein R₁, R₂, R₃, R₄, and R₅ are, independently selected from the group consisting of hydrogen, hydroxy, amino, nitro, iodo, bromo, fluoro, chloro, (C₁ -C₆) alkyl, (C₁ -C₆) alkoxy, (C₃ -C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof, and
   a cyclodextrin, a surfactant, a co-solvent, or mixtures therof.
23. The kit of preceding paragraph 22 wherein the cyclodextrin is selected from the group of hydroxypropyl-β-cyclodextrin, hyroxypropyl-γ-cyclodextrin, and sulfobutyl ether- β-cyclodextrin.
24. The kit of preceding paragraph 22, wherein the surfactant is selected from the group of: Polysorbate 80, Polysorbate 20, Cremophor EL, Cremophor RH40, Poloxamer 118, and Solutol HS-15.
25. The kit of preceding paragraph 22, wherein the co-solvent is selected from the group of: ethanol, glycofurol, glycerin formal, benzyl alcohol, PEG 400, propylene glycol, and N, N-dimethyl acetamide (DMA).
26. An aqueous solution comprising a compound of formula (Ia) 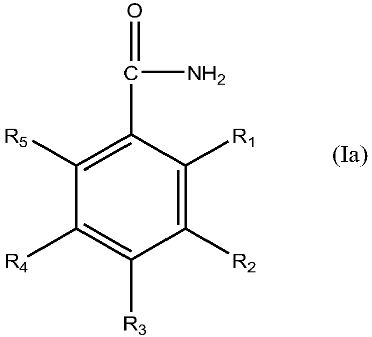 wherein R₁, R₂, R₃, R₄, and R₅ are, independently selected from the group consisting of hydrogen, hydroxy, amino, nitro, iodo, bromo, fluoro, chloro, (C₁ -C₆) alkyl, (C₁ -C₆) alkoxy, (C₃ -C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof, and
   a cyclodextrin, a surfactant, a co-solvent, or mixtures thereof.
27. A unit dosage comprising a compound of formula (Ia) 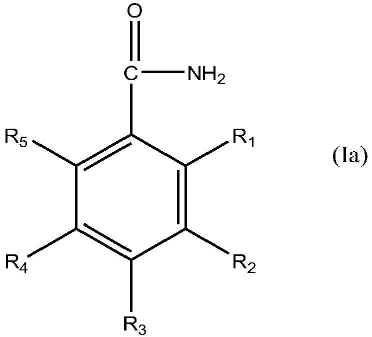 wherein R₁, R₂, R₃, R₄, and R₅ are, independently selected from the group consisting of hydrogen, hydroxy, amino, nitro, iodo, bromo, fluoro, chloro, (C₁ -C₆) alkyl, (C₁ -C₆) alkoxy, (C₃ -C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof, and
   at least one pharmaceutically acceptable solubilizer.
28. The unit dosage of preceding paragraph 27, wherein the solubilizer comprises a surfactant and optionally one or more of a cyclodextrin, a co-solvent, a lipid or mixtures thereof.
29. The unit dosage of preceding paragraph 27, wherein the solubility of the compound is at least about 1.5 times the solubility of that compound in pure water.
30. The unit dosage of preceding paragraph 27, wherein the solubility of the compound is at least about 2 times the solubility of that compound in pure water.
31. The unit dosage of preceding paragraph 27, wherein the solubility of the compound is at least about 5 times the solubility of that compound in pure water.
32. The unit dosage of preceding paragraph 27, wherein the solubility of the compound is at least about 10 times the solubility of that compound in pure water.
33. The unit dosage of preceding paragraph 27, wherein the solubility of the compound is at least about 50 times the solubility of that compound in pure water.
34. The unit dosage of preceding paragraph 27, wherein the surfactant is one or more compounds of formula II or III: 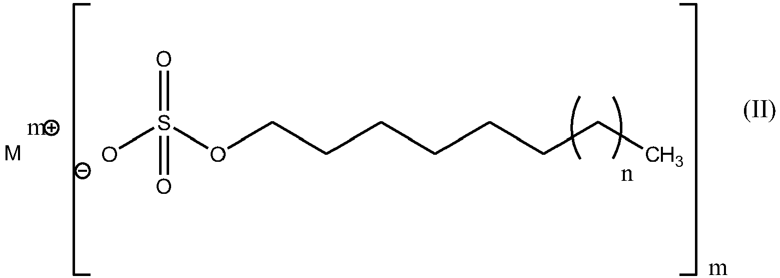 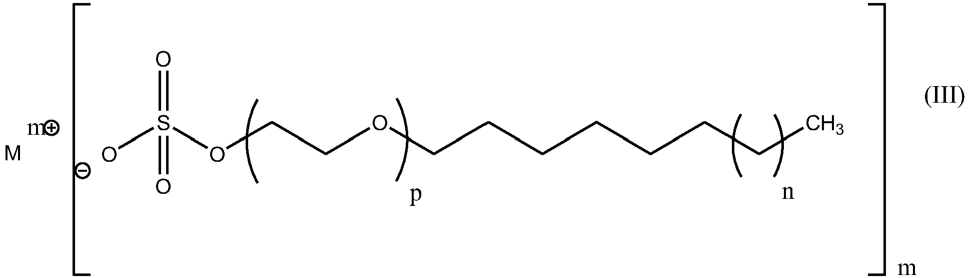
   wherein M is a metal ion having a positive charge m+, m is an integer of value 1, 2 or 3 and n is an integer of value 1 to 11 and p is a value of 1 to 10.
35. The unit dosage of preceding paragraph 27, wherein the surfactant comprises one or more poloxamer, polysorbate, polyethoxylated triglyceride or polyethoxylated fatty acid.
36. The unit dosage of preceding paragraph 35, wherein the surfactant is selected from the group of:
   Polysorbate 80, Polysorbate 20, Cremophor EL, Cremophor RH40, Poloxamer 118, and Solutol HS-15.
37. A parenteral pharmaceutical composition comprising a compound of formula (Ia) 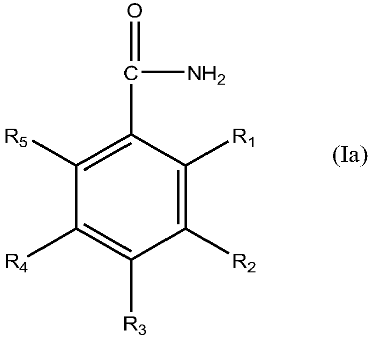 wherein R₁, R₂, R₃, R₄, and R₅ are, independently selected from the group consisting of hydrogen, hydroxy, amino, nitro, iodo, bromo, fluoro, chloro, (C₁ -C₆) alkyl, (C₁ -C₆) alkoxy, (C₃ -C₇) cycloalkyl, and phenyl, wherein at least two of the five R₁, R₂, R₃, R₄, and R₅ substituents are always hydrogen, at least one of the five substituents are always nitro, and at least one substituent positioned adjacent to a nitro is always iodo, and pharmaceutically acceptable salts, solvates, isomers, tautomers, metabolites, analogs, or prodrugs thereof;
   a cyclodextrin; and
   water.
38. The composition of preceding paragraph 37, wherein the cyclodextrin is selected from the group of hydroxypropyl-β-cyclodextrin, hyroxypropyl-γ-cyclodextrin, and sulfobutyl ether- β-cyclodextrin.
39. The composition of preceding paragraph 37, further comprising a co-solvent.
40. The composition of preceding paragraph 39, wherein the co-solvent is selected from the group of:
   ethanol, glycofurol, glycerin formal, benzyl alcohol, PEG 400, propylene glycol, and N, N-dimethyl acetamide (DMA).

## Claims

1. A pharmaceutical composition comprising 4-iodo-3-nitrobenzamide, or a pharmaceutically acceptable salt thereof, and a solubilizer, wherein the solubilizer is about 1% to about 2% (w/w) sodium lauryl sulphate, or the solubilizer is a cyclodextrin which is hydroxypropyl-β-cyclodextrin or sulphobutyl ether-β-cyclodextrin.

2. The pharmaceutical composition of Claim 1, which is formulated for oral administration.

3. The pharmaceutical composition of Claim 1 or Claim 2, wherein the solubilizer is about 1% to about 2% (w/w) sodium lauryl sulfate.

4. The pharmaceutical composition of Claim 1 or Claim 2, wherein the solubilizer is a cyclodextrin which is hydroxypropyl-β-cyclodextrin or sulphobutyl ether-β-cyclodextrin.

5. The pharmaceutical composition as claimed in Claim 4, wherein the solubilizer further comprises a physiologically compatible buffer.

6. The pharmaceutical composition as claimed in Claim 5, wherein the physiologically compatible buffer is a phosphate buffer.

7. The pharmaceutical composition as claimed in Claim 6, wherein the physiologically compatible buffer is 10 mM phosphate buffer.

8. The pharmaceutical composition as claimed in any one of Claims 5 to 7, wherein the cyclodextrin is present at a concentration of 25% and the buffer is 10 mM phosphate at pH 7.4.

9. The pharmaceutical composition as claimed in Claim 5, wherein the cyclodextrin is 2-hydroxypropyl-β-cydodextrin and is present at a concentration of 25%, and the buffer is 10 mM phosphate at pH 7.4.

10. The pharmaceutical composition as claimed in any of Claims 4 to 9, wherein the cyclodextrin is present in said pharmaceutical composition at a concentration of from 25% to 40% (w/v) of the composition.

11. The pharmaceutical composition as claimed in Claim 10, wherein the cyclodextrin is 2-hydroxypropyl-β-cyclodextrin.

12. The pharmaceutical composition as claimed in Claim 10, wherein the cyclodextrin is Kleptose^{™}.

13. The pharmaceutical composition as claimed in Claim 10, wherein the cyclodextrin is present at a concentration of 25% (w/v).

14. The pharmaceutical composition as claimed in any preceding claim, wherein the 4-iodo-3-nitrobenzamide is present in the composition as size-reduced granules.

15. The pharmaceutical composition as claimed in any preceding claim, wherein administration of the composition results in a sustained plasma concentration of the 4-iodo-3-nitrobenzamide for at least 24 hours.

16. The pharmaceutical composition as claimed in any preceding claim, wherein the composition is formulated such that when orally administered at a dose of 100 mg/kg to beagle dogs results in a plasma concentration of 4-iodo-3-nitrobenzamide of at least 10 ng/mL at 6 hours after dosing.

17. The pharmaceutical composition of any preceding claim, wherein the composition is formulated such that when orally administered at a dose of 100 mg/kg to beagle dogs results in a plasma concentration of 4-iodo-3-nitrobenzamide of at least 100 ng/mL at 6 hours after dosing.

18. The pharmaceutical composition according to any preceding claim, for use in the treatment of a cancer or a viral condition in a subject suspected of having said cancer or viral condition.

19. The pharmaceutical composition of Claim 18, wherein the cancer is a member of the group consisting of adrenal cortical cancer, anal cancer, aplastic anemia, bile duct cancer, bladder cancer, bone cancer, bone metastasis, CNS tumors, peripheral CNS cancer, breast cancer, Castleman's Disease, cervical cancer, childhood Non-Hodgkin's lymphoma, colon and rectum cancer, endometrial cancer, esophagus cancer, Ewing's family of tumors, eye cancer, gallbladder cancer, gastrointestinal carcinoid tumors, gastrointestinal stromal tumors, gestational trophoblastic disease, hairy cell leukemia, Hodgkin's disease, Kaposi's sarcoma, kidney cancer, laryngeal and hypopharyngeal cancer, acute lymphocytic leukemia, acute myeloid leukemia, children's leukemia, chronic lymphocytic leukemia, chronic myeloid leukemia, liver cancer, lung cancer, lung carcinoid tumors, Non-Hodgkin's lymphoma, male breast cancer, malignant mesothelioma, multiple myeloma, myelodysplastic syndrome, myeloproliferative disorders, nasal cavity and paranasal cancer, nasopharyngeal cancer, neuroblastoma, oral cavity and oropharyngeal cancer, osteosarcoma, ovarian cancer, pancreatic cancer, penile cancer, pituitary tumor, prostate cancer, retinoblastoma, rhabdomyosarcoma, salivary gland cancer, sarcoma (adult soft tissue cancer), melanoma skin cancer, non-melanoma skin cancer, stomach cancer, testicular cancer, thymus cancer, thyroid cancer, uterine sarcoma, vaginal cancer, vulvar cancer, Waldenstrom's macroglobulinemia and cancers of viral origin.

20. The pharmaceutical composition as claimed in Claim 18 or Claim 19, wherein the cancer is a member of the group consisting of leukemia, breast cancer, ovarian cancer, lung cancer, bladder cancer, prostate cancer, pancreatic cancer, and cervical cancer.

21. The pharmaceutical composition as claimed in any of Claims 18 to 20, wherein the cancer is a member of the group consisting of breast cancer, ovarian cancer, lung cancer, and pancreatic cancer.

22. The pharmaceutical composition as claimed in any of Claims 18 to 21, wherein the cancer is breast cancer.

23. The pharmaceutical composition as claimed in any of Claims 18 to 21, wherein the cancer is ovarian cancer.

24. The pharmaceutical composition as claimed in any of Claims 18 to 21, wherein the cancer is lung cancer.
